# EUROPÄISCHE PATENTSCHRIFT

(11) **EP 2 297 104 B1**
(45) Veröffentlichungstag und Bekanntmachung des Hinweises auf die Patenterteilung: **07.08.2013**
(21) Anmeldenummer: 09753647.8
(22) Anmeldetag: 22.05.2009
(51) Int. Cl.: C07D 213/70, C07D 413/12, C07D 417/12, C07D 417/14, A61K 31/33, A61P 9/00

(54) **2-ALKOXY-SUBSTITUIERTE DICYANOPYRIDINE UND IHRE VERWENDUNG**
2-ALKOXY-SUBSTITUTED DICYANOPYRIDINES AND USE THEREOF
DICYANOPYRIDINES SUBSTITUÉES PAR DU 2-ALCOXY ET LEUR UTILISATION

(30) Priorität: 29.05.2008 DE 102008025841
(43) Veröffentlichungstag der Anmeldung: 23.03.2011
(73) Patentinhaber: Bayer Intellectual Property GmbH, 40789 Monheim (DE)
(72) Erfinder: HÜBSCH, Walter, 42113 Wuppertal (DE); MEIBOM, Daniel, 51373 Leverkusen (DE); VAKALOPOULOS, Alexandros, 40721 Hilden (DE); ALBRECHT-KÜPPER, Barbara, 42289 Wülfrath (DE); NELL, Peter, 42115 Wuppertal (DE); ZIMMERMANN, Katja, 40489 Düsseldorf (DE); SÜSSMEIER, Frank, 80992 München (DE); KELDENICH, Joerg, 42113 Wuppertal (DE)
(74) Vertreter: BIP Patents
(86) Internationale Anmeldenummer: PCT/EP2009/003652
(87) Internationale Veröffentlichungsnummer: WO 2009/143992

(56) Entgegenhaltungen:
- WO-A-2006/027142

## Beschreibung

Die vorliegende Anmeldung betrifft neue 2-Alkoxy-substituierte Dicyanopyridine, Verfahren zu ihrer Herstellung, ihre Verwendung zur Behandlung und/oder Prävention von Krankheiten sowie ihre Verwendung zur Herstellung von Arzneimitteln zur Behandlung und/oder Prävention von Krankheiten, vorzugsweise zur Behandlung und/oder Prävention von kardiovaskulären Erkrankungen.

Adenosin, ein Purin-Nukleosid, ist in allen Zellen vorhanden und wird unter einer Vielzahl von physiologischen und pathophysiologischen Stimuli freigesetzt. Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Funktionen als hormonähnliche Substanz oder Neurotransmitter aus.

Unter normoxischen Bedingungen ist die Konzentration des freien Adenosin im Extrazellulärraum sehr niedrig. Die extrazelluläre Konzentration von Adenosin erhöht sich in den betroffenen Organen jedoch dramatisch unter ischämische bzw. hypoxischen Bedingungen. So ist beispielsweise bekannt, dass Adenosin die Thrombozyten-Aggregation hemmt und die Durchblutung der Herzkranzgefäße steigert. Weiterhin wirkt es auf den Blutdruck, die Herzfrequenz, auf die Ausschüttung von Neurotransmittern und auf die Lymphozyten-Differenzierung. In Adipozyten ist Adenosin in der Lage, die Lipolyse zu hemmen und somit die Konzentration an freien Fettsäuren und Triglyzeriden im Blut zu senken.

Diese Wirkungen von Adenosin zielen darauf ab, das Sauerstoffangebot der betroffenen Organe zu erhöhen bzw. den Stoffwechsel dieser Organe zu drosseln, um damit unter ischämische oder hypoxischen Bedingungen eine Anpassung des Organstoffwechsels an die Organdurchblutung zu erreichen.

Die Wirkung von Adenosin wird über spezifische Rezeptoren vermittelt. Bekannt sind bisher die Subtypen A1, A2a, A2b und A3. Als "Adenosinrezeptor-selektive Liganden" werden erfindungsgemäß solche Substanzen bezeichnet, die selektiv an einen oder mehrere Subtypen der Adenosin-rezeptoren binden und dabei entweder die Wirkung des Adenosin nachahmen (Adenosin-Agonisten) oder dessen Wirkung blockieren (Adenosin-Antagonisten) können.

Die Wirkungen dieser Adenosin-Rezeptoren werden intrazellulär durch den Botenstoff cAMP vermittelt. Im Falle der Bindung von Adenosin an die A2a- oder A2b-Rezeptoren kommt es über eine Aktivierung der membranständigen Adenylatzyklase zu einem Anstieg des intrazellulären cAMP, während die Bindung des Adenosin an die A1- oder A3-Rezeptoren über eine Hemmung der Adenylatzyklase eine Abnahme des intrazellulären cAMP-Gehalts bewirkt.

Im Herz-Kreislaufsystem sind die Hauptwirkungen der Aktivierung von Adenosin-Rezeptoren: Bradykardie, negative Inotropie und Protektion des Herzens vor Ischämie ("preconditioning") über A1-Rezeptoren, Dilation der Gefäße über A2a- und A2b-Rezeptoren sowie Inhibition der Fibroblasten und Glattmuskelzellproliferation über A2b-Rezeptoren.

Im Falle von A1-Agonisten (Kopplung bevorzugt über Gᵢ-Proteine) wird dabei eine Abnahme des intrazellulären cAMP-Gehaltes beobachtet (bevorzugt nach direkter Vorstimulation der Adenylatzyklase durch Forskolin). Entsprechend führen A2a- und A2b-Agonisten (Kopplung bevorzugt über Gₛ-Proteine) zu einer Zunahme und A2a- und A2b-Antagonisten zu einer Abnahme im cAMP-Gehalt der Zellen. Im Falle der A2-Rezeptoren ist eine direkte Vorstimulation der Adenylatzyklase durch Forskolin nicht hilfreich.

Die Aktivierung von A1-Rezeptoren durch spezifische A1-Agonisten führt beim Menschen zu einer frequenzabhängigen Senkung der Herzfrequenz, ohne einen Einfluss auf den Blutdruck zu haben. Selektive A1-Agonisten könnten somit unter anderem für die Behandlung von Angina pectoris und Vorhofflimmern geeignet sein.

Die kardioprotektive Wirkung der A1-Rezeptoren im Herzen kann unter anderem durch die Aktivierung dieser A1-Rezeptoren durch spezifische A1-Agonisten für die Behandlung und Organprotektion bei akutem Myokardinfarkt, akutem Koronarsyndrom, Herzinsuffizienz, Bypass Operationen, Herzkatheteruntersuchungen und Organtransplantationen genutzt werden.

Die Aktivierung von A2b-Rezeptoren durch Adenosin oder spezifische A2b-Agonisten führt über die Erweiterung von Gefäßen zu einer Blutdrucksenkung. Die Blutdrucksenkung ist von einem reflektorischen Herzfrequenzanstieg begleitet. Der Herzfrequenzanstieg kann durch die Aktivierung von A1-Rezeptoren durch spezifische A1-Agonisten reduziert werden.

Die kombinierte Wirkung von selektiven A1/A2b-Agonisten auf das Gefäßsystem und die Herzfrequenz resultiert somit in einer systemischen Blutdrucksenkung ohne relevanten Herzfrequenzanstieg. Mit einem solchen pharmakologischen Profil könnten duale A1/A2b-Agonisten zur Behandlung z.B. der Hypertonie beim Menschen eingesetzt werden.

In Adipozyten bewirkt die Aktivierung von A1- und A2b-Rezeptoren eine Inhibition der Lipolyse. Die selektive bzw. kombinierte Wirkung von A1- und A1/A2b-Agonisten auf den Lipidstoffwechsel führt somit zu einer Senkung von freien Fettsäuren und Triglyzeriden. Eine Senkung der Lipide wiederum führt bei Patienten mit Metabolischem Syndrom und bei Diabetikern zur Verringerung der Insulinresistenz und zur Verbesserung der Symptomatik.

Die Hemmung von A1-Rezeptoren durch spezifische A1-Antagonisten wirkt beim Menschen urikosurisch, natriuretisch sowie Kalium sparend diuretisch ohne Beeinflussung der glomerulären Filtrationsrate und somit nierenprotektiv. Selektive A1-Antagonisten können somit unter anderem zur Behandlung von akut dekompensierter Herzinsuffizienz und chronischer Herzinsuffizienz geeignet sein. Desweiteren können sie zur Nierenprotektion bei Nephropathie und anderen Nierenerkrankungen eingesetzt werden.

Die zuvor genannte Rezeptor-Selektivität lässt sich bestimmen durch die Wirkung der Substanzen an Zelllinien, die nach stabiler Transfektion mit der entsprechenden cDNA die jeweiligen Rezeptorsubtypen exprimieren (siehe hierzu die Druckschrift M. E. Olah, H. Ren, J. Ostrowski, K. A. Jacobson, G. L. Stiles, "Cloning, expression, and characterization of the unique bovine A1 adenosine receptor. Studies on the ligand binding site by site-directed mutagenesis", J. Biol. Chem. 267 (1992), Seiten 10764-10770, deren Offenbarung hiermit im vollen Umfang durch Bezugnahme eingeschlossen ist).

Die Wirkung der Substanzen an solchen Zelllinien lässt sich erfassen durch biochemische Messung des intrazellulären Botenstoffes cAMP (siehe hierzu die Druckschrift K. N. Klotz, J. Hessling, J. Hegler, C. Owman, B. Kull, B. B. Fredholm, M. J. Lohse, "Comparative pharmacology of human adenosine receptor subtypes - characterization of stably transfected receptors in CHO cells", Naunyn Schmiedebergs Arch. Pharmacol. 357 (1998), Seiten 1-9, deren Offenbarung hiermit im vollen Umfang durch Bezugnahme eingeschlossen ist).

Bei den aus dem Stand der Technik bekannten, als "Adenosinrezeptor-spezifisch" geltenden Liganden handelt es sich überwiegend um Derivate auf Basis des natürlichen Adenosins [S.-A. Poulsen und R. J. Quinn, "Adenosine receptors: New opportunities for future drugs", Bioorganic and Medicinal Chemistry 6 (1998), Seiten 619-641]. Diese aus dem Stand der Technik bekannten Adenosin-Liganden haben jedoch meistens den Nachteil, dass sie nicht wirklich rezeptorspezifisch wirken, schwächer wirksam sind als das natürliche Adenosin oder nach oraler Applikation nur sehr schwach wirksam sind. Deshalb werden sie überwiegend nur für experimentelle Zwecke verwendet. In klinischer Entwicklung befindliche Verbindungen dieser Art eignen sich bislang nur zur intravenösen Applikation.

In WO 01/25210, WO 02/070484 und WO 02/070485 werden substituierte 2-Thio- bzw. 2-Oxy-3,5-dicyano-4-phenyl-6-aminopyridine als Adenosinrezeptor-Liganden für die Behandlung von kardiovaskulären Erkrankungen offenbart. In WO 03/053441 werden spezifisch substituierte 2-Thio-3,5-dicyano-4-phenyl-6-aminopyridine als selektive Liganden des Adenosin A1-Rezeptors zur Behandlung kardiovaskulärer Erkrankungen beschrieben. Allerdings zeigte es sich, dass diese Verbindungen bezüglich ihrer physikochemischen Eigenschaften, wie beispielsweise ihrer Löslichkeit und/oder Formulierbarkeit, und/oder hinsichtlich ihrer *in vivo*-Eigenschaften, wie beispielsweise ihrem pharmakokinetischen Verhalten, ihrer Dosis-Wirkungsbeziehung und/oder ihrem Metabolisierungsweg, Nachteile aufweisen.

Die Herstellung substituierter 2-Thiopyridine wird in WO 98/54139 beschrieben WO 99/32117 offenbart substituierte Pyridine als Acetylcholin-Rezeptor Modulatoren zur Behandlung von ZNS-Erkrankungen. Weiterhin werden in WO 01/62233 verschiedene Pyridin- und Pyrimidin-Derivate sowie deren Verwendung als Adenosinrezeptor-Modulatoren beansprucht. Substituierte 3,5-Dicyanopyridine als Caicium-abhängige Kaliumkanalöffner zur Behandlung urologischer Erkrankungen werden in EP 1302 463-A1 offenbart. In WO 03/091246 werden Pyrrolsubstituierte Pyridine und Pyrimidine als Kinase Inhibitoren zur Behandlung von beispielsweise Krebs beschrieben. WO 2008/008059 beschreibt die Verwendung von verschiedenen heterocyclischen Verbindungen zur Behandlung von Krebs. WO 2009/015776 offenbart Oxazolsubstituierte Dicyanopyridine zur Behandlung kardiovaskulärer Erkrankungen.

WO 2006/027142 offenbart 2-Thio-3,5-Dicyano-4-Phenyl-6-aminopridine zur Behandlung kardiovaskulärer Erkrankungen.

Aufgabe der vorliegenden Erfindung ist die Bereitstellung neuer Verbindungen, die als potente und selektive Liganden des Adenosin A1- und/oder A2b-Rezeptors wirken und als solche zur Behandlung und/oder Prävention von Krankheiten, insbesondere zur Behandlung und/oder Prävention von kardiovaskulären Erkrankungen geeignet sind und ein gleiches oder verbessertes physikochemisches, pharmakokinetisches und/ oder therapeutisches Profil gegenüber den aus dem Stand der Technik bekannten Verbindungen aufweisen.

Gegenstand der vorliegenden Erfindung sind Verbindungen der Formel (I) 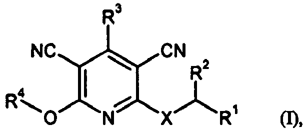 in welcher
- X: für O oder S steht,
wobei (C₆-C₁₀)-Aryl und 5- bis 10-gliedriges Heteroaryl mit 1 oder 2 Substituenten unabhängig voneinander ausgewählt aus der Gruppe Halogen, Nitro, Cyano, (C₁-C₆)-Alkyl, Trifluormethyl, Hydroxy, (C₁-C₆)-Alkoxy, Amino, Mono-(C₁-C₆)-alkylamino, Di-(C₁-C₆)-alkylamin, Hydroxycarbonyl, (C₁-C₆)-Alkoxycarbonyl, Aminocarbonyl, Mono-(C₁-C₆)-alkylaminocarbonyl, Di-(C₁-C₆)-alkylaminocarbonyl, (C₃-C₇)-Cycloalkylaminocarbonyl, Aminosulfonyl, Mono-(C₁-C₆)-alkylaminosulfonyl, Di-(C₁-C₆)-alkylaminosulfonyl, (C₁-C₆)-Alkylsulfonylamino, Pyrrolidino, Piperidino, Morpholino, Piperazino, *N*'-(C₁-C₄)-Alkylpiperazino, Pyrrolidinocarbonyl, Piperidinocarbonyl, Morpholinocarbonyl, Piperazinocarbonyl, *N*'-(C₁-C₄)-Alkylpiperazinocarbonyl und -L-R⁵ substituiert sein können,
worin
L für eine Bindung, NH oder O steht,
R⁵ für Phenyl oder 5- oder 6-gliedriges Heteroaryl steht,
worin Phenyl und 5- oder 6-gliedriges Heteroaryl ihrerseits mit 1 bis 3 Substituenten unabhängig voneinander ausgewählt aus der Gruppe Halogen, Nitro, Cyano, (C₁-C₆)-Alkyl, Trifluormethyl, Hydroxy, (C₁-C₆)-Alkoxy, Difluormethoxy, Trifluormethoxy, Amino, Mono-(C₁-C₆)-alkylamino, Di-(C₁-C₆)-alkylanüno, Hydroxycarbonyl und (C₁-C₆)-Alkoxycarbonyl sein können,
- R²: für Wasserstoff oder (C₁-C₄)-Alkyl steht,
- R³: für Phenyl oder 5- oder 6-gliedriges Heteroaryl steht,
wobei Phenyl und 5- oder 6-gliedriges Heteroaryl mit 1 bis 3 Substituenten unabhängig voneinander ausgewählt aus der Gruppe Halogen, Cyano, Hydroxy, (C₁-C₆)-Alkyl, (C₁-C₆)-Alkoxy, (C₃-C₇)-Cycloalkoxy, Tetrahydrofuranyloxy, Pyrrolidinyloxy und -NR^{A}R^{B} substituiert sein können,
worin (C₁-C₆)-Alkyl und (C₁-C₆)-Alkoxy mit 1 bis 3 Substituenten unabhängig voneinander ausgewählt aus der Gruppe Fluor, Trifluormethyl, (C₃-C₇)-Cycloalkyl, Hydroxy, (C₁-C₄)-Alkoxy, Hydroxycarbonyl, (C₁-C₄)-Alkoxycarbonyl, Amino, Aminocarbonyl, Mono-(C₁-C₄)-alkylamino und Di-(C₁-C₄)-alkylamino substituiert sein können,
und
worin (C₃-C₇)-Cycloalkoxy, Tetrahydrofuranyloxy und Pyrrolidinyloxy mit 1 oder 2 Substituenten unabhängig voneinander ausgewählt aus der Gruppe (C₁-C₄)-Alkyl, Hydroxy, Oxo und (C₁-C₄)-Alkoxy substituiert sein können,
und
worin
R^{A} für Wasserstoff oder (C₁-C₆)-Alkyl steht,
worin (C₁-C₆)-Alkyl seinerseits mit 1 bis 3 Substituenten Fluor substituiert sein kann,
und
worin (C₁-C₆)-Alkyl seinerseits mit einem Substituenten ausgewählt aus der Gruppe Hydroxy und (C₁-C₄)-Alkoxy substituiert sein kann,
R^{B} für Wasserstoff, (C₁-C₆)-Alkyl, (C₃-C₇)-Cycloalkyl, (C₁-C₆)-Alkylcarbonyl, (C₁-C₄)-Alkylsulfonyl oder (C₃-C₇)-Cycloalkylsulfonyl steht,
worin (C₁-C₆)-Alkyl seinerseits mit 1 oder 2 Substituenten unabhängig voneinander ausgewählt aus der Gruppe Fluor, Trifluormethyl, (C₃-C₇)-Cycloalkyl, Hydroxy, (C₁-C₄)-Alkoxy, Hydroxycarbonyl, (C₁-C₄)-Alkoxycarbonyl, Amino, Mono-(C₁-C₄)-alkylamino und Di-(C₁-C₄)-alkylamino substituiert sein kann,
und
worin (C₃-C₇)-Cycloalkyl seinerseits mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
oder
R^{A} und R^{B} zusammen mit dem Stickstoffatom, an das sie gebunden sind, einen 4-bis 7-gliedrigen Heterocyclus bilden, der ein weiteres Ring-Heteroatom aus der Reihe N, O oder S enthalten und mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
oder
worin zwei benachbarte Substituenten am Phenyl zusammen mit den Kohlenstoffatomen, an die sie gebunden sind, ein 1,3-Dioxolan, 1,3-Dioxan oder 2,2-Difluor-1,3-dioxolan bilden können,
- R⁴: für (C₁-C₆)-Alkyl, (C₃-C₇)-Cycloalkyl oder 4- bis 6-gliedriges Heterocyclyl steht,
wobei (C₁-C₆)-Alkyl mit 1 oder 2 Substituenten unabhängig voneinander ausgewählt aus der Gruppe Fluor, Trifluormethyl, (C₃-C₇)-Cycloalkyl, Hydroxy, (C₁-C₄)-Alkoxy, Hydroxycarbonyl, (C₁-C₄)-Alkoxycarbonyl, Amino, Mono-(C₁-C₄)-alkylamino, Di-(C₁-C₄)-alkylamino, Aminocarbonyl, Mono-(C₁-C₄)-alkylaminocarbonyl, Di-(C₁-C₄)-alkylaminocarbonyl und 5- oder 6-gliedriges Heterocyclyl substituiert sein kann,
worin (C₁-C₄)-Alkoxy mit 1 oder 2 Substituenten unabhängig voneinander ausgewählt aus der Gruppe Hydroxy und (C₁-C₄)-Alkoxy substituiert sein kann
und
worin 5- oder 6-gliedriges Heterocyclyl mit einem Substituenten ausgewählt aus der Gruppe Oxo und (C₁-C₄)-Alkyl substituiert sein kann
und
wobei (C₃-C₇)-Cycloalkyl und 4- bis 6-gliedriges Heterocyclyl mit 1 oder 2 Substituenten unabhängig voneinander ausgewählt aus der Gruppe (C₁-C₄)-Alkyl, Hydroxy, (C₁-C₄)-Alkoxy, Hydroxycarbonyl, (C₁-C₄)-Alkoxycarbonyl, Amino, Mono-(C₁-C₄)-alkylamino und Di-(C₁-C₄)-alkylamino substituiert sein können,
sowie ihre N-Oxide, Salze, Solvate, Salze der N-Oxide und Solvate der N-Oxide und Salze.

Erfindungsgemäße Verbindungen sind die Verbindungen der Formel (I) und deren N-Oxide, Salze, Solvate, Salze der N-Oxide und Solvate der Salze und N-Oxide, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit 1 bis 6 bzw. 1 bis 4 Kohlenstoffatomen. Bevorzugt ist ein linearer oder verzweigter Alkylrest mit 1 bis 4 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl und n-Hexyl.
Cycloalkyl steht im Rahmen der Erfindung für einen monocyclischen, gesättigten Carbocyclus mit 3 bis 7 bzw. 5 bis 6 Ring-Kohlenstoffatomen. Beispielhaft und vorzugsweise seien genannt: Cyclopropyl, Cyclobutyl, Cyclopentyl, Cyclohexyl und Cycloheptyl.
Alkylcarbonyl steht im Rahmen der Erfindung für einen linearen oder verzweigten Alkylrest mit 1 bis 6 bzw. 1 bis 4 Kohlenstoffatomen und einer in 1-Position angebundenen Carbonylgruppe. Beispielhaft und vorzugsweise seien genannt: Methylcarbonyl, Ethylcarbonyl, n-Propylcarbonyl, iso-Propylcarbonyl, n-Butylcarbonyl, iso-Butylcarbonyl und tert.-Butylcarbonyl.
Alkoxy steht im Rahmen der Erfindung für einen linearen oder verzweigten Alkoxyrest mit 1 bis 6 bzw. 1 bis 4 bzw. 2 bis 4 Kohlenstoffatomen. Bevorzugt ist ein linearer oder verzweigter Alkoxyrest mit 1 bis 4 bzw. 2 bis 4 Kohlenstoffatomen. Beispielhaft und vorzugsweise seien genannt: Methoxy, Ethoxy, n-Propoxy, Isopropoxy, n-Butoxy, tert.-Butoxy, n-Pentoxy und n-Hexoxy.
Cycloalkoxy steht im Rahmen der Erfindung für einen monocyclischen, gesättigten Carbocyclus mit 3 bis 7 Kohlenstoffatomen, der über ein Sauerstoffatom gebunden ist. Beispielhaft und vorzugsweise seien genannt: Cyclopropyloxy, Cyclobutyloxy, Cyclopentyloxy, Cyclohexyloxy und Cycloheptyloxy.
Alkoxycarbonyl stehen im Rahmen der Erfindung für einen linearen oder verzweigten Alkoxyrest mit 1 bis 6 bzw. 1 bis 4 Kohlenstoffatomen und einer am Sauerstoff angebundenen Carbonylgruppe. Bevorzugt ist ein linearer oder verzweigter Alkoxycarbonylrest mit 1 bis 4 Kohlenstoffatomen in der Alkoxy-Gruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Mono-alkylamino steht im Rahmen der Erfindung für eine Amino-Gruppe mit einem linearen oder verzweigten Alkylsubstituenten, der 1 bis 6 bzw. 1 bis 4 Kohlenstoffatome aufweist. Bevorzugt ist ein linearer oder verzweigter Monoalkylamino-Rest mit 1 bis 4 Kohlenstoffatomen. Beispielhaft und vorzugsweise seien genannt: Methylamino, Ethylamino, n-Propylamino, Isopropylamino, n-Butylamino, tert.-Butylamino, n-Pentylamino und n-Hexylamino.
Di-alkylamino steht im Rahmen der Erfindung für eine Amino-Gruppe mit zwei gleichen oder verschiedenen linearen oder verzweigten Alkylsubstituenten, die jeweils 1 bis 6 bzw. 1 bis 4 Kohlenstoffatome aufweisen. Bevorzugt sind lineare oder verzweigte Dialkylamino-Reste mit jeweils 1 bis 4 Kohlenstoffatomen. Beispielhaft und vorzugsweise seien genannt: *N,N-*Dimethylamino, *N,N-*Diethylamino, *N*-Ethyl-*N*-methylamino, *N*-Methyl-*N*-n-propylamino, *N*-Isopropyl-*N*-n-propylamino, *N,N*-Diisopropylamino, *N*-n-Butyl-*N*-methylamino, *N*-tert.-Butyl-*N-*methylamino, *N*-Ethyl-*N*-n-pentylamino und *N*-n-Hexyl-*N*-methylamino.
Cycloalkylamino steht im Rahmen der Erfindung für eine Amino-Gruppe mit einem monocyclischen, gesättigten Carbocyclus mit 3 bis 7 Kohlenstoffatomen. Beispielhaft und vorzugsweise seien genannt: Cyclopropylamino, Cyclobutylamino, Cyclopentylamino, Cyclohexylamino und Cycloheptylamino.
Mono-alkylaminocarbonyl steht im Rahmen der Erfindung für eine Amino-Gruppe, die über eine Carbonylgruppe verknüpft ist und die einen linearen oder verzweigten Alkylsubstituenten mit 1 bis 6 bzw. 1 bis 4 Kohlenstoffatomen aufweist. Bevorzugt ist ein Mono-alkylaminocarbonyl-Rest mit 1 bis 4 Kohlenstoffatomen in der Alkylgruppe. Beispielhaft und vorzugsweise seien genannt: Methylaminocarbonyl, Ethylaminocarbonyl, *n*-Propylaminocarbonyl, Isopropylaminocarbonyl, *n-*Butylaminocarbonyl, *tert*.-Butylaminocarbonyl, *n*-Pentylaminocarbonyl und *n-*Hexylaminocarbonyl.
Di-alkylaminocarbonyl steht im Rahmen der Erfindung für eine Amino-Gruppe, die über eine Carbonylgruppe verknüpft ist und die zwei gleiche oder verschiedene lineare oder verzweigte Alkylsubstituenten mit jeweils 1 bis 6 bzw. 1 bis 4 Kohlenstoffatomen aufweist. Bevorzugt ist ein Dialkylaminocarbonyl-Rest mit jeweils 1 bis 4 Kohlenstoffatomen pro Alkylgruppe. Beispielhaft und vorzugsweise seien genannt: *N,N*-Dimethylaminocarbonyl, *N,N-*Diethylaminocarbonyl, *N* Ethyl-*N*-methylaminocarbonyl, N-Methyl-*N*-*n*-propylaminocarbonyl, *N*-*n*-Butyl-*N*-methylaminocarbonyl, *N*-tert.-Butyl-N-methylaminocarbonyl, *N*-*n*-Pentyl-*N*-methylarminocarbonyl und *N-n*-Hexyl-*N*-methylaminocarbonyl.
Cycloalkylaminocarbonyl steht im Rahmen der Erfindung für eine Amino-Gruppe, die über eine Carbonylgruppe verknüpft ist und die einen monocyclischen, gesättigten Carbocyclus mit 3 bis 7 Kohlenstoffatomen aufweist. Beispielhaft und vorzugsweise seien genannt: Cyclopropylaminocarbonyl, Cyclobutylaminocarbonyl, Cyclopentylaminocarbonyl, Cyclohexylaminocarbonyl und Cycloheptylaminocarbonyl.
Mono-alkylaminosulfonyl steht im Rahmen der Erfindung für eine Amino-Gruppe, die über eine Sulfonylgruppe verknüpft ist und die einen linearen oder verzweigten Alkylsubstituenten mit 1 bis 6 Kohlenstoffatomen aufweist. Beispielhaft und vorzugsweise seien genannt: Methylaminosulfonyl, Ethylaminosulfonyl, *n*-Propylaminosulfonyl, Isopropylaminosulfonyl, *n*-Butylaminosulfonyl und *tert*.-Butylaminosulfonyl.
Di-alkylaminosulfonyl steht im Rahmen der Erfindung für eine Amino-Gruppe, die über eine Sulfonylgruppe verknüpft ist und die zwei gleiche oder verschiedene lineare oder verzweigte Alkylsubstituenten mit jeweils 1 bis 6 Kohlenstoffatomen aufweist. Beispielhaft und vorzugsweise seien genannt: *N,N*-Dimethylaminosulfonyl, *N,N-*Diethylaminosulfonyl, *N*-Ethyl-*N*-methylaminosulfonyl, *N*-Methyl-*N*-*n*-propylaminosulfonyl, *N*-*n*-Butyl-*N*-methylaminosulfonyl und *N-tert.-*Butyl-*N*-methylaminosulfonyl.
Alkylsulfonyl steht in Rahmen der Erfindung für einen linearen oder verzweigten Alkylrest mit 1 bis 4 Kohlenstoffatomen, der über eine Sulfonylgruppe gebunden ist. Beispielhaft und vorzugsweise seinen genannt: Methylsulfonyl, Ethylsulfonyl, n-Propylsulfonyl, iso-Propylsulfonyl, n-Butylsulfonyl und tert.-Butylsulfonyl.
Cycloalkylsulfonyl steht in Rahmen der Erfindung für einen monocyclischen, gesättigten Carbocyclus mit 3 bis 7 Kohlenstoffatomen, der über eine Sulfonylgruppe gebunden ist. Beispielhaft und vorzugsweise seinen genannt: Cyclopropylsulfonyl, Cyclobutylsulfonyl, Cyclopentylsulfonyl, Cyclohexylsulfonyl und Cycloheptylsulfonyl.
Alkylsulfonylamino steht im Rahmen der Erfindung für eine Amino-Gruppe mit einem linearen oder verzweigten Alkylsulfonyl-Substituenten, der 1 bis 6 Kohlenstoffatome aufweist und über die Sulfonylgruppe mit dem N-Atom verknüpft ist. Beispielhaft und vorzugsweise seien genannt: Methylsulfonylamino, Ethylsulfonylamino, *n*-Propylsulfonylamino, Isopropylsulfonylamino, *n-*Butylsulfonylamino, *tert*.-Butylsulfonylamino, *n*-Pentylsulfonylamino und *n*-Hexylsulfonylamino.
Aryl steht im Rahmen der Erfindung für einen aromatischen Carbocyclus mit 6 oder 10 Ring-Kohlenstoffatomen. Bevorzugte Arylreste sind Phenyl und Naphthyl.
Heterocyclyl steht im Rahmen der Erfindung für einen gesättigten Heterocyclus mit insgesamt 4 bis 6 Ringatomen, der ein oder zwei Ring-Heteroatome aus der Reihe N, O und/oder S enthält und über ein Ring-Kohlenstoffatom oder gegebenenfalls ein Ring-Stickstoffatom verknüpft ist. Beispielhaft seien genannt: Azetidinyl, Pyrrolidinyl, Pyrazolidinyl, Tetrahydrofuranyl, Piperidinyl, Piperazinyl, Tetrahydropyranyl, Morpholinyl und Thiomorpholinyl. Bevorzugt sind Azetidinyl, Pyrrolidinyl, Tetrahydrofuranyl, Piperidinyl, Piperazinyl, Tetrahydropyranyl und Morpholinyl. Heteroaryl steht im Rahmen der Erfindung für einen monocyclischen oder gegebenenfalls bicyclischen aromatischen Heterocyclus (Heteroaromaten) mit insgesamt 5 bis 10 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl, Triazinyl, Benzofuranyl, Benzothienyl, Benzimidazolyl, Benzoxazolyl, Benzothiazolyl, Benzotriazolyl, Indolyl, Indazolyl, Chinolinyl, Isochinolinyl, Naphthyridinyl, Chinazolinyl, Chinoxalinyl, Phthalazinyl, Pyrazolo[3,4-b]pyridinyl. Bevorzugt sind monocyclische 5- oder 6-gliedrige Heteroaryl-Reste mit bis zu zwei Ring-Heteroatomen aus der Reihe N, O und/oder S wie beispielsweise Furyl, Thienyl, Thiazolyl, Oxazolyl, Isothiazolyl, Isoxazolyl, Pyrazolyl, Imidazolyl, Pyridyl, Pyrimidinyl, Pyridazinyl, Pyrazinyl.
Halogen schließt im Rahmen der Erfindung Fluor, Chlor, Brom und Iod ein. Bevorzugt sind Chlor oder Fluor.
Eine Oxo-Gruppe steht im Rahmen der Erfindung für ein Sauerstoffatom, das über eine Doppelbindung an ein Kohlenstoffatom gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem oder zwei gleichen oder verschiedenen Substituenten.

Bevorzugt sind im Rahmen der vorliegenden Erfindung Verbindungen der Formel (I), in welcher
- X: für S steht,
- R¹: für Phenyl oder 5- oder 6-gliedriges Heteroaryl steht,
wobei Phenyl und 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Trifluormethyl, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, Aminocarbonyl, Mono-(C₁-C₄)-alkylaminocarbonyl, Di-(C₁-C₄)-alkylaminocarbonyl, (C₃-C₆)-Cycloalkylaminocarbonyl, (C₁-C₄)-Alkylsulfonylamino, Morpholino, Piperazino, *N'*-(C₁-C₄)-Alkylpiperazino und -L-R⁵ substituiert sein können,
worin
L für eine Bindung oder NH steht,
R⁵ für Phenyl oder 5- oder 6-gliedriges Heteroaryl steht,
worin Phenyl und 5- oder 6-gliedriges Heteroaryl ihrerseits mit 1 oder 2 Substituenten unabhängig voneinander ausgewählt aus der Gruppe Fluor, Chlor, Cyano, (C₁-C₄)-Alkyl, Trifluormethyl, (C₁-C₄)-Alkoxy, Trifluormethoxy, Amino, Hydroxycarbonyl und (C₁-C₄)-Alkoxycarbonyl sein können,
- R²: für Wasserstoff oder Methyl steht,
- R³: für Phenyl, Pyrrolyl, Oxazolyl, Thiazolyl, Isoxazolyl, Pyrazolyl, Imidazolyl und Pyridyl steht,
wobei Phenyl, Pyrrolyl, Oxazolyl, Thiazolyl, Isoxazolyl, Pyrazolyl, Imidazolyl und Pyridyl mit 1 oder 2 Substituenten unabhängig voneinander ausgewählt aus der Gruppe Fluor, (C₁-C₆)-Alkyl, Hydroxy, (C₁-C₄)-Alkoxy und -NR^{A}R^{B} substituiert sein können,
worin (C₁-C₆)-Alkyl und (C₁-C₄)-Alkoxy mit 1 bis 3 Substituenten unabhängig voneinander ausgewählt aus der Gruppe Fluor, Trifluormethyl, Hydroxy, Methoxy, Ethoxy, Hydroxycarbonyl, Amino, Methylamino, Ethylamino, *N,N*-Dimethylamino und *N,N-*Diethylamino substituiert sein können,
und
worin
R^{A} für Wasserstoff oder (C₁-C₄)-Alkyl steht,
worin (C₁-C₄)-Alkyl seinerseits mit einem Substituenten ausgewählt aus der Gruppe Hydroxy und (C₁-C₄)-Alkoxy substituiert sein kann,
R^{B} für Wasserstoff oder (C₁-C₄)-Alkyl steht,
worin (C₁-C₄)-Alkyl seinerseits mit 1 oder 2 Substituenten unabhängig voneinander ausgewählt aus der Gruppe Hydroxy, (C₁-C₄)-Alkoxy und Hydroxycarbonyl substituiert sein kann,
- R⁴: für (C₁-C₆)-Alkyl, (C₄-C₆)-Cycloalkyl oder 5- oder 6-gliedriges Heterocyclyl steht,
wobei (C₁-C₆)-Alkyl mit 1 oder 2 Substituenten unabhängig voneinander ausgewählt aus der Gruppe Fluor, Trifluormethyl, (C₃-C₇)-Cyloalkyl, Hydroxy, Methoxy, Ethoxy, Hydroxycarbonyl, Methoxycarbonyl, Ethoxycarbonyl, Amino, Methylamin, Ethylamino, *N,N-*Dimethylamino, *N,N-*Diethylamino und 5- oder 6-gliedriges Heterocyclyl substituiert sein kann,
worin 5- oder 6-gliedriges Heterocyclyl seinerseits mit einem Substituenten ausgewählt aus der Gruppe Oxo und Methyl substituiert sein kann,
und
wobei (C₄-C₆)-Cycloalkyl und 5- oder 6-gliedriges Heterocyclyl mit 1 oder 2 Substituenten unabhängig voneinander ausgewählt aus der Gruppe Methyl, Hydroxy, Methoxy, Hydroxycarbonyl, Methoxycarbonyl, Ethoxycarbonyl, Amino, Methylamino und *N,N-*Dimethylamino substituiert sein können,
sowie ihre Salze, Solvate und Solvate der Salze.

Bevorzugt sind im Rahmen der vorliegenden Erfindung auch Verbindungen der Formel (I), in welcher
- X: für O oder S steht,
- R¹: für Phenyl, Thiazolyl, Oxazolyl oder Pyridyl steht,
wobei Phenyl und Pyridyl mit 1 oder 2 Substituenten unabhängig voneinander ausgewählt aus der Gruppe Fluor, Chlor, Cyano, Methyl, Trifluormethyl, Methoxy, Hydroxycarbonyl, Methoxycarbonyl, Ethoxycarbonyl, Aminocarbonyl, Methylaminocarbonyl, Ethylaminocarbonyl, *N,N-*Dimethylaminocarbonyl und *N,N-*Diethylaminocarbonyl substituiert sind,
und
wobei Thiazolyl und Oxazolyl mit einem Substituenten -L-R⁵ substituiert sind,
worin
L für eine Bindung oder NH steht,
R⁵ für Phenyl steht,
worin Phenyl seinerseits mit 1 oder 2 Substituenten unabhängig voneinander ausgewählt aus der Gruppe Fluor, Chlor, Methyl, Methoxy, Ethoxy, Hydroxycarbonyl, Methoxycarbonyl und Ethoxycarbonyl sein kann, und
wobei Thiazolyl und Oxazolyl mit einem Substituenten ausgewählt aus der Gruppe Fluor, Methyl, Ethyl, Methoxy, Hydroxycarbonyl und Methoxycarbonyl substituiert sein können,
- R²: für Wasserstoff oder Methyl steht,
- R³: für Phenyl, Pyrrolyl, Oxazolyl, Thiazolyl, Isoxazolyl, Pyrazolyl, Imidazolyl und Pyridyl steht,
wobei Phenyl, Pyrrolyl, Oxazolyl, Thiazolyl, Isoxazolyl, Pyrazolyl, Imidazolyl und Pyridyl mit 1 oder 2 Substituenten unabhängig voneinander ausgewählt aus der Gruppe Fluor, (C₁-C₆)-Alkyl, Hydroxy, (C₁-C₄)-Alkoxy und -NR^{A}R^{B} substituiert sein können,
worin (C₁-C₆)-Alkyl und (C₁-C₄)-Alkoxy mit 1 bis 3 Substituenten unabhängig voneinander ausgewählt aus der Gruppe Fluor, Trifluormethyl, Hydroxy, Methoxy, Ethoxy, Hydroxycarbonyl, Amino, Methylamino, Ethylamino, *N,N*-Dimethylamino und *N,N*-Diethylamino substituiert sein können,
und
worin
R^{A} für Wasserstoff oder (C₁-C₄)-Alkyl steht,
worin (C₁-C₄)-Alkyl seinerseits mit einem Substituenten ausgewählt aus der Gruppe Hydroxy und (C₁-C₄)-Alkoxy substituiert sein kann,
R^{B} für Wasserstoff oder (C₁-C₄)-Alkyl steht,
worin (C₁-C₄)-Alkyl seinerseits mit 1 oder 2 Substituenten unabhängig voneinander ausgewählt aus der Gruppe Hydroxy, (C₁-C₄)-Alkoxy und Hydroxycarbonyl substituiert sein kann,
- R⁴: für (C₁-C₆)-Alkyl oder (C₄-C₆)-Cycloalkyl steht,
wobei (C₁-C₆)-Alkyl mit 1 oder 2 Substituenten unabhängig voneinander ausgewählt aus der Gruppe Fluor, Trifluormethyl, (C₃-C₇)-Cycloalkyl, Hydroxy, Methoxy und Ethoxy substituiert sein kann,
und
wobei (C₄-C₆)-Cycloalkyl mit 1 oder 2 Substituenten unabhängig voneinander ausgewählt aus der Gruppe Methyl, Hydroxy und Methoxy substituiert sein kann,
sowie ihre Salze, Solvate und Solvate der Salze.

Besonders bevorzugt sind im Rahmen der vorliegenden Erfindung Verbindungen der Formel (I), in welcher
- X: für S steht,
- R¹: für Phenyl, Thiazolyl, Oxazolyl oder Pyridyl steht,
wobei Phenyl und Pyridyl mit 1 oder 2 Substituenten unabhängig voneinander ausgewählt aus der Gruppe Fluor, Chlor, Cyano, Methyl, Trifluormethyl, Methoxy, Hydroxycarbonyl, Methoxycarbonyl, Ethoxycarbonyl, Aminocarbonyl, Methylaminocarbonyl, Ethylaminocarbonyl, *N,N*-Dimethylaminocarbonyl und *N,N-*Diethylaminocarbonyl substituiert sind,
und
wobei Thiazolyl und Oxazolyl mit einem Substituenten -L-R⁵ substituiert sind,
worin
L für eine Bindung oder NH steht,
R⁵ für Phenyl steht,
worin Phenyl seinerseits mit 1 oder 2 Substituenten unabhängig voneinander ausgewählt aus der Gruppe Fluor, Chlor, Methyl, Methoxy, Ethoxy, Hydroxycarbonyl, Methoxycarbonyl und Ethoxycarbonyl sein kann, und
wobei Thiazolyl und Oxazolyl mit einem Substituenten ausgewählt aus der Gruppe Fluor, Methyl, Ethyl, Methoxy, Hydroxycarbonyl und Methoxycarbonyl substituiert sein können,
- R²: für Wasserstoff steht,
- R³: für Phenyl oder Thiazolyl steht,
wobei Phenyl mit 1 oder 2 Substituenten unabhängig voneinander ausgewählt aus der Gruppe (C₁-C₆)-Alkyl, Hydroxy und (C₁-C₄)-Alkoxy substituiert sein kann,
worin (C₁-C₆)-Alkyl und (C₂-C₄)-Alkoxy mit 1 oder 2 Substituenten unabhängig voneinander ausgewählt aus der Gruppe Hydroxy und Methoxy substituiert sein können,
und
wobei Thiazolyl mit einem Substituenten (C₁-C₆)-Alkyl substituiert sein kann,
worin (C₁-C₆)-Alkyl mit 1 oder 2 Substituenten unabhängig voneinander ausgewählt aus der Gruppe Hydroxy und Methoxy substituiert sein kann,
- R⁴: für (C₁-C₄)-Alkyl steht,
wobei Alkyl mit 1 oder 2 Substituenten Hydroxy substituiert sein kann,
sowie ihre Salze, Solvate und Solvate der Salze.

Bevorzugt sind im Rahmen der vorliegenden Erfindung auch Verbindungen der Formel (I), in welcher
- R¹: für Phenyl oder Pyridyl steht,
wobei Phenyl und Pyridyl mit 1 oder 2 Substituenten unabhängig voneinander ausgewählt aus der Gruppe Fluor, Chlor, Cyano, Methyl, Trifluormethyl, Methoxy, Hydroxycarbonyl, Methoxycarbonyl, Ethoxycarbonyl, Aminocarbonyl, Methylaminocarbonyl, Ethylaminocarbonyl, Cyclopropylaminocarbonyl, *N,N-*Dimethylaminocarbonyl und *N,N-*Diethylaminocarbonyl substituiert sind.

Bevorzugt sind im Rahmen der vorliegenden Erfindung auch Verbindungen der Formel (I), in welcher
- R¹: für Thiazolyl oder Oxazolyl steht,
wobei Thiazolyl und Oxazolyl mit einem Substituenten -L-R⁵ substituiert sind,
worin
L für eine Bindung oder NH steht,
R⁵ für Phenyl steht,
worin Phenyl seinerseits mit 1 oder 2 Substituenten unabhängig voneinander ausgewählt aus der Gruppe Fluor, Chlor, Methyl, Methoxy, Ethoxy, Hydroxycarbonyl, Methoxycarbonyl und Ethoxycarbonyl sein kann, und
wobei Thiazolyl und Oxazolyl mit einem Substituenten ausgewählt aus der Gruppe Fluor, Methyl, Methoxy, Hydroxycarbonyl und Methoxycarbonyl substituiert sein können.

Bevorzugt sind im Rahmen der vorliegenden Erfindung auch Verbindungen der Formel (I), in welcher
- R³: für Phenyl steht,
wobei Phenyl mit 1 oder 2 Substituenten unabhängig voneinander ausgewählt aus der Gruppe Hydroxy, (C₁-C₆)-Alkyl und (C₁-C₄)-Alkoxy substituiert sein kann,
worin (C₁-C₆)-Alkyl und (C₂-C₄)-Alkoxy mit 1 oder 2 Substituenten unabhängig voneinander ausgewählt aus der Gruppe Hydroxy und Methoxy substituiert sein können.

Bevorzugt sind im Rahmen der vorliegenden Erfindung auch Verbindungen der Formel (I), in welcher
- R³: für eine Gruppe der Formel 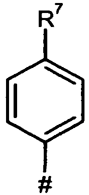 steht, wobei
- #: für die Anknüpfstelle an die 4-Position des Pyridins steht,
- R⁷: für Hydroxy, (C₁-C₆)-Alkyl oder (C₁-C₄)-Alkoxy steht,
worin (C₁-C₆)-Alkyl und (C₂-C₄)-Alkoxy mit 1 oder 2 Substituenten unabhängig voneinander ausgewählt aus der Gruppe Hydroxy und Methoxy substituiert sein können.

Bevorzugt sind im Rahmen der vorliegenden Erfindung auch Verbindungen der Formel (I), in welcher
- R⁴: für (C₁-C₆)-Alkyl, (C₄-C₆)-Cycloalkyl oder 5- oder 6-gliedriges Heterocyclyl steht,
wobei (C₁-C₆)-Alkyl mit 1 oder 2 Substituenten unabhängig voneinander ausgewählt aus der Gruppe Fluor, Trifluormethyl, (C₃-C₇)-Cycloalkyl, Hydroxy, Methoxy, Ethoxy, Hydroxycarbonyl, Methoxycarbonyl, Ethoxycarbonyl, Amino, Methylamino, Ethylamino, *N,N*-Dimethylamino, *N,N*-Diethylamino und 5- oder 6-gliedriges Heterocyclyl substituiert sein kann,
worin Ethoxy seinerseits mit einem Substituenten ausgewählt aus der Gruppe Hydroxy und Methoxy substituiert sein kann,
und
wobei (C₃-C₇)-Cycloalkyl und 5- oder 6-gliedriges Heterocyclyl mit 1 oder 2 Substituenten unabhängig voneinander ausgewählt aus der Gruppe Methyl, Hydroxy, Methoxy, Hydroxycarbonyl, Methoxycarbonyl, Ethoxycarbonyl, Amino, Methylamino und *N,N-*Dimethylamino substituiert sein können.

Bevorzugt sind im Rahmen der vorliegenden Erfindung auch Verbindungen der Formel (I), in welcher X für O oder S steht.

Bevorzugt sind im Rahmen der vorliegenden Erfindung auch Verbindungen der Formel (I), in welcher X für S steht.

Bevorzugt sind im Rahmen der vorliegenden Erfindung auch Verbindungen der Formel (I), in welcher X für O steht.

Bevorzugt sind im Rahmen der vorliegenden Erfindung auch Verbindungen der Formel (I), in welcher R² für Wasserstoff steht.

Bevorzugt sind im Rahmen der vorliegenden Erfindung auch Verbindungen der Formel (I), in welcher R² für Methyl steht.

Bevorzugt sind im Rahmen der vorliegenden Erfindung auch Verbindungen der Formel (I), in welcher
- R⁴: für (C₁-C₄)-Alkyl steht,
wobei Alkyl mit 1 oder 2 Substituenten Hydroxy substituiert sein kann.

Weiterer Gegenstand der vorliegenden Erfindung ist ein Verfahren zur Herstellung der erfndungsgemäßen Verbindungen der Formel (I), dadurch gekennzeichnet, dass man

### [A] eine Verbindung der Formel (II-A)

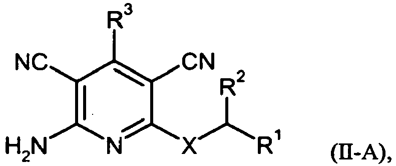

in welcher X, R¹, R² und R³ jeweils die oben angegebenen Bedeutungen haben,
zunächst mit Kupfer(II)chlorid und Isoamylnitrit in einem geeigneten Lösungsmittel in eine Verbindung der Formel (III-A) 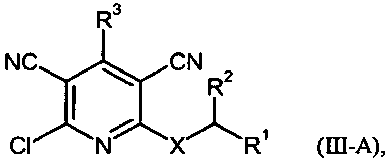 in welcher X, R¹, R² und R³ jeweils die oben angegebenen Bedeutungen haben,
überführt, und diese anschliessend in einem inerten Lösungsmittel in Gegenwart einer geeigneten Base mit einer Verbindung der Formel (IV)

R⁴—OH (IV),

in welcher R⁴ die oben angegebene Bedeutung hat,
umsetzt,
oder

### [B] im Fall, dass X für S steht, eine Verbindung der Formel (II-B)

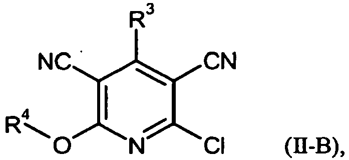

in welcher R³ und R⁴ jeweils die oben angegebenen Bedeutungen haben,
in einem inerten Lösungsmittel mit einem Alkalisulfid zu einer Verbindung der Formel (III-B) 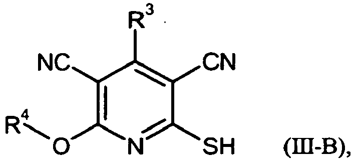 in welcher R³ und R⁴ jeweils die oben angegebenen Bedeutungen haben,
umsetzt, und diese anschliessend in einem inerten Lösungsmittel in Gegenwart einer Base mit einer Verbindung der Formel (V) 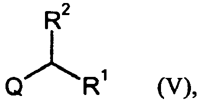 in welcher R¹ und R² die oben angegebenen Bedeutungen haben und
- Q: für eine geeignete Abgangsgruppe, vorzugsweise für Halogen, insbesondere Chlor, Brom oder Iod, oder für Mesylat, Tosylat oder Triflat steht,
umsetzt,
anschließend gegebenenfalls vorhandene Schutzgruppen abspaltet und die resultierenden Verbindungen der Formel (I) gegebenenfalls mit den entsprechenden (*i*) Lösungsmitteln und/oder (*ii*) Basen oder Säuren in ihre Solvate, Salze und/oder Solvate der Salze überführt.

In den Verbindungen der Formeln (II-A) und (II-B) bzw. in den Resten R³ und/oder R⁴ gegebenenfalls vorhandene funktionelle Gruppen - wie insbesondere Amino-, Hydroxy- und Carboxylgruppen - können bei diesem Verfahren, falls zweckmäßig oder erforderlich, auch in temporär geschützter Form vorliegen. Die Einführung und Entfernung solcher Schutzgruppen erfolgt hierbei nach üblichen, dem Fachmann bekannten Methoden [siehe z.B. T.W. Greene und P.G.M. Wuts, Protective Groups in Organic Synthesis, Wiley, New York, 1999; M. Bodanszky und A. Bodanszky, The Practice of Peptide Synthesis, Springer-Verlag, Berlin, 1984]. Bei Vorhandensein mehrerer Schutzgruppen kann die Entfernung gegebenenfalls simultan in einer Eintopf-Reaktion oder in separaten Reaktionsschritten vorgenommen werden.

Als Amino-Schutzgruppe wird bevorzugt *tert*.-Butoxycarbonyl (Boc) oder Benzyloxycarbonyl (Z) verwendet. Zum Schutz von Carboxylgruppen eignen sich insbesondere die entsprechenden Methyl-, Ethyl- oder *tert*.-Butylester. Für eine Hydroxy-Funktion wird als Schutzgruppe vorzugsweise Benzyl oder eine Silylgruppe wie Trimethylsilyl, *tert*.-Butyldimethylsilyl oder Dimethylphenylsilyl eingesetzt. Bei Vorliegen einer 1,2- oder 1,3-Diol-Gruppierung wird bevorzugt ein von symmetrischen Ketonen wie Aceton oder Cyclohexanon abgeleitetes Ketal (1,3-Dioxolan bzw. 1,3-Dioxan) als gemeinsame Schutzgruppe verwendet.

Das zuvor beschriebene Verfahren kann durch die folgenden Reaktionsschemata 1 und 2 beispielhaft erläutert werden:

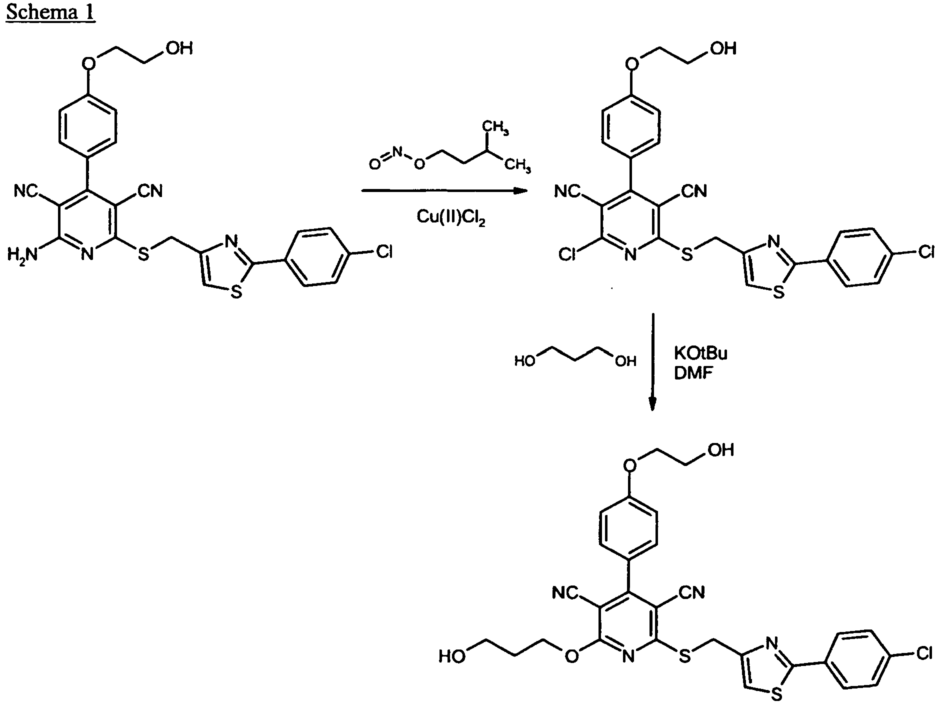

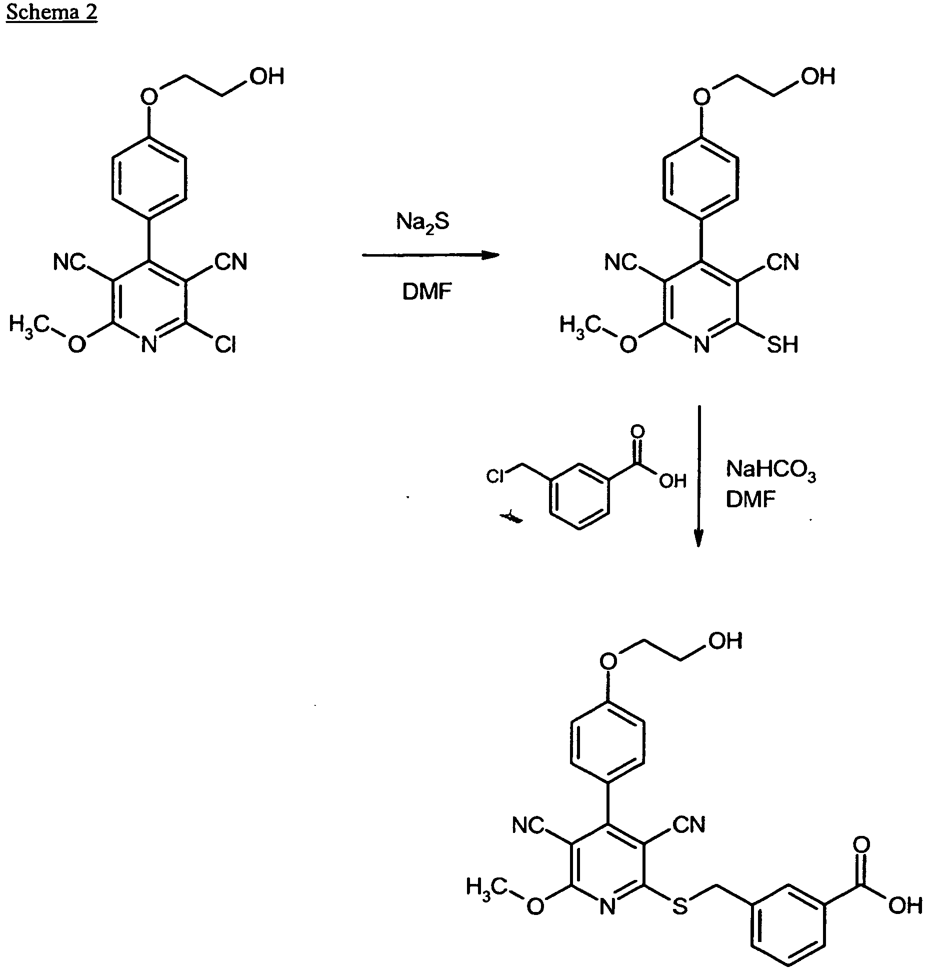

Als Lösungsmittel für die Reaktion (III-A) + (IV) eignen sich alle organischen Lösungsmittel, die unter den Reaktionsbedingungen inert sind. Hierzu gehören Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, Trichlormethan und Chlorbenzol, oder andere Lösungsmittel wie Dimethylformamid (DMF), Dimethylsulfoxid (DMSO), *N*-Methylpyrrolidinon (NMP), Acetonitril oder Pyridin. Ebenso ist es möglich, Gemische der zuvor genannten Lösungsmittel einzusetzen. Bevorzugt wird Dimethylformamid verwendet.

Als Basen für diese Umsetzung eignen sich die üblichen anorganischen oder organischen Basen. Hierzu gehören bevorzugt Alkalihydride wie Natriumhydrid, Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN) sowie Phosphazen-Basen (so genannte "Schwesinger-Basen") wie beispielsweise P2-t-Bu oder P4-t-Bu. Bevorzugt sind Cäsiumcarbonat, Kalium-tert.butylat, Natriumhydrid und P4-tBu.

Die Base kann hierbei in einer Menge von 1 bis 10 Mol, bevorzugt von 1 bis 5 Mol, insbesondere von 1 bis 3 Mol, bezogen auf 1 Mol der Verbindung der Formel (IV), eingesetzt werden.

Die Reaktion (III-A) + (IV) erfolgt im Allgemeinen in einem Temperaturbereich von -78°C bis +140°C, bevorzugt im Bereich von -20°C bis +100°C, insbesondere bei 0°C bis +60°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Der Verfahrensschritt (B-A) → (III-A) erfolgt im Allgemeinen mit einem Molverhältnis von 2 bis 12 Mol Kupfer(II)chlorid und 2 bis 12 Mol Isoamylnitrit bezogen auf 1 Mol der Verbindung der Formel (II-A).

Als Lösungsmittel für diesen Verfahrensschritt eignen sich alle organischen Lösungsmittel, die unter den Reaktionsbedingungen inert sind. Hierzu gehören acyclische und cyclische Ether wie Diethylether und Tetrahydrofuran, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder andere Lösungsmittel wie Dimethylformamid, Acetonitril oder Pyridin. Ebenso ist es möglich, Gemische dieser Lösungsmittel einzusetzen. Bevorzugte Lösungsmittel sind Acetonitril und Dimethylformamid.

Die Reaktion erfolgt im Allgemeinen in einem Temperaturbereich von -78°C bis +180°C, bevorzugt im Bereich von +20°C bis +100°C, insbesondere bei +20°C bis +60°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Als Lösungsmittel für die Reaktion (III-B) + (V) eignen sich alle organischen Lösungsmittel, die unter den Reaktionsbedingungen inert sind. Hierzu gehören Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, Trichlormethan und Chlorbenzol, oder andere Lösungsmittel wie Dimethylformamid (DMF), Dimethylsulfoxid (DMSO), *N*-Methylpyrrolidinon (NMP), Acetonitril oder Pyridin. Ebenso ist es möglich, Gemische der zuvor genannten Lösungsmittel einzusetzen. Bevorzugt wird Dimethylformamid verwendet.

Als Basen für diese Umsetzung eignen sich die üblichen anorganischen oder organischen Basen. Hierzu gehören bevorzugt Alkalihydride wie Natriumhydrid, Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt wird Natriumhydrogencarbonat verwendet.

Die Base wird hierbei in der Regel in einer Menge von 1 bis 1.25 Mol, bevorzugt in äquimolarer Menge, bezogen auf 1 Mol der Verbindung der Formel (V), eingesetzt.

Die Reaktion (III-B) + (V) erfolgt im Allgemeinen in einem Temperaturbereich von -20°C bis +120°C, bevorzugt bei +20°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Bei der Umsetzung (II-B) → (III-B) wird als Alkalisulfid vorzugsweise Natriumsulfid in einer Menge von 1 bis 10 Mol, bevorzugt von 1 bis 8 Mol, insbesondere von 1 bis 5 Mol, bezogen auf 1 Mol der Verbindung der Formel (II-B), eingesetzt.

Als Lösungsmittel für diesen Verfahrensschritt eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder dipolare Lösungsmittel wie Acetonitril, Pyridin, Dimethylformamid, Dimethylsulfoxid oder *N*-Methylpyrrolidinon. Wasser ist als Lösungsmittel ebenfalls geeignet. Ebenso ist es möglich, Gemische der zuvor genannten Lösungsmittel einzusetzen. Bevorzugtes Lösungsmittel ist Dimethylformamid.

Die Reaktion erfolgt im Allgemeinen in einem Temperaturbereich von 0°C bis +180°C, bevorzugt im Bereich von +20°C bis +120°C, insbesondere bei +40°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Alternativ können Verbindungen der Formel (I), in welcher X für O steht, ausgehend von Verbindungen der Formel (II-B) durch Umsetzung mit Verbindungen der Formel (VI) 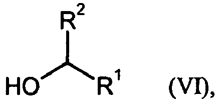 in welcher R¹ und R² die oben angegebenen Bedeutungen haben,
erhalten werden.

Als inerte Lösungsmittel für die Reaktion (II-B) + (VI) → (I) eignen sich insbesondere acyclische und cyclische Ether wie Diethylether, Methyl-tert.-butylether, 1,2-Dimethoxyethan, Tetrahydrofuran und Dioxan, Kohlenwasserstoffe wie Benzol, Toluol, Xylol, Hexan und Cyclohexan, oder andere Lösungsmittel wie Dimethylformamid (DMF), Dimethylsulfoxid (DMSO), N-Methylpyrrolidinon (NMP) und Pyridin. Ebenso ist es möglich, Gemische dieser Lösungsmittel einzusetzen. Bevorzugt wird Dimethylformamid verwendet.

Als Basen für diese Umsetzung sind insbesondere Alkalialkoholate wie Natrium- oder Kaliummethanolat, Natrium- oder Kaliumethanolat oder Natrium- oder Kalium-tert.-butylat, Alkalihydride wie Lithium-, Natrium- oder Kaliumhydrid, Amide wie Natriumamid, Lithium-, Natrium- oder Kalium-bis-(trimethylsilyl)amid oder Lithiumdiisopropylamid, oder metallorganische Verbindungen wie Butyllithium oder Phenyllithium geeignet. Bevorzugt wird Kalium-tert.-butylat verwendet.

Die Base wird hierbei in der Regel in einer Menge von 1 bis 1.25 Mol, bevorzugt in äquimolarer Menge, bezogen auf 1 Mol der Verbindung der Formel (VI), eingesetzt.

Die Reaktionen (II-B) + (VI) → (I) erfolgt im Allgemeinen in einem Temperaturbereich von -20°C bis +120°C, bevorzugt bei +20°C bis +100°C, gegebenenfalls in der Mikrowelle. Die Umsetzungen können bei normalem, erhöhtem oder erniedrigtem Druck durchgeführt werden (z.B. im Bereich von 0.5 bis 5 bar). Im Allgemeinen arbeitet man bei Normaldruck.

Die Verbindungen der Formel (IV) und (VI) sind kommerziell erhältlich, literaturbekannt oder nach literaturbekannten Methoden herstellbar.

Die Verbindungen der Formel (V) sind kommerziell erhältlich, literaturbekannt oder nach literaturbekannten Methoden herstellbar. So können beispielsweise durch Reaktion von Amiden, Thioamiden bzw. Thioharnstoff Derivaten mit einem 1,3-Dihalogenaceton 2-substituierte Oxazol-und Thiazol-Derivate der Formel (V-A), (V-B) bzw. (V-C) erhalten werden (siehe Schema 3):

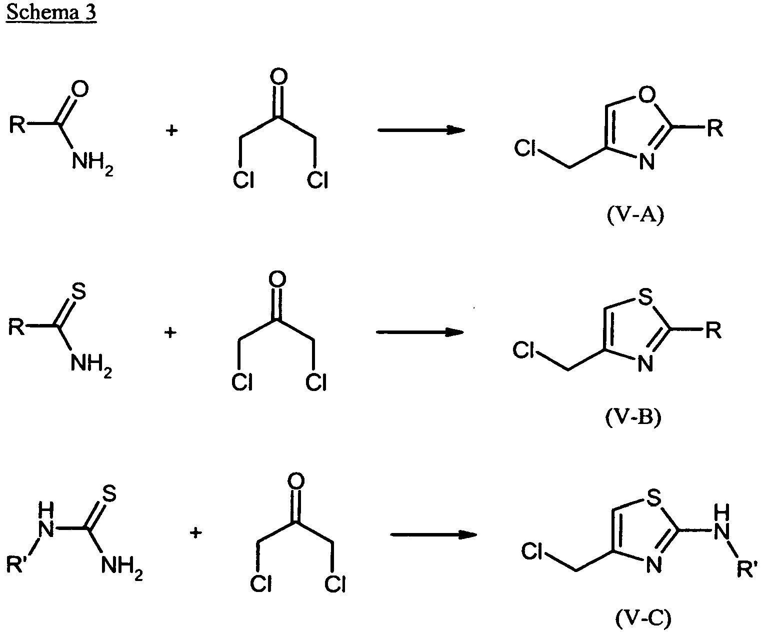

Im Falle der Verbindungen (V-C) können diese entweder analog zur Literatur hergestellt und isoliert werden [vgl. z.B. I. Simiti et al., Chem. Ber. 95, 2672-2679 (1962)], oder sie können *in situ* erzeugt und direkt weiter umgesetzt werden. Bevorzugt ist die *in situ*-Erzeugung unter Verwendung von 1,3-Dichloraceton in Dimethylformamid oder Ethanol als Lösungsmittel. Die Darstellung erfolgt im Allgemeinen in einem Temperaturbereich von 0°C bis +140°C, bevorzugt im Bereich von +20°C bis +120°C, insbesondere bei +60°C bis +100°C.

Verbindungen der Formel (II-A), worin X für S steht, können in Analogie zu literaturbekannten Methoden beispielsweise dadurch hergestellt werden, dass man Aldehyde der Formel (VII) 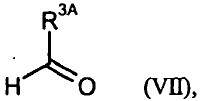 in welcher
- R^{3A}: für Phenyl oder C-gebundenes 5- oder 6-gliedriges Heteroaryl steht
wobei Phenyl und C-gebundenes 5- oder 6-gliedriges Heteroaryl im oben angegebenen Bedeutungsumfang substituiert sein kann,
in Gegenwart einer Base mit zwei Äquivalenten Cyanothioacetamid zu Verbindungen der Formel (VIII) 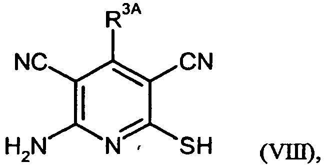 in welcher R^{3A} die oben angegebene Bedeutung hat,
umsetzt,
und diese anschliessend in einem inerten Lösungsmittel in Gegenwart einer Base mit einer Verbindung der Formel (V) umsetzt [siehe Schema 4; vgl. z.B. Dyachenko et al., Russ. J. Chem. 33 (7), 1014-1017 (1997), 34 (4), 557-563 (1998); Dyachenko et al., Chemistry of Heterocyclic Compounds 34 (2), 188-194 (1998); Qintela et al., Eur. J. Med. Chem. 33, 887-897 (1998); Kandeel et al., Z. Naturforsch. 42b, 107-111 (1987); Reddy et al., J. Med. Chem. 49, 607-615 (2006); Evdokimov et al., Org. Lett. 8, 899-902 (2006)].

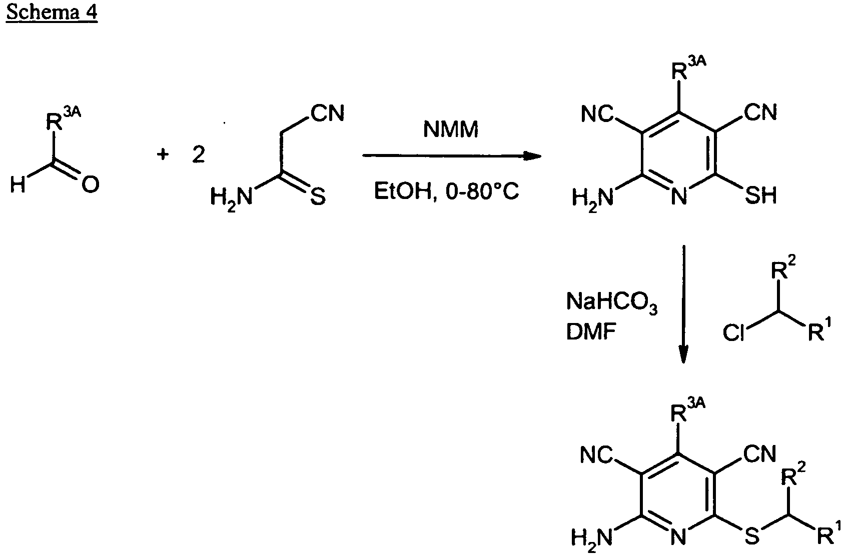

Die Verbindungen der Formel (VII) sind kommerziell erhältlich, literaturbekannt oder nach literaturbekannten Methoden herstellbar.

Für die Umsetzung (VIII) → (II-A) kommen die für den Verfahrensschritt (III-B) + (V) → (I) genannten Bedingungen zum Einsatz.

Verbindungen der Formel (II-A), worin X für O steht, können ausgehend von Verbindungen der Formel (IX) 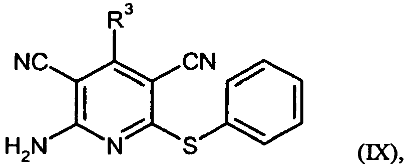 in welcher R³ die oben angegebene Bedeutung hat,
durch Umsetzung in einem inerten Lösungsmittel in Gegenwart einer geeigneten Base mit einer Verbindung der Formel (VI) erhalten werden.

Für diesen Verfahrensschritt kommen die für die Reaktion (II-B) + (VI) → (I) genannten Bedingungen zum Einsatz.

Diese Herstellungsmethode wird durch das folgende Reaktionsschema erläutert:

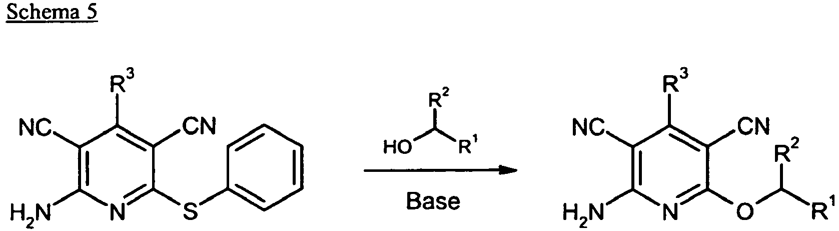

Die Verbindungen der Formel (IX) können in Analogie zu literaturbeschriebenen Verfahren hergestellt werden [vgl. z.B. Kambe et al., Synthesis, 531-533 (1981); Elnagdi et al., Z. Naturforsch. 47b, 572-578 (1991); Reddy et al., J. Med. Chem. 49, 607-615 (2006); Evdokimov et al., Org. Lett. 8, 899-902 (2006)] oder durch Umsetzung von Verbindungen der Formel (II), in welcher X für S steht, in Analogie zu literaturbeschriebenen Verfahren [vgl. z. B. Fujiwara, H. et al., Heterocycles 1993, 36 (5), 1105-1113, Su et al., J. Med Chem. 1988, 31, 1209-1215].

Verbindungen der Formel (II-B) können hergestellt werden, indem man Verbindungen der Formel (VII) in einem geeigneten Lösungsmittel in Gegenwart einer geeigneten Base mit 2 Äquivalenten Malonsäuredinitril und Verbindungen der Formel (X)

R⁴—O⁻ M⁺ (X),

in welcher R⁴ die oben angegebene Bedeutung hat und
- M⁺: für ein Alkaliion, vorzugsweise Natrium- oder Kaliumion, steht,
in Verbindungen der Formel (XI) 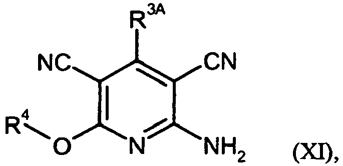 in welcher R^{3A} und R⁴ jeweils die oben angegebenen Bedeutungen haben,
überführt und diese anschliessend mit Kupfer(II)chlorid und Isoamylnitrit in einem geeigneten Lösungsmittel umsetzt.

Das beschriebene Verfahren wird durch das folgende Reaktionsschema beispielhaft erläutert:

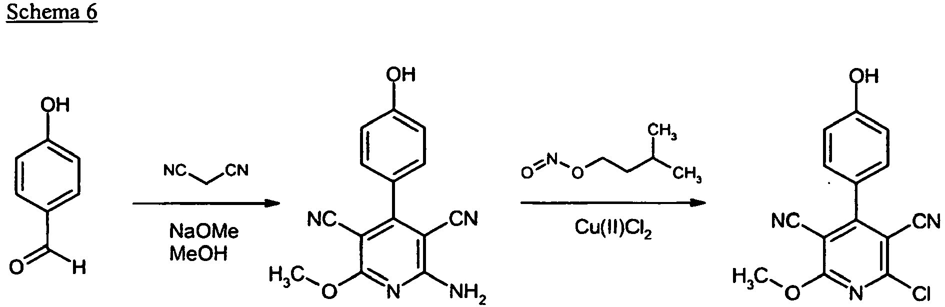

Weitere Verbindungen der Formel (II-A), in welcher X für S und steht, können hergestellt werden, indem man die Verbindung der Formel (XII) 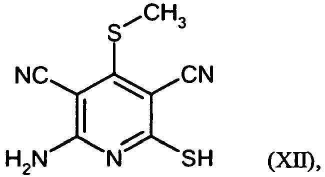 in einem inerten Lösungsmittel in Gegenwart einer Base mit einer Verbindung der Formel (V) in eine Verbindung der Formel (XIII) 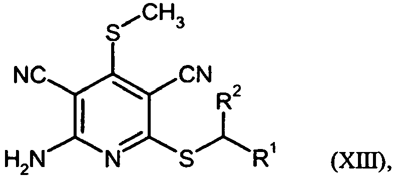 in welcher R¹ und R² die oben angegebenen Bedeutungen haben,
überführt und diese dann in einem inerten Lösungsmittel oder ohne Lösungsmittel mit einer Verbindung der Formel (XIV)

R^{3B}—H (XIV),

in welcher
- R^{3B}: für N-gebundenes 5- oder 6-gliedriges Heteroaryl steht,
wobei N-gebundenes 5- oder 6-gliedriges Heteroaryl in dem für R³ angegebenen Bedeutungsumfang substituiert sein kann,
umsetzt.

Die Umsetzung (XII) + (V) → (XIII) erfolgt unter den für den Verfahrensschritt (III-B) + (V) → (I) genannten Bedingungen.

Als Lösungsmittel für den Verfahrensschritt (XIII) + (XIV) eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan oder Chlorbenzol, oder andere Lösungsmittel wie Dimethylformamid (DMF), Dimethylsulfoxid (DMSO), *N*-Methylpyrrolidinon (NMP), Acetonitril oder Pyridin. Wasser ist als Lösungsmittel ebenfalls geeignet. Ebenso ist es möglich, Gemische der zuvor genannten Lösungsmittel einzusetzen. Gegebenenfalls kann die Reaktion auch vorteilhaft in Gegenwart eines Überschusses der Verbindung (XIV) ohne Zusatz eines weiteren Lösungsmittels durchgeführt werden. Bevorzugt wird die Umsetzung in Aceton oder *N*-Methylpyrrolidinon als Lösungsmittel durchgeführt.

Der Verfahrensschritt (XIII) + (XIV) erfolgt im Allgemeinen in einem Temperaturbereich von 0°C bis +180°C, bevorzugt im Bereich von +20°C bis +100°C, insbesondere bei +60°C bis +100°C, gegebenenfalls in der Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die Verbindungen der Formel (XIV) sind kommerziell erhältlich, literaturbekannt oder können in Analogie zu literaturbekannten Verfahren hergestellt werden.

Die Verbindung der Formel (XII) lässt sich auf einfache Weise durch Umsetzung von [Bis(methyl-thio)methylen]malononitril mit Cyanothioacetamid in Gegenwart einer Base wie Triethylamin erhalten.

Weitere Verbindungen der Formel (II-A), in welcher X für O steht, können hergestellt werden, indem man die Verbindung der Formel (XV) 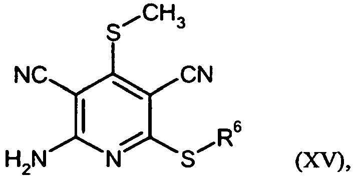 in welcher
- R⁶: für (C₁-C₄)-Alkyl oder Phenyl steht,
in einem inerten Lösungsmittel in Gegenwart einer Base mit einer Verbindung der Formel (VI) in eine Verbindung der Formel (XVI) 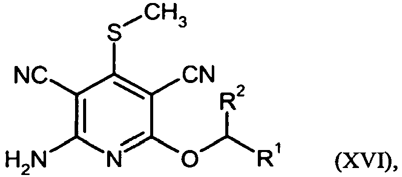 in welcher R' und R² die oben angegebenen Bedeutungen haben,
überführt und diese dann in einem inerten Lösungsmittel oder ohne Lösungsmittel mit einer Verbindung der Formel (XIV) umsetzt, oder
alternativ eine Verbindung der Formel (XV) zuerst in einem inerten Lösungsmittel oder ohne Lösungsmittel mit einer Verbindung der Formel (XIV) zu Verbindungen der Formel (XVII) 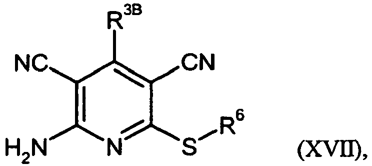 in welcher R^{3B} und R⁶ jeweils die oben angegebenen Bedeutungen haben,
umsetzt, und diese anschliessend in einem inerten Lösungsmittel in Gegenwart einer geeigneten Base mit einer Verbindung der Formel (VI) umsetzt.

Die Verbindungen der Formel (XV), in welcher R⁶ für Phenyl steht, lassen sich aus der Verbindung der Formel (XII) in Analogie zu dem in Fujiwara, H. et al., Heterocycles 1993, 36 (5), 1105-1113 beschriebenen Verfahren herstellen.

Die Verbindungen der Formel (XV), in welcher R⁶ für (C₁-C₄)-Alkyl steht, lassen sich aus der Verbindung der Formel (XII) in Analogie zu dem in Su et al., J. Med Chem. 1988, 31, 1209-1215 beschriebenen Verfahren herstellen.

Die Reaktion (XV) + (VI) wird unter den für den Verfahrensschritt (II-B) + (VI) → (I) genannten Bedingungen durchgeführt.

Als Lösungsmittel für die Verfahrensschritte (XV) bzw. (XVI) + (XIV) eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan oder Chlorbenzol, oder andere Lösungsmittel wie Dimethylformamid (DMF), Dimethylsulfoxid (DMSO), N-Methylpyrrolidinon (NMP), Acetonitril oder Pyridin. Wasser ist als Lösungsmittel ebenfalls geeignet. Ebenso ist es möglich, Gemische der zuvor genannten Lösungsmittel einzusetzen. Gegebenenfalls kann die Reaktion auch vorteilhaft in Gegenwart eines Überschusses der Verbindung (XIV) ohne Zusatz eines weiteren Lösungsmittels durchgeführt werden. Bevorzugt wird die Umsetzung in Aceton oder N-Methylpyrrolidinon als Lösungsmittel durchgeführt.

Die Verfahrensschritte (XV) bzw. (XVI) + (XIV) erfolgen im Allgemeinen in einem Temperaturbereich von 0°C bis +180°C, bevorzugt im Bereich von +20°C bis +100°C, insbesondere bei +60°C bis +100°C, gegebenenfalls in der Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die zuvor beschriebenen Verfahren werden in den folgenden Schemata erläutert:

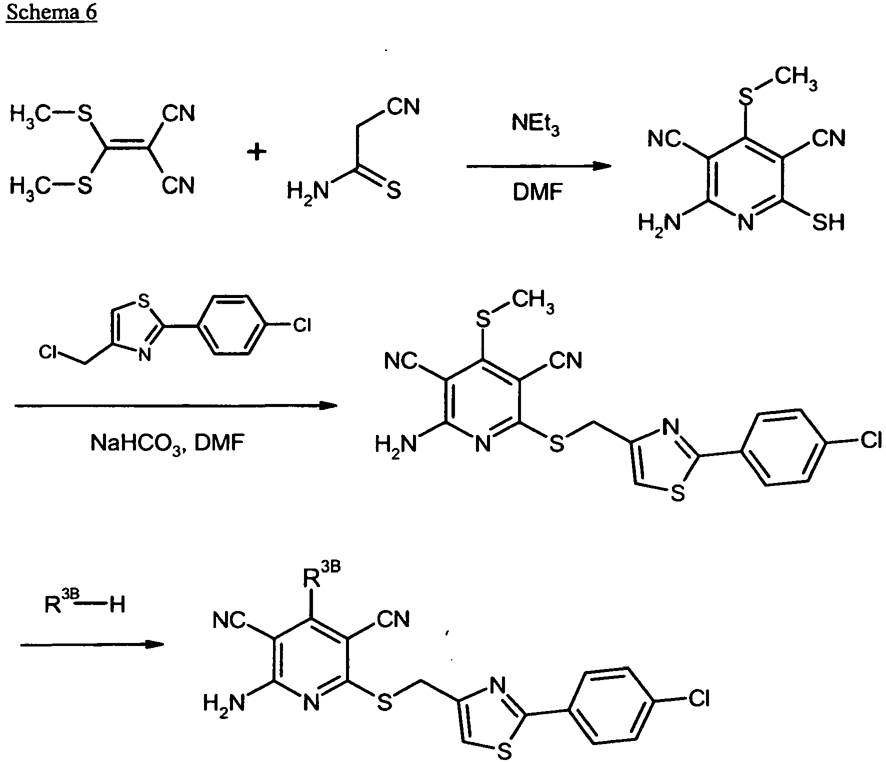

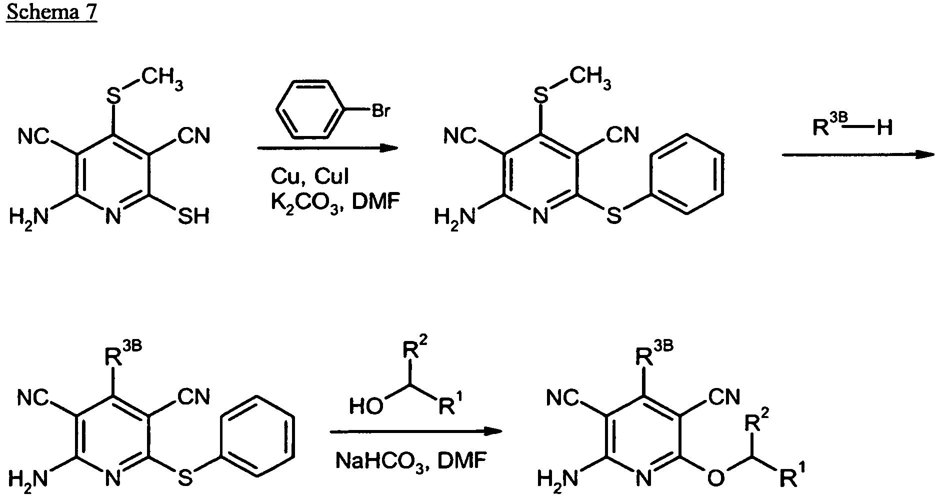

Weitere erfindungsgemäße Verbindungen können gegebenenfalls auch hergestellt werden durch Umwandlungen von funktionellen Gruppen einzelner Substituenten, insbesondere den unter R¹, R², R³, R⁴, R⁵ und R⁷ aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetallkatalysierte Kupplungsreaktionen, Eliminierungen, Alkylierung, Aminierung, Veresterung, Esterspaltung, Veretherung, Etherspaltung, Bildung von Carbonamiden, sowie Einführung und Entfernung temporärer Schutzgruppen.

Gegenüber den aus dem Stand der Technik bekannten Substanzen weisen die erfindungsgemäßen Verbindungen ein verbessertes Eigenschaftsprofil auf, wie beispielsweise eine erhöhte Löslichkeit in wässrig-organischen, für die Formulierung relevanten Lösungsmittelsystemen, eine längere pharmakokinetische Halbwertszeit nach oraler Gabe und/oder eine erhöhte metabolische Stabilität.

Als "selektive Liganden an Adenosin A1- und/oder A2b-Rezeptoren" werden im Rahmen der vorliegenden Erfindung solche Adenosin-Rezeptorliganden bezeichnet, bei denen einerseits eine deutliche Wirkung an A1- und/oder A2b-Adenosinrezeptor-Subtypen und andererseits keine oder eine deutliche schwächere Wirkung (Faktor 10 oder höher) an A2a- und A3-Adenosinrezeptor-Subtypen zu beobachten ist, wobei bezüglich der Testmethoden für die Wirk-Selektivität Bezug genommen wird auf die im Abschnitt B-1. beschriebenen Tests.

Überraschenderweise zeigen die erfindungsgemäßen Verbindungen ein nicht vorhersehbares, wertvolles pharmakologisches Wirkspektrum und sind daher insbesondere zur Prävention und/oder Behandlung von Erkrankungen geeignet.

Die pharmazeutische Wirksamkeit der erfindungsgemäßen Verbindungen lässt sich durch ihre Wirkung als potente, selektive Liganden an den Adenosin A1- und/oder A2b-Rezeptoren erklären. Sie wirken hierbei als selektive A1-Agonisten, selektive duale A1/A2b-Agonisten oder selektive A1-Antagonisten. Die erfindungsgemäßen Verbindungen zeigen ein gleiches oder verbessertes physikochemisches, pharmakokinetisches und/ oder therapeutisches Profil. Die erfindungsgemäßen Verbindungen wirken vorwiegend als selektive Adenosin A1-Agonisten.

Als "selektive Liganden an Adenosin A1- und/oder A2b-Rezeptoren" werden im Rahmen der vorliegenden Erfindung solche Adenosin-Rezeptorliganden bezeichnet, bei denen einerseits eine deutliche Wirkung am A1- und/oder A2b-Adenosinrezeptor-Subtypen und andererseits keine oder eine deutliche schwächere Wirkung (Faktor 10 oder höher) an A2a- und A3-Adenosinrezeptor-Subtypen zu beobachten ist, wobei bezüglich der Testmethoden für die Wirk-Selektivität Bezug genommen wird auf die im Abschnitt B-1. und B-5. beschriebenen Tests.

Die erfindungsgemäßen Verbindungen können in Abhängigkeit von ihrer jeweiligen Struktur als volle, als partielle Adenosinrezeptor-Agonisten oder als Adenosinrezeptor-Antagonisten fungieren. Partielle Adenosinrezeptor-Agonisten sind hierbei definiert als Rezeptorliganden, die eine funktionelle Antwort an Adenosinrezeptoren auslösen, welche geringer ist als bei vollen Agonisten (wie beispielsweise Adenosin selbst). Partielle Agonisten weisen infolgedessen eine geringere Wirksamkeit bezüglich der Rezeptoraktivierung auf als volle Agonisten.

Die Verbindungen der Formel (I) sind allein oder in Kombination mit einem oder mehreren anderen Wirkstoffen zur Prävention und/oder Behandlung verschiedener Erkrankungen geeignet, so beispielsweise Erkrankungen des Herzkreislauf-Systems (kardiovaskulären Erkrankungen), zur Kardioprotektion nach Schädigungen des Herzens sowie von Stoffwechsel- und Nierenerkrankungen.

Im Sinne der vorliegenden Erfindung sind unter Erkrankungen des Herzkreislauf-Systems bzw. kardiovaskulären Erkrankungen beispielsweise die folgenden Erkrankungen zu verstehen: Hypertonie, periphere und kardiale Gefäßerkrankungen, koronare Herzerkrankung, koronare Restenose wie z.B. Restenose nach Ballondilatation von peripheren Blutgefäßen, Myokardinfarkt, akutes Koronarsyndrom, akutes Koronarsyndrom mit ST-Erhebung, akutes Koronarsyndrom ohne ST-Erhebung, stabile und instabile Angina pectoris, Herzmuskelschwäche, Prinzmetal Angina, persistierende ischämische Dysfunktion (hibernating Myokardium), vorübergehende postischämische Dysfunktion (stunned Myokardium), Herzinsuffizienz, Tachykardien, atriale Tachykardie, Arrhythmien, Vorhof und Kammerflimmern, persistierendes Vorhofflimmern, permanentes Vorhofflimmern, Vorhofflimmern mit normaler linksventrikulärer Funktion, Vorhofflimmern mit eingeschränkter linksventrikulärer Funktion, Wolff-Parkinson-White Syndrom, periphere Durchblutungsstörungen, erhöhte Spiegel von Fibrinogen und von LDL geringer Dichte, sowie erhöhte Konzentrationen von Plasminogenaktivator-Inhibitor 1 (PAI-1), insbesondere koronare Herzerkrankung, akutes Koronarsyndrom, Angina pectoris, Herzinsuffizienz, Myokardinfarkt und Vorhofflimmern

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akute dekompensierte Herzinsuffizienz, Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische sowie systolische Herzinsuffizienz. Weiterhin eignen sich die erfindungsgemäßen Verbindungen auch zur Reduktion des von einem Infarkt betroffenen Myokardbereichs sowie zur Prävention von Sekundärinfarkten.

Des weiteren sind die erfindungsgemäßen Verbindungen zur Prävention und/oder Behandlung von thromboembolischen Erkrankungen, Reperfusionsschäden nach Ischämie, mikro- und makrovaskulären Schädigungen (Vaskulitis), arteriellen sowie venösen Thrombosen, Ödemen, Ischämien wie Myokardinfarkt, Hirnschlag und transitorischen ischämischen Attacken, zur Kardioprotektion bei Koronararterien Bypass Operationen (CABG), primären PTCAs, PTCAs nach Thrombolyse, Rescue-PTCA, Herztransplantationen und Operationen am offenen Herzen, sowie zur Organprotektion bei Transplantationen, Bypass Operationen, Herzkatheteruntersuchungen und anderen operativen Eingriffen geeignet.

Weiterhin eignen sich die erfindungsgemäßen Verbindungen zur Behandlung und/oder Prävention von Nierenerkrankungen, insbesondere von Niereninsuffizienz. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, obstruktive Uropathie, Glomerulonephritis, akute Glomerulonephritis, tubulointerstitielle Erkrankungen, nephropathische Erkrankungen wie primäre und angeborene Nierenerkrankung, Nierenentzündung, durch toxische Substanzen induzierte Nephropathie, diabetische Nephropathie, Pyelonephritis, Nierenzysten und Nephrosklerose, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prävention von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weitere Indikationsgebiete, für die die erfindungsgemäßen Verbindungen eingesetzt werden können, sind beispielsweise die Prävention und/oder Behandlung von Erkrankungen des Urogenitalbereiches, wie z.B. Reizblase, erektile Dysfunktion und weibliche sexuelle Dysfunktion, daneben aber auch die Prävention und/oder Behandlung von inflammatorischen Erkrankungen, wie z.B. entzündliche Dermatosen (Psoriasis, Akne, Ekzeme, Neurodermitis, Dermatitis, Keratitis, Narbenbildung, Warzenbildung, Frostbeulen), von Erkrankungen des Zentralen Nervensystems und neurodegenerativen Störungen (Schlaganfall, Alzheimer'sche Krankheit, Parkinson'sche Krankheit, Demenz, Epilepsie, Depressionen, Multiple Sklerose), von Schmerzzustanden, Krebserkrankungen (Hautkrebs, Liposarcome, Karzinome des Magen-Darm-Traktes, der Leber, Bauchspeicheldrüse, Lunge, Niere, Harnleiter, Prostata und des Genitaltraktes) sowie von Übelkeit und Erbrechen in Verbindung mit Krebstherapien.

Weitere Indikationsgebiete sind beispielsweise die Prävention und/oder Behandlung von Entzündung- und Immunerkrankungen (Morbus Crohn, Colitis ulcerosa, Lupus erythematodes, rheumatoide Arthritis) und von Erkrankungen der Atemwege, wie beispielsweise chronischobstruktive Atemwegserkrankungen (chronische Bronchitis, COPD), Asthma, Lungenemphysem, Bronchiektasien, zystische Fibrose (Mukoviszidose) und pulmonale Hypertonie, insbesondere pulmonale arterielle Hypertonie.

Schließlich kommen die erfindungsgemaßen Verbindungen auch für die Prävention und/ oder Behandlung von Diabetes, insbesondere Diabetes mellitus, Gestationsdiabetes, Insulin-abhängiger Diabetes und Nicht-Insulin-abhängiger Diabetes, von diabetischen Folgeerkrankungen wie z.B. Retinopathie, Nephropathie und Neuropathie, von metabolischen Erkrankungen (Metabolisches Syndrom, Hyperglykämie, Gestationsdiabetes, Hyperinsulinämie, Insulinresistenz, Glukose-Intoleranz, Fettsucht (Adipositas)) sowie von Arteriosklerose und Dyslipidämien (Hypercholesterolämie, Hypertriglyceridämie, erhöhte Konzentrationen der postprandialen Plasma-Triglyceride, Hypoalphalipoproteinämie, kombinierte Hyperlipidämien), insbesondere von Diabetes, Metabolischem Syndrom und Dyslipidämien, in Betracht

Darüber hinaus können die erfindungsgemäßen Verbindungen auch zur Behandlung und/oder Prävention von Schilddrüsenerkrankungen (Hyperthyreose), Erkrankungen der Bauchspeicheldrüse (Pankreatitis), Leberfibrose, viralen Erkrankungen (HPV, HCMV, HIV), Kachexie, Osteoporose, Gicht, Inkontinenz sowie zur Wundheilung und Angiogenese eingesetzt werden.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prophylaxe von koronarer Herzerkrankung, akutem Koronarsyndrom, Angina pectoris, Herzinsuffizienz, Myokardinfarkt und Vorhofflimmern.

Weiterer Gegenstand der vorliegenden Erfindung sind die erfindungsgemäßen Verbindungen zur Verfahren zur Behandlung und/oder Prophylaxe von Diabetes, Metabolischem Syndrom und Dyslipidämien.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen.

Als geeignete Kombinationswirkstoffe seien beispielhaft und vorzugsweise genannt: den Fettstoffwechsel verändernde Wirkstoffe, Antidiabetika, Blutdruck-Senker, durchblutungsfördernd und/ oder antithrombotisch wirkende Mittel, Antioxidantien, Chemokin-Rezeptor-Antagonisten, p38-Kinase-Inhibitoren, NPY-Agonisten, Orexin-Agonisten, Anorektika, PAF-AH-Inhibitoren, Antiphlogistika (COX-Inhibitoren, LTB₄-Rezeptor-Antagonisten), Analgetika wie beispielsweise Aspirin, Anti-Depressiva und andere Pyschopharmaka.

Gegenstand der vorliegenden Erfindung sind insbesondere Kombinationen mindestens einer der erfindungsgemäßen Verbindungen mit mindestens einem den Fettstoffwechsel verändernden Wirkstoff, einem Antidiabetikum, einem blutdrucksenkenden Wirkstoff und/oder einem antithrombotisch wirkenden Mittel.

Die erfindungsgemäßen Verbindungen können vorzugsweise mit einem oder mehreren
- den Fettstoffwechsel verändernden Wirkstoffen, beispielhaft und vorzugsweise aus der Gruppe der HMG-CoA-Reduktase-Inhibitoren, Inhibitoren der HMG-CoA-Reduktase-Expression, Squalensynthese-Inhibitoren, ACAT-Inhibitoren, LDL-Rezeptor-Induktoren, Cholesterin-Absorptionshemmer, polymeren Gallensäureadsorber, Gallensäure-Reabsorptionshemmer, MTP-Inhibitoren, Lipase-Inhibitoren, LpL-Aktivatoren, Fibrate, Niacin, CETP-Inhibitoren, PPAR-α-, PPAR-γ- und/oder PPAR-δ-Agonisten, RXR-Modulatoren, FXR-Modulatoren, LXR-Modulatoren, Thyroidhormone und/oder Thyroidmimetika, ATP-Citrat-Lyase-Inhibitoren, Lp(a)-Antagonisten, Cannabinoid-Rezeptor 1-Antagonisten, Leptin-Rezeptor-Agonisten, Bombesin-Rezeptor-Agonisten, Histamin-Rezeptor-Agonisten sowie der Antioxidantien/Radikalfänger;
- Antidiabetika, die in der Roten Liste 2004/II, Kapitel 12 genannt sind, sowie beispielhaft und vorzugsweise jenen aus der Gruppe der Sulphonylharnstoffe, Biguanide, Meglitinid-Derivate, Glukosidase-Inhibitoren, Inhibitoren der Dipeptidyl-Peptidase IV (DPP-IV-Inhibitoren), Oxadiazolidinone, Thiazolidindione, GLP 1-Rezeptor-Agonisten, Glukagon-Antagonisten, Insulin-Sensitizer, CCK 1-Rezeptor-Agonisten, Leptin-Rezeptor-Agonisten, Inhibitoren von Leberenzymen, die an der Stimulation der Glukoneogenese und/ oder Glykogenolyse beteiligt sind, Modulatoren der Glukoseaufnahme sowie der Kaliumkanalöffner, wie z.B. denjenigen, die in WO 97/26265 und WO 99/03861 offenbart sind;
- den Blutdruck senkenden Wirkstoffen, beispielhaft und vorzugsweise aus der Gruppe der Calcium-Antagonisten, Angiotensin All-Antagonisten, ACE-Hemmer, Renin-Inhibitoren, beta-Rezeptoren-Blocker, alpha-Rezeptoren-Blocker, Aldosteron-Antagonisten, Mineralocorticoid-Rezeptor-Antagonisten, ECE-Inhibitoren, ACE/NEP Inhibitoren sowie der Vasopeptidase-Inhibitoren; und/oder
- antithrombotisch wirkenden Mitteln, beispielhaft und vorzugsweise aus der Gruppe der Thrombozytenaggregationshemmer oder der Antikoagulantien
- Diuretika;
- Vasopressin-Rezeptor-Antagonisten;
- organischen Nitraten und NO-Donatoren;
- positiv-inotrop wirksamen Verbindungen;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil sowie PDE 3-Inhibitoren wie Milrinone;
- natriuretischen Peptiden, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Agonisten des Prostacyclin-Rezeptors (IP-Rezeptors), wie beispielsweise Iloprost, Beraprost, Cicaprost;
- Hemmer des I_{f} (funny channel) Kanals, wie beispielsweise Ivabradine;
- Calcium-Sensitizern, wie beispielhaft und vorzugsweise Levosimendan;
- Kalium-Supplements;
- NO- und Häm-unabhängige Aktivatoren der Guanylatcyclase, wie insbesondere Cinaciguat sowie die in WO 01/19355, WO 01/19776, WO 01/19778, WO 01/19780, WO 02/070462 und WO 02/070510 beschriebenen Verbindungen;
- NO-unabhängige, jedoch Häm-abhängige Stimulatoren der Guanylatcyclase, wie insbesondere Riociguat sowie die in WO 00/06568, WO 00/06569, WO 02/42301 und WO 03/095451 beschriebenen Verbindungen;
- Inhibitoren der humanen neutrophilen Elastase (HNE), wie beispielsweise Sivelestat und DX-890 (Reltran);
- die Signaltransduktionskaskade inhibierenden Verbindungen, wie beispielsweise Tyrosinkinase-Inhibitoren, insbesondere Sorafenib, Imatinib, Gefitinib und Erlotinib; und/oder
- den Energiestoffwechsel des Herzens beeinflussenden Verbindungen, wie beispielweise Etomoxir, Dichloracetat, Ranolazine und Trimetazidine
kombiniert werden.

Unter den Fettstoffwechsel verändernden Wirkstoffen werden vorzugsweise Verbindungen aus der Gruppe der HMG-CoA-Reduktase-Inhibitoren, Squalensynthese-Inhibitoren, ACAT-Inhibitoren, Cholesterin-Absorptionshemmer, MTP-Inhibitoren, Lipase-Inhibitoren, Thyroidhormone und/oder Thyroidmimetika, Niacin-Rezeptor-Agonisten, CETP-Inhibitoren, PPAR-α-Agonisten, PPAR-γ-Agonisten, PPAR-δ-Agonisten, polymeren Gallensäureadsorber, Gallensäure-Reabsorptionshemmer, Antioxidantien/Radikalfänger sowie der Cannabinoid-Rezeptor 1-Antagonisten verstanden.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem Thyroidhormon und/oder Thyroidmimetikum, wie beispielhaft und vorzugsweise D-Thyroxin oder 3,5,3'-Triiodothyronin (T3), verabreicht.

Bei einer bevorzugten Ausführungsform der Erfindung werden die erfindungsgemäßen Verbindungen in Kombination mit einem Agonisten des Niacin-Rezeptors, wie beispielhaft und vorzugsweise Niacin, Acipimox, Acifran oder Radecol, verabreicht.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem PPAR-γ-Agonisten beispielsweise aus der Klasse der Thiazolidindione, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-δ-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Antioxidans/Radikalfänger, wie beispielhaft und vorzugsweise Probucol, AGI-1067, BO-653 oder AEOL-10150, verabreicht.

Bei einer bevorzugten Ausführungsform der Erfindung werden die erfindungsgemäßen Verbindungen in Kombination mit einem Cannabinoid-Rezeptor 1-Antagonisten, wie beispielhaft und vorzugsweise Rimonabant oder SR-147778, verabreicht.

Unter Antidiabetika werden vorzugsweise Insulin und Insulinderivate sowie oral wirksame hypoglykämische Wirkstoffe verstanden. Insulin und Insulinderivate umfasst hierbei sowohl Insuline tierischen, menschlichen oder biotechnologischen Ursprungs als auch Gemische hieraus. Die oral wirksamen hypoglykämischen Wirkstoffe umfassen vorzugsweise Sulphonylharnstoffe, Biguanide, Meglitinid-Derivate, Glukosidase-Inhibitoren und PPAR-gamma-Agonisten.

Bei einer bevorzugten Ausführungsform der Erfindung werden die erfindungsgemäßen Verbindungen in Kombination mit Insulin verabreicht.

Bei einer bevorzugten Ausführungsform der Erfindung werden die erfindungsgemäßen Verbindungen in Kombination mit einem Sulphonylharnstoff, wie beispielhaft und vorzugsweise Tolbutamid, Glibenclamid, Glimepirid, Glipizid oder Gliclazid, verabreicht.

Bei einer bevorzugten Ausführungsform der Erfindung werden die erfindungsgemäßen Verbindungen in Kombination mit einem Biguanid, wie beispielhaft und vorzugsweise Metformin, verabreicht.

Bei einer bevorzugten Ausführungsform der Erfindung werden die erfindungsgemäßen Verbindungen in Kombination mit einem Meglitinid-Derivat, wie beispielhaft und vorzugsweise Repaglinid oder Nateglinid, verabreicht.

Bei einer bevorzugten Ausführungsform der Erfindung werden die erfindungsgemäßen Verbindungen in Kombination mit einem Glukosidase-Inhibitor, wie beispielhaft und vorzugsweise Miglitol oder Acarbose, verabreicht.

Bei einer bevorzugten Ausführungsform der Erfindung werden die erfindungsgemäßen Verbindungen in Kombination mit einem DPP-IV-Inhibitor, wie beispielhaft und vorzugsweise Sitagliptin und Vildagliptin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, beta-Rezeptoren-Blocker, alpha-Rezeptoren-Blocker und Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Valsartan, Candesartan, Embusartan, Olmesartan oder Telmisartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem alpha-Rezeptoren-Blocker, wie beispielhaft und vorzugsweise Prazosin, verabreicht.

Bei einer bevorzugten Ausführungsfonm der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Bei einer bevorzugten Ausführungsform der Erfindung werden die erfindungsgemäßen Verbindungen in Kombination mit einem Aldosteron- oder Mineralokortikoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Vasopressin-Rezeptor-Antagonisten, wie beispielhaft und vorzugsweise Conivaptan, Tolvaptan, Lixivaptan oder SR-121463, verabreicht.

Bei einer bevorzugten Ausführungsform der Erfindung werden die erfindungsgemäßen Verbindungen in Kombination mit einem organischen Nitrat oder NO-Donator, wie beispielhaft und vorzugsweise Natriumnitroprussid, Nitroglycerin, Isosorbidmononitrat, Isosorbiddinitrat, Molsidomin oder SIN-1, oder in Kombination mit inhalativem NO verabreicht.

Bei einer bevorzugten Ausführungsform der Erfindung werden die erfindungsgemäßen Verbindungen in Kombination mit einer positiv-inotrop wirksamen Verbindung, wie beispielhaft und vorzugsweise Herzglycosiden (Digoxin), beta-adrenergen und dopaminergen Agonisten wie Isoproterenol, Adrenalin, Noradrenalin, Dopamin oder Dobutamin, verabreicht.

Bei einer bevorzugten Ausführungsform der Erfindung werden die erfindungsgemäßen Verbindungen in Kombination mit Antisympathotonika wie Reserpin, Clonidin oder alpha-Methyl-Dopa, mit Kaliumkanal-Agonisten wie Minoxidil, Diazoxid, Dihydralazin oder Hydralazin, oder mit Stickoxid freisetzenden Stoffen wie Glycerinnitrat oder Nitroprussidnatrium verabreicht.

Unter antithrombotisch wirkenden Mitteln werden vorzugsweise Verbindungen aus der Gruppe der Thrombozytenaggregationshemmer oder der Antikoagulantien verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Besonders bevorzugt im Rahmen der vorliegenden Erfindung sind Kombinationen enthaltend mindestens eine der erfindungsgemäßen Verbindungen sowie einen oder mehrere weitere Wirkstoffe ausgewählt aus der Gruppe bestehend aus HMG-CoA-Reduktase-Inhibitoren (Statine), Diuretika, beta-Rezeptoren-Blocker, organische Nitrate und NO-Donatoren, ACE-Inhibitoren, Angiotensin AII-Antagonisten, Aldosteron- und Mineralokortikoid-Rezeptor-Antagonisten, Vasopressin-Rezeptor-Antagonisten, Thrombozytenaggregationshemmer und Antikoagulantien, sowie deren Verwendung zur Behandlung und/oder Prävention der zuvor genannten Erkrankungen.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

**Verwendete Abkürzungen:**
- aq.: wässrig
- Bsp.: Beispiel
- c: Konzentration
- d: Dublett (bei NMR)
- dd: Dublett von Dublett (bei NMR)
- DBU: 1,8-Diazabicyclo[5.4.0]undec-7-en
- DC: Dünnschichtchromatographie
- DCI: direkte chemische Ionisation (bei MS)
- DMF: *N,N*-Dimethylformamid
- DMSO: Dimethylsulfoxid
- d. Th.: der Theorie (bei Ausbeute)
- ee: Enantiomerenüberschuss
- EI: Elektronenstoß-Ionisation (bei MS)
- ESI: Elektrospray-Ionisation (bei MS)
- Et: Ethyl
- Fp.: Schmelzpunkt
- h: Stunde(n)
- HPLC: Hochdruck-, Hochleistungsflüssigchromatographie
- kat.: katalytisch
- konz.: konzentriert
- LC-MS: Flüssigchromatographie-gekoppelte Massenspektrometrie
- Lit.: Literatur(stelle)
- Me: Methyl
- MeCN: Acetonitril
- min: Minute(n)
- MS: Massenspektrometrie
- NMM: *N*-Methylmorpholin
- NMR: Kernresonanzspektrometrie
- q: Quartett (bei NMR)
- rac.: racemisch
- RP-HPLC: reverse phase HPLC
- RT: Raumtemperatur
- Rₜ: Retentionszeit (bei HPLC)
- s: Singulett (bei NMR)
- s br: breites Singulett (bei NMR)
- t: Triplett (bei NMR)
- t-Bu: tert.-Butyl
- TFA: Trifluoressigsäure
- THF: Tetrahydrofuran
- verd.: verdünnt

### HPLC-, LC-MS- und GC-MS-Methoden:

Methode 1 (LC-MS): Gerätetyp MS: Waters (Micromass) Quattro Micro; Gerätetyp HPLC: Agilent 1100 Serie; Säule : Thermo Hypersil GOLD 3µ 20 x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100% A → 3.0 min 10% A → 4.0 min 10% A → 4.01 min 100% A (Flow 2.5ml) → 5.00 min 100% A; Ofen: 50°C; Fluss: 2 ml/min; UV-Detektion: 210nm.

Methode 2 (LC-MS): Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 210 nm.

Methode 3 (LC-MS): Gerätetyp MS: Micromass ZQ; Gerätetyp HPLC: Waters Alliance 2795; Säule: Phenomenex Synergi 2.5 µ MAX-RP 100A Mercury 20 mm x 4mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

Methode 4 (LC-MS): Instrument: Micromass Quattro Premier mit Waters UPLC Acquity ; Säule: Thermo Hypersil GOLD 1,9µ 50 x 1mm; Eluent A: 1 1 Wasser + 0.5ml 50%ige Ameisensäure , Eluent B: 1 1 Acetonitril + 0.5ml 50%ige Ameisensäure; Gradient: 0.0 min 90% A → 0.1 min 90% A → 1.5 min 10% A → 2.2 min 10% A; Ofen:50°C; Fluss: 0.33 ml/min; UV-Detektion: 210 nm.

Methode 5 (LC-MS): Gerätetyp MS: Micromass ZQ; Gerätetyp HPLC: Waters Alliance 2795; Säule: Phenomenex Synergi 2µ Hydro-RP Mercury 20 mm x 4mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 210 nm.

Methode 6 (LC-MS): Gerätetyp MS: Micromass ZQ; Gerätetyp HPLC: Waters Alliance 2795; Säule: Merck Chromolith SpeedROD RP-18e 100 x 4.6 mm; Eluent A: 1 1 Wasser + 0.5 ml 50%ige Ameisensäure; Eluent B: 1 1 Acetonitril + 0.5 ml 50%ige Ameisensäure; Gradient: 0.0 min 10% B → 7.0 min 95% B → 9.0 min 95% B; Ofen: 35°C; Fluss: 0.0 min 1.0 ml/min → 7.0 min 2.0 ml/min → 9.0 min 2.0 ml/min; UV-Detektion: 210 nm.

Methode 7 (präparative HPLC): Säule: Grom-Sil C18, 10 µm, 250 mm x 30 mm. Eluent A: Wasser + 0.1% Ameisensäure, Eluent B: Acetonitril. Fluß: 50 ml/min. Programm: 0-5 min: 10% B; 5-38 min: Gradient bis 95% B, UV-Detektion: 210 nm.

Methode 8 (LC-MS): Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; W-Detektion: 208- 400 nm.

Methode 9 (LC-MS): Instrument: Micromass Quattro LCZ mit HPLC Agilent Serie 1100; Säule: Phenomenex Onyx Monolithic C18, 100 mm x 3 mm. Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 208- 400 nm.

Methode 10 (LC-MS): Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.

Methode 11 (LC-MS): MHZ-Q-GEM-1Gerätetyp MS: Micromass Quattro LCZ; Gerätetyp HPLC: HP 1100 Series; UV DAD; Säule: Phenomenex Gemini 3µ 30 mm x 3.00 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min. 2 ml/min; Ofen: 50°C; UV-Detektion: 210 nm.

### Ausgangsverbindungen und Intermediate:

### Beispiel 1A

### 2-Amino-4-phenyl-6-sulfanylpyridin-3,5-dicarbonitril

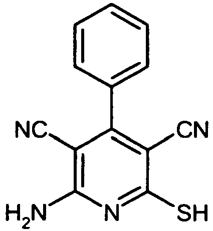

Die Darstellung erfolgte wie in WO 03/053441 für Beispiel 6 beschrieben.

MS (ESIpos): m/z = 253 [M+H]⁺

### Beispiel 2A

### 2-Amino-6-({[2-(4-chlorphenyl)-1,3-thiazol-4-yl]methyl}sulfanyl)-4-phenylpyridin-3,5-dicarbonitril

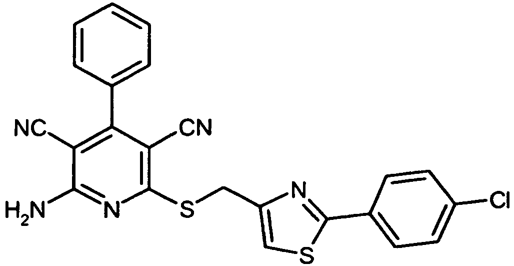

2 g (7.927 mmol) 2-Amino-4-phenyl-6-sulfanylpyridin-3,5-dicarbonitril wurden in 15 ml DMF vorgelegt, mit 2.13 g (8.720 mmol) 4-(Chlormethyl)-2-(4-chlorphenyl)-1,3-thiazol und 1.99 g (23.781 mmol) Natriumhydrogencarbonat versetzt und über Nacht bei Raumtemperatur gerührt. Das Reaktionsgemisch wurde mit Wasser versetzt, der ausgefallene Feststoff abfiltriert, mit MTBE gewaschen und am Hochvakuum getrocknet. Man erhielt 3.87 g (100 % d. Th.) der Zielverbindung.

¹H-NMR (400 MHz, DMSO-d₆): δ = 8.14 (s br, 2H), 7.97-7.92 (m, 3H), 7.59-7.50 (m, 7H), 4.64 (s, 2H).

LC-MS (Methode 4): Rₜ = 1.49 min; MS (ESIpos): m/z = 460 [M+H]⁺.

### Beispiel 3A

### 2-Chlor-6-({[2-(4-chlorphenyl)-1,3-thiazol-4-yl]methyl}sulfanyl)-4-phenylpyridin-3,5-dicarbonitril

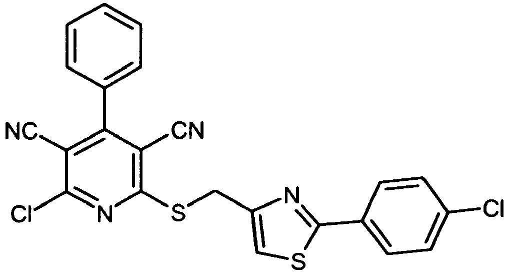

9g (19.762 mmol) 2-Amino-6-({[2-(4-chlorphenyl)-1,3-thiazol-4-yl]methyl}sulfanyl)-4-phenylpyridin-3,5-dicarbonitril wurden in 200 ml Acetonitril vorgelegt, mit 7.98 ml (59.285 mmol) Isoamylnitrit und 7.97 g (59.285 mmol) Kupfer(II)chlorid versetzt und 4 Stunden bei 60°C gerührt. Der Ansatz wurde mit 1N Salzsäure versetzt und der ausgefallene Feststoff abfiltriert. Dieser wurde über eine Kieselgelsäule (Eluent: Toluol) gereinigt. Man erhält 5.98 g (63 % d. Th.) der Zielverbindung.

¹H-NMR (400 MHz, DMSO-d₆): δ = 7.96 (d, 2H), 7.76 (s, 1H), 7.66-7.62 (m, 5H), 7.57 (d, 2H), 4.78 (s, 2H).

LC-MS (Methode 3): Rₜ = 2.82 min; MS (ESIpos): m/z = 479 [M+H]⁺.

### Beispiel 4A

### 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril

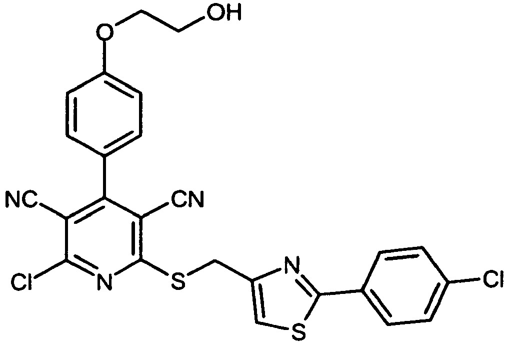

Die Darstellung erfolgte in Analogie zu Beispiel 3A aus den entsprechenden Ausgangsverbindungen.

LC-MS (Methode 2): Rₜ = 2.93 min; MS (ESIpos): m/z = 539 [M+H]⁺.

### Beispiel 5

### 2-Amino-6-sulfanyl-4-(1,3-thiazol-5-yl)pyridin-3,5-dicarbonitril

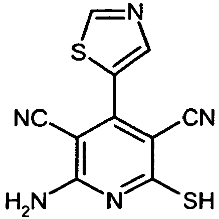

1.14 g (9.539 mmol) 5-Thiazolcarboxaldehyd wurden mit 1.91 g (19.077 mmol) Cyanothioacetamid in 20 ml Ethanol vorgelegt, mit 2.097 ml (19.077 mmol) 4-Methylmorpholin versetzt und 4 Stunden refluxiert. Anschließend wurde 20 Stunden bei Raumtemperatur gerührt. Das Reaktionsgemisch wurde eingeengt und der Rückstand an Kieselgel gereinigt (Eluent: Methylenchlorid/Methanol 100:0 → 10:1). Die produktenthaltenden Fraktionen wurden eingeengt und der Rückstand mit Acetonitril ausgerührt. Der Feststoff wurde abfiltriert und am Hochvakuum getrocknet. Man erhielt 920 mg (37 % d. Th.) der Zielverbindung.

¹H-NMR (400 MHz, DMSO-d₆): δ = 9.31 (s, 1H), 8.16 (s, 1H), 7.50-6.99 (s br, 2H).

LC-MS (Methode 2): Rₜ = 1.29 min; MS (ESIpos): m/z = 260 [M+H]⁺.

### Beispiel 6A

### 2-Amino-6-({[2-(4-chlorphenyl)-1,3-thiazol-4-yl]methyl}sulfamyl)-4-(1,3-thiazol-5-yl)pyridin-3,5-dicarbonitril

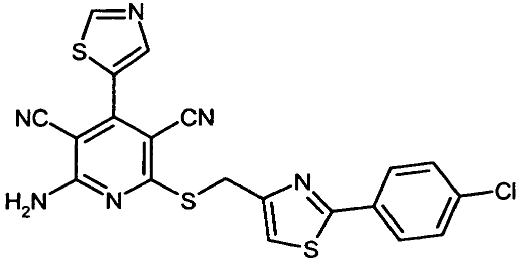

370 mg (1.427 mmol) 2-Amino-6-sulfanyl-4-(1,3-thiazol-5-yl)pyridin-3,5-dicarbonitril wurden mit 383 mg (1.570 mmol) 4-(Chlormethyl)-2-(4-chlorphenyl)-1,3-thiazol und 359 mg Natriumhydrogencarbonat 1 Stunde in 7 ml DMF gerührt. Das Reaktionsgemisch wurde mit 150 ml Acetonitril und 100 ml Wasser versetzt, der ausgefallene Feststoff abfiltriert und am Hochvakuum getrocknet. Man erhält 569 mg (85 % d. Th.) der Zielverbindung.

¹H-NMR (400 MHz, DMSO-d₆): δ = 9.38 (s, 1H), 8.27 (s, 1H), 8.19-8.03 (s br, 2H), 7.94 (d, 2H), 7.89 (s, 1H), 7.57 (d, 2H), 4.62 (s, 2H).

LC-MS (Methode 2): Rₜ = 2.67 min; MS (ESIpos): m/z = 467 [M+H]⁺.

### Beispiel 7A

### 2-Chlor-6-({[2-(4-chlorphenyl)-1,3-thiazol-4-yl]methyl}sulfanyl)-4-(1,3-thiazol-5-yl)pyridin-3,5-dicarbonitril

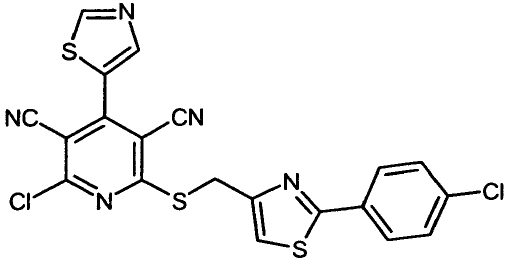

569 mg (1.218 mmol) 2-Amino-6-({[2-(4-chlorphenyl)-1,3-thiazol-4-yl]methyl}sulfanyl)-4-(1,3-thiazol-5-yl)pyridin-3,5-dicarbonitril wurden in 20 ml konzentrierter Salzsäure gelöst, auf 0°C gekühlt und bei dieser Temperatur mit 252 mg (3.655 mmol) Natriumnitrit versetzt. Es wurde 1h bei 0°C gerührt, dann auf Raumtemperatur erwärmt und über Nacht bei Raumtemperatur gerührt. Das Reaktionsgemisch wurde mittels präparativer HPLC (Acetonitril/Wasser: 10:90 → 95:5) gereinigt. Man erhält 445 mg (68 % d. Th.) der Zielverbindung.

¹H-NMR (400 MHz, DMSO-d₆): δ = 9.49 (s, 1H), 8.42 (s, 1H), 7.95 (d, 2H), 7.75 (s, 1H), 7.58 (d, 2H), 4.78 (s, 2H).

LC-MS (Methode 2): Rₜ = 2.99 min; MS (ESIpos): m/z = 486 [M+H]⁺.

### Beispiel 8A

### 2-Amino-4-[4-(2-hydroxyethoxy)phenyl]-6-methoxypyridin-3,5-dicarbonitril

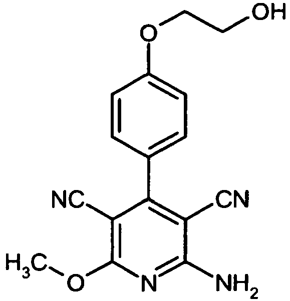

Unter Argon wurden 10 g (60.176 mmol) 4-(2-Hydroxyethoxy)benzaldehyd in 125 ml Methanol gelöst, mit 8.15 g (123.362 mmol) Malonsäuredinitril und 19.506 g (361.058 mmol) Natriummethylat versetzt und 3 Stunden bei Raumtemperatur gerührt. Anschließend wurde das Reaktionsgemisch eingeengt, der Rückstand in Essigsäureethylester aufgenommen, mit gesättigter wässriger Ammoniumchlorid-Lösung gewaschen. Die organische Phase wurde über Natriumsulfat getrocknet und eingeengt. Der Rückstand wurde mit Methanol ausgerührt, der ausgefallene Feststoff abfiltriert und am Hochvakuum getrocknet. Man erhielt 5.2 g (22% d. Th.) der Zielverbindung.

¹H-NMR (400 MHz, DMSO-d₆): δ = 8.14-7.80 (s br, 2H), 7.46 (d, 2H), 7.10 (d, 2H), 4.92 (t, 1H), 4.08 (t, 2H), 3.97 (s, 3H), 3.75 (q, 2H).

LC-MS (Methode 4): Rₜ = 0.86 min; MS (ESIpos): m/z = 311 [M+H]⁺.

### Beispiele 9A

### 2-Chlor-4-[4-(2-hydroxyethoxy)phenyl]-6-methoxypyridin-3,5-dicarbonitril

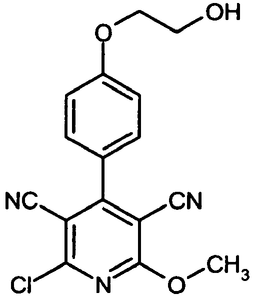

3.3 g (10.634 mmol) 2-Amino-4-[4-(2-hydroxyethoxy)phenyl]-6-methoxypyridin-3,5-dicarbonitril wurden in 300 ml Acetonitril vorgelegt, mit 8.578 g (63.806 mmol) Kupfer(II)chlorid und 8.59 ml (63.806 mmol) Isoamylnitrit versetzt und über Nacht bei Raumtemperatur gerührt. Das Reaktionsgemisch wurde mit 1N Salzsäure versetzt und mit Essigsäureethylester extrahiert. Die organische Phase wurde mit gesättiger wässriger Natriumhydrogencarbonat-Lösung und Wasser gewaschen, über Natriumsulfat getrocknet und eingeengt. Das Reaktionsgemisch wurde mittels präparativer HPLC (Acetonitril/Wasser: 10:90 → 95:5) gereinigt. Es wurden 2.11 g (58% d. Th.) der Zielverbindung erhalten.

¹H-NMR (400 MHz, DMSO-d₆): δ = 7.60 (d, 2H), 7.19 (d, 2H), 4.13 (s, 3H), 4.11 (t, 2H), 3.75 (t, 2H).

LC-MS (Methode 2): R, = 2.13 min; MS (ESIpos): m/z = 330 [M+H]⁺.

### Beispiele 10A

### 4-[4-(2-Hydroxyethoxy)phenyl]-2-methoxy-6-sulfanylpyridin-3,5-dicarbonitril

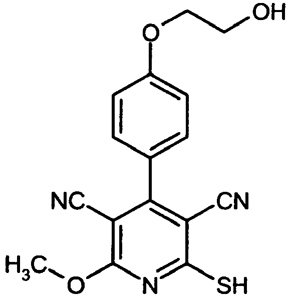

4.0 g (12.1 mmol) 2-Chlor-4-[4-(2-hydroxyethoxy)phenyl]-6-methoxypyridin-3,5-dicarbonitril wurden in 16 ml wasserfreiem DMF gelöst, mit 1,42g (18.2 mmol) wasserfreiem Natriumsulfid versetzt und 2h bei Raumtemperatur gerührt. Es wurde mit 36 ml Wasser versetzt und die Lösung unter Rühren langsam zu einer 50°C warmen Mischung von 61 ml 1M Salzsäure und 20 ml Methanol getropft. Die gelbe Suspension wurde 1h bei RT gerührt, abgesaugt, der Niederschlag mit Wasser gewaschen, und über Nacht bei 45°C im Vakuum getrocknet.

Ausbeute: 3.27 g (82 % d. Th., Reinheit 29 %)

LC-MS (Methode 6): Rₜ = 3.27 min (29.4 F1%); MS (ESIpos): m/z = 328 [M+H]⁺

Das Produkt wurde ohne weitere Reinigung weiter umgesetzt. Zu analytischen Zwecken wurde eine kleine Probe mittels präparativer HPLC (Methode 7) gereinigt:
¹H-NMR (500 MHz, DMSO-d₆): δ = 7.59-7.48 (m, 2H), 7.20- 7.10 (m, 2H), 4.75-4.35 (br), 4.10 (t, 2H), 3.93 (s, 3H), 3.65 (t, 2H).

### Beispiel 11A

### 2-Mercapto-6-methoxy-4-phenylpyridin-3,5-dicarbonitril

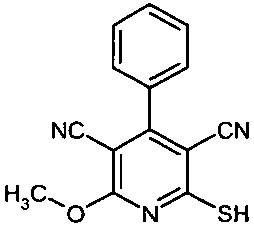

Die Darstellung erfolgt in Analogie zu Beispiel 10A aus den entsprechenden Ausgangsverbindungen.

LC-MS (Methode 6): Rₜ = 5.72 min; MS (ESIneg): m/z = 266 [M-H]⁺.

### Beispiel 12A

### 2-Amino-6-ethoxy-4-[4-(2-hydroxyethoxy)phenyl]pyridin-3,5-dicarbonitril

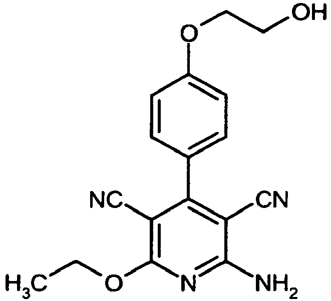

40 g (240.705 mmol) 4-(2-Hydroxyethoxy)benzaldehyd wurden in 500 ml Ethanol vorgelegt und mit 35.6 g (493.445 mmol) Malonsäuredinitril versetzt. Anschließend wurden 539 ml (1444.232 mmol) 21%ige Natriumethylat-Lösung in Ethanol zugetropft. Es wurde 24 Stunden bei Raumtemperatur gerührt. Der Ansatz wurde eingeengt, der Rückstand in Essigester aufgenommen, mit Wasser und gesättigter wässriger Natriumchlorid-Lösung gewaschen, die organische Phase über Natriumsulfat getrocknet und eingeengt. Der Rückstand wurde in Ethanol gelöst, auf Kieselgel aufgezogen und an Kieselgel (Eluent: Methylenchlorid/ Ethanol 200:1 → 50:1) gereinigt. Das gewonnene Produkt wurde in 200 ml Ethanol suspendiert, schrittweise mit 1000 ml Wasser versetzt und 1 Stunde bei Raumtemperatur gerührt. Der Feststoff wurde abfiltriert, mit Wasser/ Ethanol 1:1 gewaschen und bei 50°C im Vakuumtrockenschrank getrocknet. Es wurden 15.4 g (19 % d. Th.) der Zielverbindung erhalten.

¹H-NMR (400 MHz, DMSO-d₆): δ = 8.05-7.72 (s br, 2H), 7.46 (d, 2H), 7.10 (d, 2H), 4.94 (t, 1H), 4.44 (q, 2H), 4.08 (t, 2H), 3.75 (q, 2H), 1.34 (t, 3H).

LC-MS (Methode 2): Rₜ = 2.02 min; MS (ESIpos): m/z = 325 [M+H]⁺.

### Beispiele 13A

### 2-Chlor-6-ethoxy-4-[4-(2-hydroxyethoxy)phenyl]pyridin-3,5-dicarbonitril

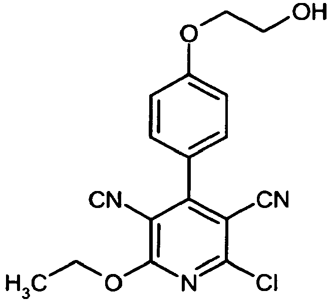

14.8 g (45.631 mmol) 2-Amino-6-ethoxy-4-[4-(2-hydroxyethoxy)phenyl]pyridin-3,5-dicarbonitril wurden in 830 ml Acetonitril vorgelegt, mit 32.07 g (273.785 mmol) Isoamylnitrit und 36.81 g (273.785 mmol) Kupfer(II)chlorid versetzt und 3 Stunden bei 60°C gerührt. Das Reaktionsgemisch wurde mit 800 ml 1N Salzsäure versetzt und mit Essigsäureethylester extrahiert. Die organische Phase wurde mit gesättigter wässriger Natriumhydrogencarbonat-Lösung und gesättigter wässriger Natriumchlorid-Lösung gewaschen, über Natriumsulfat getrocknet und eingeengt. Der Rückstand wurde mit Ethanol ausgerührt, der Feststoff abfiltriert, mit Ethanol gewaschen und am Hochvakuum getrocknet. Da in der Mutterlauge noch Produkt enthalten ist, wurde diese auf 35 g Kieselgel aufgezogen und an einer Kieselgelsäule (Eluent: Cyclohexan/ Essigsäureethylester 2:1 → 1:1) gereinigt. Insgesamt wurden 11.9 g (72 % d. Th.) der Zielverbindung erhalten.

¹H-NMR (400 MHz, DMSO-d₆): δ = 7.59 (d, 2H), 7.18 (d, 2H), 4.94 (s br, 1H), 4.57 (q, 2H), 4.10 (t, 2H), 3.76 (t, 2H), 1.41 (t, 3H).

LC-MS (Methode 8): Rₜ = 2.30 min; MS (ESIpos): m/z = 344 [M+H]⁺.

### Beispiel 14A

### 2-Ethoxy-4-[4-(2-hydroxyethoxy)phenyl]-6-sulfanylpyridin-3,5-dicarbonitril

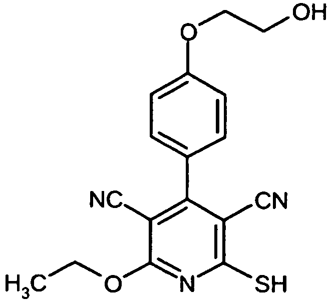

1 g (2.909 mmol) 2-Chlor-6-ethoxy-4-[4-(2-hydroxyethoxy)phenyl]pyridin-3,5-dicarbonitril wurden in 7.5 ml DMF vorgelegt, mit 454 mg (5.818 mmol) Natriumsulfid versetzt und über Nacht bei Raumtemperatur gerührt. Der Ansatz wurde eingeengt, der Rückstand mit Acetoniril ausgerührt, der Feststoff abfiltriert und am Hochvakuum getrocknet. Man erhielt 230 mg (24% d. Th.) der Zielverbindung.

LC-MS (Methode 4): Rₜ = 0.79 min; MS (ESIpos): m/z = 342 [M+H]⁺.

### Beispiel 15A

### 2-Amino-4-(4-hydroxyphenyl)-6-methoxypyridin-3,5-dicarbonitril

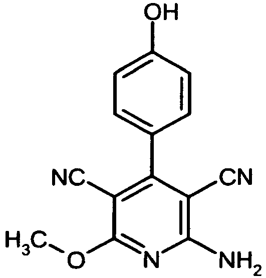

500 mg (4.094 mmol) 4-Hydroxybenzaldehyd und 554 mg (8.393 mmol) Malonsäuredinitril wurden in 5 ml Methanol vorgelegt und mit 4.42 g (24.565 mmol) Natriummethylat, 30%ig in Methanol, versetzt. Anschließend wurde 2 Stunden bei Raumtemperatur gerührt. Der ausgefallene Feststoff wurde abfiltriert, mit Methanol gewaschen und am Hochvakuum getrocknet. Man erhielt 613 mg (76 % d. Th.) der Zielverbindung.

LC-MS (Methode 3): Rₜ = 1.33 min; MS (ESIpos): m/z = 267 [M+H]⁺.

### Beispiel 16A

### 2-Chlor-4-(4-hydroxyphenyl)-6-methoxypyridin-3,5-dicarbonitril

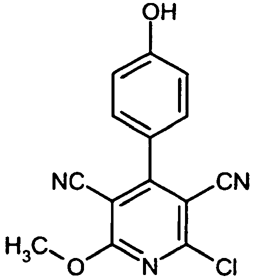

7.98 g (15.285 mmol) 2-Amino-4-(4-hydroxyphenyl)-6-methoxypyridin-3,5-dicarbonitril wurden in 120 ml Acetonitril vorgelegt, mit 6.175 ml (45.855 mmol) Isoamylnitrit und 6.165 g (45.855 mmol) Kupfer(II)chlorid versetzt und über Nacht bei Raumtemperatur gerührt. Das Reaktionsgemisch wurde mit 1N Salzsäure versetzt und mit Essigsäureethylester extrahiert. Die organische Phase wurde mit gesättigter wässriger Natriumhydrogencarbonat-Lösung und mit Wasser gewaschen, über Natriumsulfat getrocknet und eingeengt. Der Rückstand wurde über eine Kieselgelsäule (Eluent: Toluol/Essigsäureethylester 2:1) gereinigt. Man erhält 1.3 g (23 % d. Th) der Zielverbindung.

¹H-NMR (400 MHz, DMSO-d₆): δ = 10.29 (s, 1H), 7.49 (d, 2H), 6.98 (d, 2H), 4.12 (s, 3H).

LC-MS (Methode 1): Rₜ = 2.04 min; MS (ESIpos): m/z = 286 [M+H]⁺.

### Beispiel 17A

### 4-(4-Hydroxyphenyl)-2-methoxy-6-sulfanylpyridin-3,5-dicarbonitril

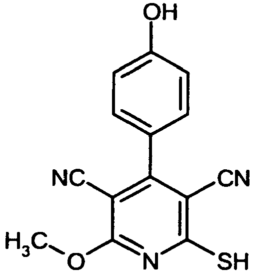

1.3 g (4.550 mmol) 2-Chlor-4-(4-hydroxyphenyl)-6-methoxypyridin-3,5-dicarbonitril wurden in 10 ml DMF vorgelegt, mit 426 mg (5.460 mmol) Natriumsulfid versetzt und über Nacht bei Raumtemperatur gerührt. Das Reaktionsgemisch wurde ohne weitere Aufreinigung weiter umgesetzt.

LC-MS (Methode 4): Rₜ = 0.74 min; MS (ESIpos): m/z = 284 [M+H]⁺.

### Beispiel 18A

### 4-(Chlormethyl)-N-(4-fluorphenyl)-1,3-thiazol-2-amin

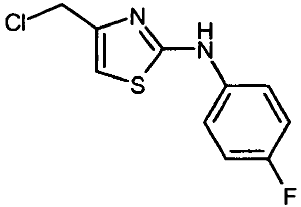

Methode A: Zu einer Suspension von 202 g (1.19 mol) 4-Fluorphenyl-thio-harnstoff in 660 ml Aceton dosierte man innerhalb 1.5h eine warme Lösung von 160 g (1.26 mol) 1,3-Dichloraceton in 480 ml Aceton und rührt 4.5h bei 40°C und über Nacht bei RT nach. Die Kristalle wurden abgesaugt, mit Aceton gewaschen und im Vakuum bei 50°C getrocknet.

Ausb.: 328 g farblose Kristalle (93% d. Th.)

Nach NMR bestand das erhaltene Produkt zu ca. 22% aus der gewünschten Titelverbindung im Gemisch mit ca. 78% der nicht-dehydratisierten Zwischenstufe 4-(Chlormethyl)-2-[(4-fluorophenyl)amino]-4,5-dihydro-1,3-thiazol-4-ol. Dieses Produkt wurde ohne weitere Trennung als solches weiter eingesetzt.

Methode B: 600 mg (4,7 mmol) 1,3-Dichloraceton und 768 mg (4.5 mmol) 4-Fluorphenyl-thioharnstoff wurden in 10 ml DMF gelöst und 4h bei 80°C gerührt. Es wurde mit 200 ml Wasser versetzt und dreimal mit Essigsäurethylester extrahiert. Die vereinigten organischen Phasen wurden mit Wasser und gesättigter wässriger Natriumchlorid-Lösung gewaschen über Natriumsulfat getrockent und zu einem dunklen Öl eingeengt. Dieser Rückstand wurde an 150g Kieselgel mit i-Hexan → i-Hexan / Essigsäurethylester (10:1) chromatographiert. Das Produkt wurde direkt weiter eingesetzt.

Ausb.: 714 mg (60% d.Th.)

LC-MS (Methode 5): Rₜ = 2.14 min; MS (ESIpos): m/z = 243 [M+H]⁺

¹H-NMR (400 MHz, DMSO-d₆): δ = 10.28 (s, 1H); 7.69-7.62 (m, 2H); 7.20-7.12 (m, 2H); 6.94 (s, 1H); 4.67 (s, 2H).

### Beispiel 19A

### 4-(Chlormethyl)-2-(4-chlorphenyl)-1,3-thiazol

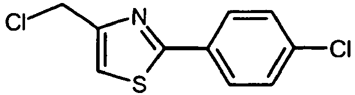

Die Darstellung erfolgte wie in WO 03/053441 für Beispiel 6 beschrieben.

### Beispiel 20A

### 4-(Chlormethyl)-2-(4-chlorphenyl)-1,3-oxazol

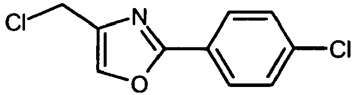

1 g (6.43 mmol) 4-Chlorbenzamid wurde zusammen mit 816 mg 1,3-Dichloraceton (6.43 mmol) 1h auf 135°C erhitzt. Man lässt auf RT abkühlen, gibt 1 ml konz. Schwefelsäure hinzu und rührt die Mischung 5 min. Dann wurde auf Eis gegossen und der Niederschlag abgesaugt (946 mg) Dieser Niederschlag wurde an 100 g Kieselgel durch Säulenchromatographie mit einem Gradienten aus Cyclohexan/Ethylacetat 20:1 bis 5:1 gereinigt.

Ausbeute: 532 mg (36% d. Th.)

LC-MS (Methode 5): Rₜ = 2.35 min; MS (ESIpos): m/z = 228 [M+H]⁺

¹H-NMR (400 MHz, DMSO-d₆): δ = 8.31(s, 1H), 8.0 (d, 2H), 7.62 (d, 2H), 4.75 (s, 2H).

### Beispiel 21A

### 4-(Chlormethyl)-2-(3,4-difluorphenyl)-1,3-thiazol

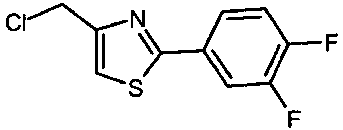

3.3 g (26 mmol) 1,3-Dichloraceton und 4.5 g mg (26 mmol) 3,4-Difluorphenyl-thioharnstoff wurden in 45 ml Ethanol über Nacht am Rückfluss gekocht. Das Lösungsmittel wurde im Vakuum verdampft und der Rückstand durch zweimalige Kieselgelchromatographie (Cyclohexan/Essigsäureethylester 4:1 und i-Hexan/ Essigsäureethylester 10:1) gereinigt.

Ausbeute: 1.94 g (30% d.Th.)

LC-MS (Methode 8): Rₜ = 3.26 min; MS (ESIpos): keine Ionisierung

¹H-NMR (400 MHz, DMSO-d₆): δ = 8.03-7.94 (m, 1H), 7.88 (s, 1H), 7.84-7.80 (m, 1H), 7.63-7.54 (m, 1H), 4.89 (s, 2H).

### Beispiel 22A

### 4-(Hydroxymethyl)-N-methylpyridin-2-carboxamid

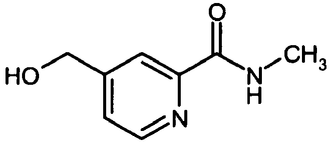

Die Darstellung wurde, wie in US 6,689,883 für Intermediate H beschrieben, durchgeführt.

### Beispiel 23A

### 4-(Chlormethyl)-N-methylpyridin-2-carboxamid-Hydrochlorid

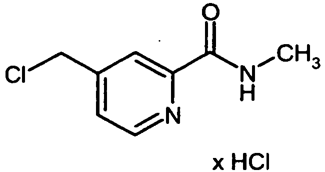

10 g (45.32 mmol) der Verbindung aus Beispiel 22A wurden in 160 ml Dichlormethan suspendiert und auf 0°C abgekühlt. Nach Zugabe von 16.18 g (135.96 mmol) Thionylchlorid wurde das Reaktionsgemisch auf RT erwärmt und über Nacht bei RT gerührt. Der Ansatz wurde danach eingedampft und der Rückstand im Hochvakuum getrocknet.

Ausbeute: 10 g (quant.)

LC-MS (Methode 4): Rₜ = 0.71 min; MS (ESIpos): m/z = 185 [M+H]⁺

¹H-NMR (400 MHz, DMSO-d₆): δ = 8.85-8.78 (m, 1H), 8.65 (d, 1H), 8.10 (s, 1H), 7.64 (d, 1H), 4.90 (s, 2H), 2.83 (d, 3H).

### Beispiel 24A

### 4-{[(6-Amino-3,5-dicyano-4-phenylpyridin-2-yl)sulfanyl]methyl}-N-methylpyridin-2-carboxamid

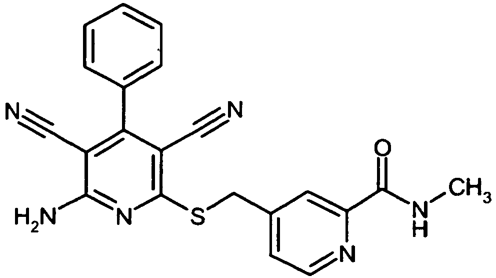

1 g (3.96 mmol) 2-Amino-4-phenyl-6-sulfanylpyridin-3,5-dicarbonitril, 0.96 g (4.36 mmol) 4-(Chlormethyl)-N-methylpyridin-2-carboxamid-Hydrochlorid und 1.33 g (15.84 mmol) Natriumhydrogencarbonat wurden in 20 ml DMF gelöst und für 2h bei RT gerührt. Das Reaktionsgemisch wurde mit 500 ml Wasser versetzt. Der Niederschlag wurde abfiltriert und mit Wasser gewaschen.

Ausbeute: 1.42 g (90% d. Th.)

LC-MS (Methode 3): Rₜ = 1.74 min; MS (ESIpos): m/z = 401 [M+H]⁺.

¹H-NMR (400 MHz, DMSO-d₆): δ (400 MHz) = 8.74 (q, 1H), 8.55 (d, 1H), 8.30-7.96 (s br, 2H), 8.15 (s, 1H), 7.80-7.75 (m, 1H), 7.58-7.50 (m, 5H), 4.60 (s, 2H), 2.81 (d, 3H).

### Beispiel 25A

### 4-{[(6-Chlor-3,5-dicyano-4-phenylpyridin-2-yl)thio]methyl}-N-methylpyridin-2-carboxamid

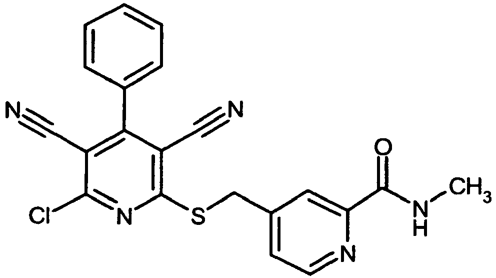

1.42 g (3.55 mmol) der Verbindung aus Beispiel 24A wurden in 30 ml Acetonitril vorgelegt, mit 0.96 ml (7.10 mmol) Isopentylnitrit und 0.95 g (7.10 mmol) Kupfer(II)chlorid versetzt und 5 Stunden bei 60°C gerührt. Anschließend wurde nochmals dieselbe Menge Kupfer(II)chlorid hinzugegeben und über Nacht bei 60°C gerührt. Der Ansatz wurde mit 7 ml 1N Salzsäure versetzt, dreimal mit Essigsäureethylester extrahiert und die vereinten organischen Phasen wurden mit gesättigter wässriger Natriumchlorid-Lösung gewaschen, über Natriumsulfat getrocknet und eingeengt. Der Rückstand wurde mittels präparativer HPLC (Zusatz 0.1 % TFA) gereinigt.

Ausbeute: 1.31 g (87 % d. Th.)

LC-MS (Methode 2): Rₜ = 2.46 min; MS (ESIpos): m/z = 420 [M+H]⁺.

### Beispiel 26A

### 4-{[(4R)-2,2-Dimethyl-1,3-dioxolan-4-yl]methoxy}benzolcarbaldehyd

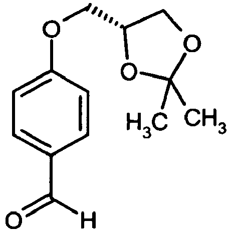

2 g (16.38 mmol) p-Methoxybenzaldehyd wurden gemeinsam mit 3.7 g (24.57 mmol) (4S)-4-(Chlormethyl)-2,2-dimethyl-1,3-dioxolan und 15.8 g (114.64 mmol) Kaliumcarbonat über Nacht bei 130°C in 10 ml DMF gerührt. Anschließend wurde der Ansatz auf Wasser gegeben und mit Methylenchlorid extrahiert. Die organische Phase wurde mit gesättigter wässriger Natriumchlorid-Lösung gewaschen, über Natriumsulfat getrocknet und eingeengt. Der Rückstand wurde an einer Kieselgelsäule (Eluent: Cyclohexan/ Essigester 5:1) gereinigt. Es wurden 2.42 g (61 % d. Th.) der Zielverbindung erhalten.

¹H-NMR (400 MHz, DMSO-d₆): δ = 9.87 (s, 1H), 7.87 (d, 2H), 7.15 (d, 2H), 4.47-4.41 (m, 1H), 4.19-4.15 (m, 1H), 4.13-4.08 (m, 2H), 3.79-3.75 (m, 1H), 1.36 (s, 3H), 1.31 (s, 3H).

LC-MS (Methode 4): Rₜ = 1.00 min; MS (ESIpos): m/z = 237 [M+H]⁺.

### Beispiel 27A

### 2-Amino-4-(4-{[(4R)-2,2-dimethyl-1,3-dioxolan-4-yl]methoxy}phenyl)-6-methoxypyridin-3,5-dicarbonitril

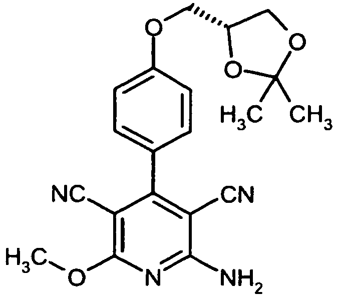

1.2 g (5.08 mmol) 4-{[(4R)-2,2-Dimethyl-1,3-dioxolan-4-yl]methoxy}benzolcarbaldehyd und 688 mg (10.41 mmol) Malonsäuredinitril wurden in 10 ml Methanol vorgelegt, mit 1.65 g (30.47 mmol) Natriummethylat (30 %ig in Methanol gelöst) versetzt und 2 Stunden bei Raumtemperatur gerührt. Der ausgefallene Feststoff wurde abfiltriert, mit Methanol gewaschen und im Hochvakuum getrocknet. Es wurden 451 mg (23 % d. Th.) der Zielverbindung erhalten.

¹H-NMR (400 MHz, DMSO-d₆): δ = 7.92 (s, 2H), 7.47 (d, 2H), 7.13 (d, 2H), 4.47-4.41 (m, 1H), 4.14-4.05 (m, 3H), 3.96 (s, 3H), 3.80-3.75 (m, 1H), 1.37 (s, 3H), 1.32 (s, 3H).

LC-MS (Methode 2): Rₜ = 2.19 min; MS (ESIpos): m/z = 381 [M+H]⁺.

### Beispiel 28A

### 2-Chlor-4-(4-{[(2S)-2,3-dihydroxypropyl]oxy}phenyl)-6-methoxypyridin-3,5-dicarbonitril

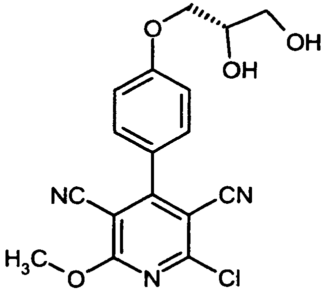

450 mg (1.18 mmol) 2-Amino-4-(4-{[(4R)-2,2-dimethyl-1,3-dioxolan-4-yl]methoxy}phenyl)-6-methoxypyridin-3,5-dicarbonitril wurden gemeinsam mit 478 µl (3.55 mmol) Isoamylnitrit und 477 mg (3.55 mmol) Kupfer(II)chlorid 4 Stunden bei 65°C in 10 ml Acetonitril gerührt. Das Reaktionsgemisch wurde mittels präparativer HPLC (Acetonitril/Wasser: 10:90 → 95:5) gereinigt. Es wurden 202 mg (44 % d. Th.) der Zielverbindung erhalten.

¹H-NMR (400 MHz, DMSO-d₆): δ = 7.59 (d, 2H), 7.18 (d, 2H), 4.13 (s, 3H), 4.09 (d, 1H), 4.01-3.92 (m, 1H), 3.85-3.80 (m, 1H), 3.47 (d, 2H).

LC-MS (Methode 4): Rₜ = 1.02 min; MS (ESIneg): m/z = 340 [M-H-H₂O]⁺.

### Beispiel 29A

### 4-(4-{[(2S)-2,3-Dihydroxypropyl]oxy}phenyl)-2-methoxy-6-sulfanylpyridin-3,5-dicarbonitril

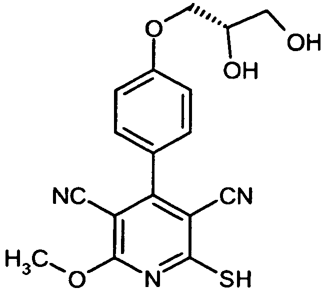

100 mg (0.278 mmol) 2-Chlor-4-(4-{[(2S)-2,3-dihydroxypropyl]oxy}phenyl)-6-methoxypyridin-3,5-dicarbonitril wurden in 5 ml DMF vorgelegt, mit 26 mg (0.334 mmol) Natriumsulfid versetzt und 4 Stunden bei Raumtemperatur gerührt. Das Reaktionsgemisch wurde ohne weitere Aufreinigung weiter umgesetzt.

LC-MS (Methode 4): Rₜ = 0.69 min; MS (ESIpos): m/z = 358 [M+H]⁺.

### Beispiel 30A

### [2-(4-Chlorphenyl)-1,3-oxazol-4-yl]methanol

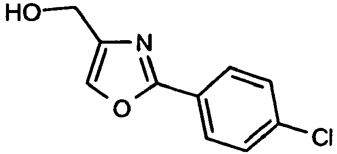

6 g (26.307 mmol) 4-(Chlormethyl)-2-(4-chlorphenyl)-1,3-oxazol wurden 3 h in 526 ml (52.613 mmol) 0.1 N Natronlauge unter Rückfluss erhitzt. Anschließend wurde auf Raumtemperatur abgekühlt, der ausgefallene Feststoff abfiltriert, mit 0.1N Natronlauge gewaschen und im Hochvakuum getrocknet. Man erhielt 4.79 g (85 % d. Th.) der Zielverbindung.

LC-MS (Methode 4): Rₜ = 0.94 min; MS (ESIpos): m/z = 210 [M+H]⁺.

### Ausführungsbeispiele:

### Beispiel 1

### 2-({[2-(4-Chlorphenyl)-1,3-thiazol-4-yl]methyl}sulfanyl)-4-phenyl-6-propoxypyridin-3,5-dicarbonitril

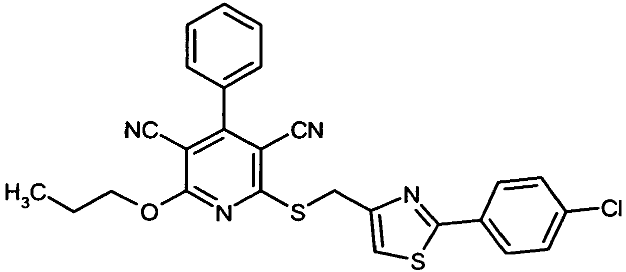

37 µl (0.501 mmol) 1-Propanol wurden in 2 ml DMF vorgelegt, mit 20 mg (0.184 mmol) Kalium-tert.-butylat versetzt und 15 Minuten gerührt, bevor 100 mg (0.167 mmol) 2-Chlor-6-({[2-(4-chlorphenyl)-1,3-thiazol-4-yl]methyl}sulfanyl)-4-phenylpyridin-3,5-dicarbonitril, in 2 ml DMF gelöst, zugegeben wurden. Das Reaktionsgemisch wurde eine Stunde bei Raumtemperatur gerührt und anschließend mittels präparativer HPLC (Acetonitril/Wasser: 10:90 → 95:5, Zusatz von 0.1 % TFA) gereinigt. Es wurden 13 mg (15 % d. Th.) der Zielverbindung erhalten.

¹H-NMR (400 MHz, DMSO-d₆): δ = 7.95 (d, 2H), 7.74 (s, 1H), 7.62-7.57 (m, 7H), 4.79 (s, 2H), 4.53 (t, 2H), 1.79-1.70 (m, 2H), 0.93 (t, 3H).

LC-MS (Methode 1): Rₜ = 3.07 min; MS (ESIpos): m/z = 503 [M+H]⁺.

### Beispiel 2

### 2-({[2-(4-Chlorphenyl)-1,3-thiazol-4-yl]methyl}sulfanyl)-6-(3-hydroxypropoxy)-4-phenylpyridin-3,5-dicarbonitril

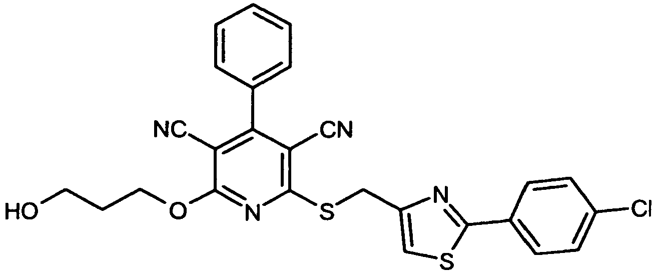

68 µl (0.939 mmol) 1,3-Propandiol wurden in 2 ml DMF vorgelegt, mit 38 mg (0.344 mmol) Kalium-tert.-butylat versetzt und 15 Minuten gerührt, bevor 150 mg (0.313 mmol) 2-Chlor-6-({[2-(4-chlorphenyl)-1,3-thiazol-4-yl]methyl}sulfanyl)-4-phenylpyridin-3,5-dicarbonitril, in 2 ml DMF gelöst, zugegeben wurden. Das Reaktionsgemisch wurde 1 Stunde bei Raumtemperatur gerührt und anschließend mittels präparativer HPLC (Acetonitril/Wasser: 10:90 → 95:5, Zusatz von 0.1 % TFA) gereinigt. Es wurden 98 mg (59 % d. Th.) der Zielverbindung erhalten.

¹H-NMR (400 MHz, DMSO-d₆): δ = 7.96 (d, 2H), 7.75 (s, 1H), 7.62-7.57 (m, 7H), 4.78 (s, 2H), 4.68 (t, 2H), 3.55 (t, 2H), 1.94-1.87 (m, 2H).

LC-MS (Methode 2): Rₜ = 2.90 min; MS (ESIpos): m/z = 519 [M+H]⁺.

### Beispiel 3

### 2-({[2-(4-Chlorphenyl)-1,3-thiazol-4-yl]methyl}sulfanyl)-4-[4-(2-hydroxyethoxy)phenyl]-6-(3-hydroxypropoxy)pyridin-3,5-dicarbonitril

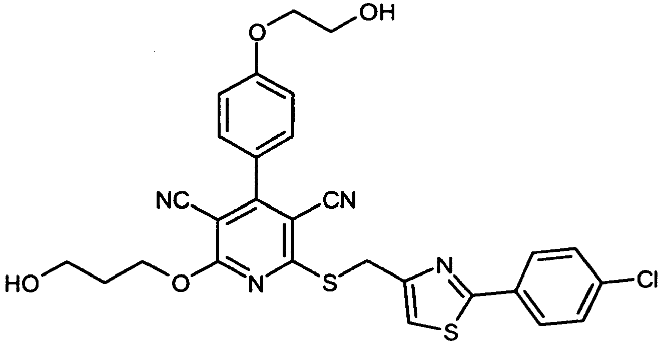

Die Darstellung erfolgt in Analogie zu Beispiel 2 aus Beispiel 4A.

¹H-NMR (400 MHz, DMSO-d₆): δ = 7.97 (d, 2H), 7.74 (s, 1H), 7.60-7.52 (m, 4H), 7.15 (d, 2H), 4.91 (br s, 1H), 4.80 (s, 2H), 4.70-4.58 (m, 3H), 4.09 (t, 2H), 3.74 (t, 2H), 3.54 (t, 2H), 1.90 (Quintett, 2H).

LC-MS (Methode 4): Rₜ = 1.36 min; MS (ESIpos): m/z = 579 [M+H]⁺.

### Beispiel 4

### 2-({[2-(4-Chlorphenyl)-1,3-thiazol-4-yl]methyl}sulfanyl)-4-[4-(2-hydroxyethoxy)phenyl]-6-(2-methylpropoxy)pyridin-3,5-dicarbonitril

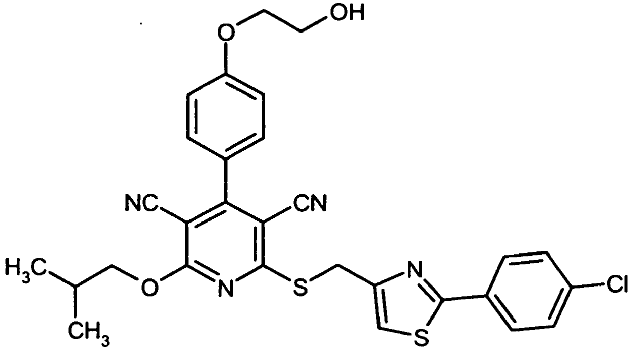

Die Darstellung erfolgt in Analogie zu Beispiel 2 aus Beispiel 4A.

¹H-NMR (400 MHz, DMSO-d₆): δ = 7.97 (d, 2H), 7.71 (s, 1H), 7.61-7.53 (m, 4H), 7.15 (d, 2H), 4.92 (t, 1H), 4.79 (s, 2H), 4.30 (d, 2H), 4.09 (t, 2H), 3.74 (q, 2H), 2.05 (Septett, 1H), 0.91 (d, 6H).

LC-MS (Methode 3): Rₜ = 2.76 min; MS (ESIpos): m/z = 577 [M+H]⁺.

### Beispiel 5

### 2-({[2-(4-Chlorphenyl)-1,3-thiazol-4-yl]methyl}sulfanyl)-6-[(1-methylpyrrolidin-3-yl)oxy]-4-phenylpyridin-3,5-dicarbonitril

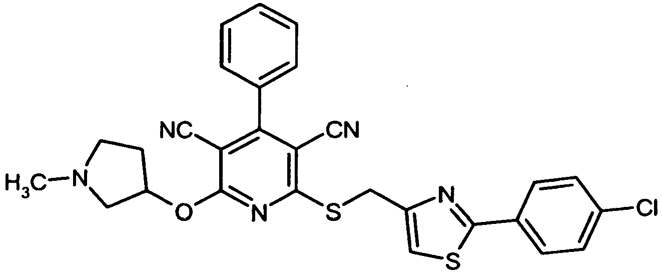

25 µl (0.229 mmol) 3-Hydroxy-N-methylpyrrolidin wurden in 0.5 ml THF vorgelegt, auf 0°C abgekühlt und mit 249 µl (0.249 mmol) Phosphazen-Base P(4)-t-Bu (1M in THF) versetzt und 10 Minuten bei dieser Temperatur gerührt. Anschließend wurden 120 mg (0.208 mmol) der Verbindung aus Beispiel 3A zugegeben und über Nacht bei RT gerührt. Das Reaktionsgemisch wurde anschließend mittels präparativer HPLC (Zusatz von 0.15% Salzsäure) gereinigt. Es wurden 38 mg der verunreinigten Zielverbindung erhalten. Dies wurde mit Dichlormethan gelöst, zweimal mit 1N Salzsäure, einmal mit Wasser und einmal mit gesättigter wässriger Natriumchlorid-Lösung gewaschen. Nach Trocknen über Natriumsulfat wurde die organische Phase einrotiert und das Rohprodukt nochmals über präparativer HPLC (Zusatz von 0.15 % Salzsäure) gereinigt.

Ausbeute: 7 mg (6 % d. Th.)

¹H-NMR (400 MHz, DMSO-d₆): δ = 10.40 (br s, 1H), 7.98 (d, 2H), 7.80 (d, 1H), 7.65-7.58 (m, 7H), 5.96-5.87 (m, 1H), 4.82 (s, 2H), 4.10-3.83 (m, 1H), 3.78-3.68 (m, 1H), 3.58-3.42 (m, 1H), 3.30-3.09 (m, 1H), 2.89 (d, 3H), 2.44-2.18 (m, 2H).

LC-MS (Methode 2): Rₜ = 2.00 min; MS (ESIpos): m/z = 544 [M+H]⁺.

### Beispiel 6

### 2-({[2-(4-Chlorphenyl)-1,3-thiazol-4-yl]methyl}sulfanyl)-6-(3-hydroxypropoxy)-4-(1,3-triazol-5-yl)pyridin-3,5-dicarbonitril

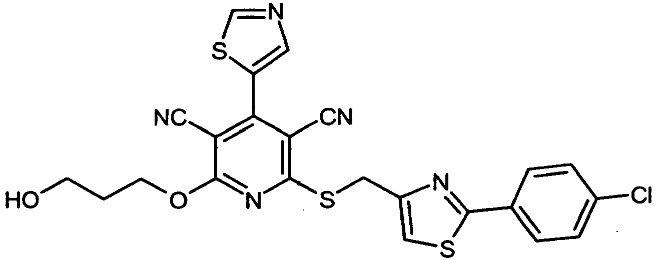

30 µl (0.419 mmol) 1,3-Propandiol wurden in 2 ml DMF vorgelegt, mit 18 mg (0.154 mmol) Kalium-tert.-butylat versetzt und 15 Minuten gerührt, bevor 75 mg (0.14 mmol) 2-Chlor-6-({[2-(4-chlorphenyl)-1,3-thiazol-4-yl]methyl}sulfanyl)-4-(1,3-thiazol-5-yl)pyridin-3,5-dicarbonitril, in 2 ml DMF gelöst, zugegeben wurden. Das Reaktionsgemisch wurde 30 Minuten bei Raumtemperatur gerührt und anschließend mittels präparativer HPLC (Acetonitril/Wasser: 10:90 → 95:5) gereinigt. Es wurden 11 mg (15 % d. Th.) der Zielverbindung erhalten.

¹H-NMR (400 MHz, DMSO-d₆): δ = 9.45 (d, 1H), 8.38 (d, 1H), 7.95 (d, 2H), 7.74 (s, 1H), 7.58 (d, 2H), 4.81 (s, 2H), 4.68 (t, 2H), 4.64 (t, 1H), 3.53 (q, 2H), 1.93-1.87 (m, 2H).

LC-MS (Methode 3): Rₜ = 2.22 min; MS (ESIpos): m/z = 526 [M+H]⁺.

### Beispiel 7

### 2-({[2-(4-Chlorphenyl)-1,3-oxazol-4-yl]methyl}sulfanyl)-4-[4-(2-hydroxyethoxy)phenyl]-6-methoxypyridin-3,5-dicarbonitril

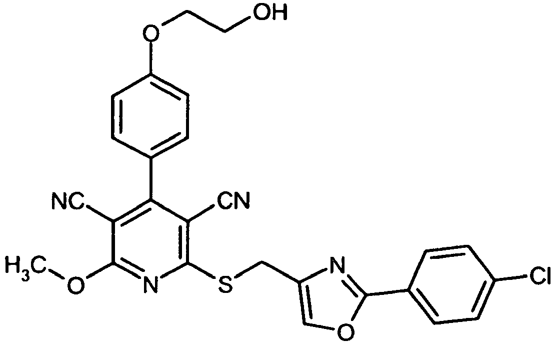

400 mg (1.222 mmol) 4-[4-(2-Hydroxyethoxy)phenyl]-2-methoxy-6-sulfanylpyridin-3,5-dicarbonitril wurden gemeinsam mit 306 mg (1.344 mmol) 4-(Chlormethyl)-2-(4-chlorphenyl)-1,3-oxazol und 410 mg (4.888 mmol) Natriumhydrogencarbonat in 8 ml DMF über Nacht bei Raumtemperatur gerührt. Die Reaktionsmischung wurde mittels präparativer HPLC (Acetonitril/ Wasser: 10:90 → 95:5) gereinigt. Es wurden 10.4 mg (1.6 % d. Th.) der Zielverbindung erhalten.

¹H-NMR (400 MHz, DMSO-d₆): δ = 8.21 (s, 1H), 7.97 (d, 2H), 7.61 (d, 2H), 7.54 (d, 2H), 7.14 (d, 2H), 4.87 (t, 1H), 4.63 (s, 2H), 4.21 (s, 3H), 4.09 (t, 2H), 3.75 (m, 2H).

LC-MS (Methode 4): Rₜ = 1.39 min; MS (ESIpos): m/z = 519 [M+H]⁺.

### Beispiel 8

### 2-({[2-(4-Chlorphenyl)-1,3-thiazol-4-yl]methyl}sulfanyl)-4-[4-(2-hydroxyethoxy)phenyl]-6-methoxypyridin-3,5-dicarbonitril

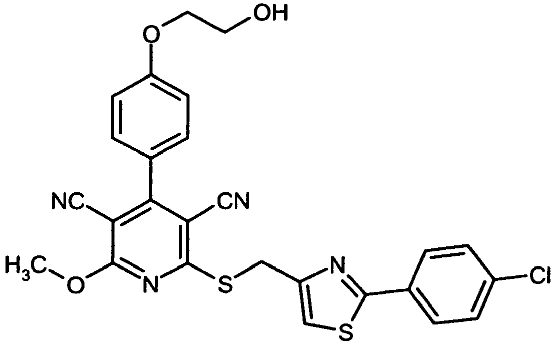

2.095 g (6.399 mmol) 4-[4-(2-Hydroxyethoxy)phenyl]-2-methoxy-6-sulfanylpyridin-3,5-dicarbonitril wurden gemeinsam mit 1.875 g (7.679 mmol) 4-(Chlormethyl)-2-(4-chlorphenyl)-1,3-thiazol und 1.613 g (19.197 mmol) Natriumhydrogencarbonat in 50 ml DMF über Nacht bei Raumtemperatur gerührt. Die Reaktionsmischung wurde mit Wasser und Methanol versetzt und 2 Minuten ins Ultraschallbad gestellt. Es fiel ein weißer Feststoff aus, welcher abfiltriert, mit Methanol gewaschen und am Hochvakuum getrocknet wurde. Die Mutterlauge wurde auf die Hälfte eingeengt und 30 Minuten in den Kühlschrank gegeben. Der ausgefallene Feststoff wurde abfiltriert, mit Methanol gewaschen und am Hochvakuum getrocknet. Die beiden ausgefallenen Feststoffe wurden vereinigt, so dass insgesamt 2.98 g (85 % d. Th.) der Zielverbindung erhalten wurden.

¹H-NMR (400 MHz, DMSO-d₆): δ = 7.95 (d, 2H), 7.76 (s, 1H), 7.63-7.53 (m, 4H), 7.15 (d, 2H), 4.93 (s br, 1H), 4.82 (s, 2H), 4.18 (s, 3H), 4.09 (t, 2H), 3.75 (t br, 2H).

LC-MS (Methode 3): Rₜ = 2.45 min; MS (ESIpos): m/z = 535 [M+H]⁺.

### Beispiel 9

### 3-[({3,5-Dicyano-4-[4-(2-hydroxyethoxy)phenyl]-6-methoxypyridin-2-yl}thio)methyl]benzoesäure

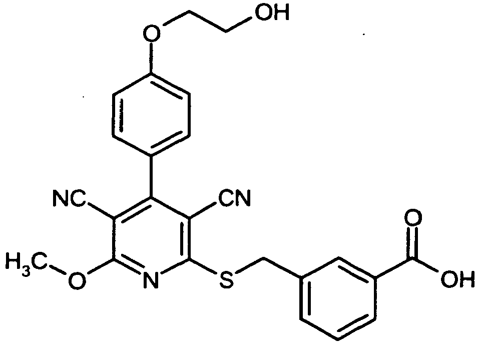

50 mg (0.153 mmol) der Verbindung aus Beispiel 10A wurden gemeinsam mit 29 mg (0.168 mmol) 3-(Chlormethyl)-benzoesäure und 38.5 mg (0.458 mmol) Natriumhydrogencarbonat in 0.6 ml DMF 2 h bei Raumtemperatur gerührt. Die Reaktionsmischung wurde mit Wasser versetzt und mittels präparativer HPLC (Zusatz von 0.15 % Salzsäure) gereinigt.

Ausbeute: 28 mg (39% d. Th.)

¹H-NMR (400 MHz, DMSO-d₆): δ = 13.05 (s br, 1H), 8.11 (s, 1H), 7.84 (d, 1H), 7.76 (d, 1H), 7.56 (d, 2H), 7.50 (t, 1H), 7.13 (d, 2H), 4.73 (s, 2H), 4.18 (s, 3H), 4.09 (t, 2H), 3.80 (br s, 1H), 3.74 (t, 2H).

LC-MS (Methode 9): Rₜ = 3.28 min; MS (ESIpos): m/z = 462 [M+H]+.

### Beispiel 10

### 3-{[(3,5-Dicyano-6-methoxy-4-phenylpyridin-2-yl)thio]methyl}benzoesäure

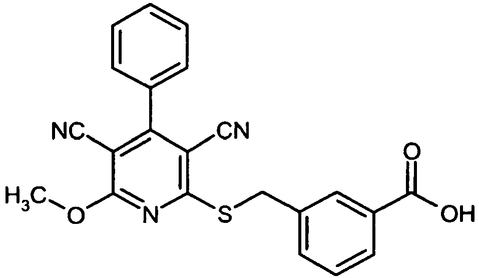

75 mg (0.177 mmol) der Verbindung aus Beispiel 11A wurden gemeinsam mit 33 mg (0.194 mmol) 3-(Chlormethyl)-benzoesäure und 44.5 mg (0.530 mmol) Natriumhydrogencarbonat in 1.0 ml DMF über Nacht bei Raumtemperatur gerührt. Die Reaktionsmischung wurde mit Wasser versetzt und mittels präparativer HPLC (Zusatz von 0.15 % Salzsäure) gereinigt.

Ausbeute: 67 mg (94% d. Th.)

¹H-NMR (400 MHz, DMSO-d₆): δ = 13.08 (s br, 1H), 8.12 (s, 1H), 7.86 (d, 1H), 7.76 (d, 1H), 7.62-7.56 (m, 5H), 7.50 (t, 1H), 4.75 (s, 2H), 4.19 (s, 3H).

LC-MS (Methode 10): Rₜ = 3.50 min; MS (ESIpos): m/z = 402 [M+H]+.

### Beispiel 11

### 4-[4-(2-Hydroxyethoxy)phenyl]-2-methoxy-6-[(3-methoxybenzyl)sulfanyl]pyridin-3,5-dicarbonitril

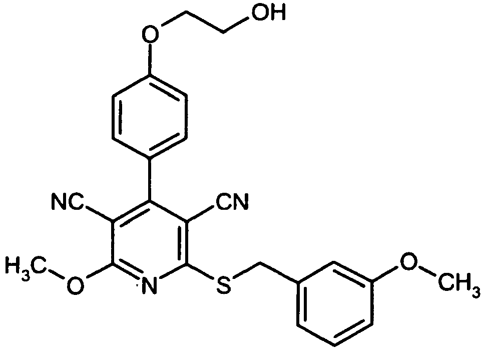

75 mg (0.229 mmol) 4-[4-(2-Hydroxyethoxy)phenyl]-2-methoxy-6-sulfanylpyridin-3,5-dicarbonitril wurden gemeinsam mit 36 µl (0.253 mmol) 3-Methoxybenzylbromid und 52 mg (0.380 mmol) Kaliumcarbonat in 1 ml DMF über Nacht bei Raumtemperatur gerührt. Die Reaktionsmischung wurde mittels präparativer HPLC (Acetonitril/ Wasser: 10:90 → 95:5, Zusatz von 0.1 % Ameisensäure) gereinigt. Es wurden 16 mg (56 % d. Th.) der Zielverbindung erhalten.

¹H-NMR (400 MHz, DMSO-d₆): δ = 7.56 (d, 2H), 7.28 (t, 1H), 7.15 (d, 2H), 7.07-7.06 (m, 2H), 6.88 (m, 1H), 4.95 (t, 1H), 4.65 (s, 2H), 4.17 (s, 3H), 4.10 (t, 2H), 3.75 (m, 2H).

LC-MS (Methode 5): Rₜ = 2.39 min; MS (ESIpos): m/z = 448 [M+H]⁺.

### Beispiel 12

### 4-[4-(2-Hydroxyethoxy)phenyl]-2-methoxy-6-[(pyridin-3-ylmethyl)sulfanyl]pyridin-3,5-dicarbonitril

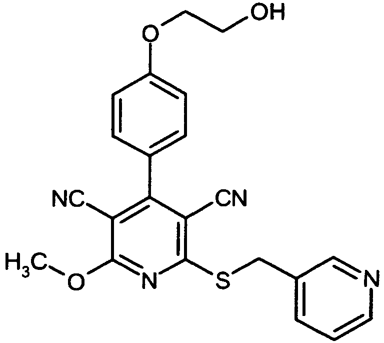

494 mg (1.5 mmol) 2-Chlor-4-[4-(2-hydroxyethoxy)phenyl]-6-methoxypyridin-3,5-dicarbonitril wurden in 5 ml DMF vorgelegt, mit 140 mg (1.8 mmol) Natriumsulfid versetzt und 3 Stunden bei Raumtemperatur gerührt. Anschließend wurden 311 mg (2.25 mmol) Kaliumcarbonat und 307 mg (1.8 mmol) 3-Picolylchlorid-Hydrochlorid zugegeben. Das Reaktionsgemisch wurde über Nacht bei 45°C gerührt und anschließend mittels präparativer HPLC (Acetonitril/ Wasser: 10:90 → 95:5, Zusatz von 0.1 % Ameisensäure) gereinigt. Es wurden 255 mg (40 % d. Th.) der Zielverbindung erhalten.

¹H-NMR (400 MHz, DMSO-d₆): δ = 8.71 (s, 1H), 8.50 (d, 1H), 7.90 (d, 1H), 7.54 (d, 2H), 7.40 (dd, 1H), 7.14 (d, 2H), 4.70 (s, 2H), 4.13 (s, 3H), 4.10 (t, 2H), 3.75 (m, 2H).

LC-MS (Methode 5): Rₜ = 1.63 min; MS (ESIpos): m/z = 419 [M+H]⁺.

### Beispiel 13

### 2-[({2-[(4-Fluorphenyl)amino]-1,3-thiazol-4-yl}methyl)sulfanyl]-4-[4-(2-hydroxyethoxy)phenyl]-6-methoxypyridin-3,5-dicarbonitril

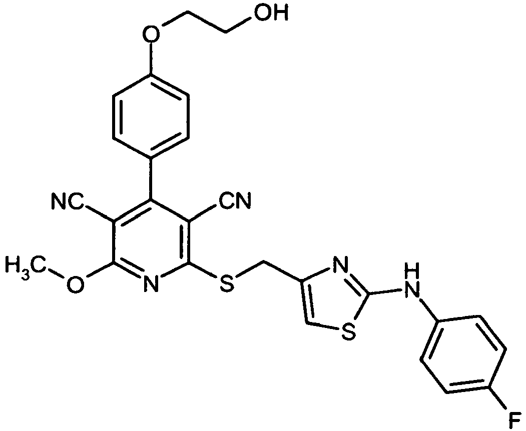

122 mg (0.371 mmol) 4-[4-(2-Hydroxyethoxy)phenyl]-2-methoxy-6-sulfanylpyridin-3,5-dicarbonitril wurden in 3 ml DMF und 1 ml Ethanol gelöst, mit 118 mg (0.397 mmol) der Verbindung aus Beispiel 18A (Methode A), 130 mg (0.94 mmol) Kaliumcarbonat und 20 mg (0.53 mmol) Natriumborhydrid versetzt und über Nacht bei 50°C gerührt. Nach Zugabe von 0.4 ml 5N Essigsäure wurde mittels präparativer HPLC (Acetonitril/ Wasser: 10:90 → 95:5, Zusatz von 0.1 % Ameisensäure) gereinigt. Es wurden 59 mg (30 % d. Th.) der Zielverbindung erhalten.

¹H-NMR (400 MHz, DMSO-d₆): δ = 10.27 (s, 1H), 7.61-7.59 (m, 1H), 7.55 (d, 2H), 7.15 (d, 2H), 7.12 (t, 2H), 6.86 (s, 1H), 4.93 (t, 1H), 4.61 (s, 2H), 4.19 (s, 3H), 4.09 (t, 2H), 3.74 (q, 2H).

LC-MS (Methode 5): Rₜ = 2.42 min; MS (ESIpos): m/z = 534[M+H]⁺.

### Beispiel 14

### 2-({[2-(3,4-Difluorphenyl)-1,3-thiazol-4-yl]methyl}sulfanyl)-4-[4-(2-hydroxyethoxy)phenyl]-6-methoxypyridin-3,5-dicarbonitril

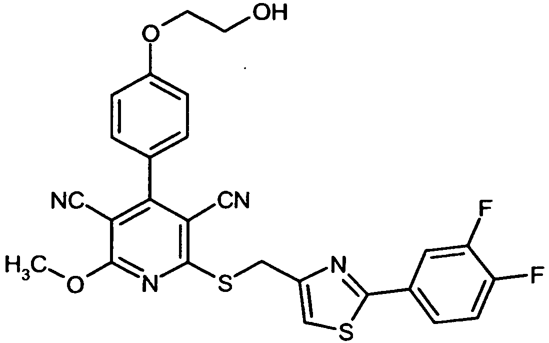

36 mg (0.111 mmol) 2-Chlor-4-[4-(2-hydroxyethoxy)phenyl]-6-methoxypyridin-3,5-dicarbonitril wurden in 1 ml DMF vorgelegt, mit 34 mg (0.138 mmol) 4-(Chlormethyl)-2-(3,4-difluorphenyl)-1,3-thiazol und 28 mg (0.332 mmol) Natriumhydrogencarbonat versetzt und über Nacht bei Raumtemperatur gerührt. Das Reaktionsgemisch wurde mittels präparativer HPLC (Acetonitril/ Wasser: 10:90 → 95:5, Zusatz von 0.1 % Ameisensäure) gereinigt. Es wurden 33 mg (48 % d. Th.) der Zielverbindung erhalten.

¹H-NMR (400 MHz, DMSO-d₆): δ = 7.99-7.95 (m, 1H), 7.80-7.77 (m, 2H), 7.61-7.57 (m, 1H), 7.15 (d, 2H), 4.93 (t, 1H), 4.81 (s, 2H), 4.18 (s, 3H), 4.09 (t, 2H), 3.75 (q, 2H).

LC-MS (Methode 11): Rₜ = 2.80 min; MS (ESIpos): m/z = 537 [M+H]⁺.

### Beispiel 15

### 2-({[2-(4-Chlorphenyl)-1,3-oxazol-4-yl]methyl}sulfanyl)-6-ethoxy-4-[4-(2-hydroxyethoxy)phenyl]pyridin-3,5-dicarbonitril

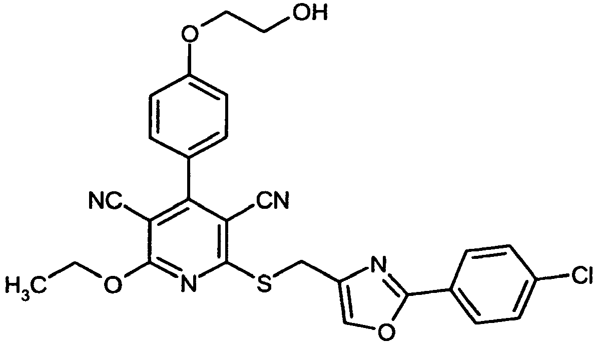

100 mg (0.293 mmol) 2-Ethoxy-4-[4-(2-hydroxyethoxy)phenyl]-6-sulfanylpyridin-3,5-dicarbonitril wurden gemeinsam mit 73 mg (0.322 mmol) 4-(Chlormethyl)-2-(4-chlorphenyl)-1,3-oxazol und 98 mg (1.172 mmol) Natriumhydrogencarbonat in 2 ml DMF über Nacht bei Raumtemperatur gerührt. Die Reaktionsmischung wurde mittels präparativer HPLC (Acetonitril/ Wasser: 10:90 → 95:5) gereinigt. Es wurden 41.5 mg (26 % d. Th.) der Zielverbindung erhalten.

¹H-NMR (400 MHz, DMSO-d₆): δ = 8.23 (s, 1H), 7.96 (d, 2H), 7.62 (d, 2H), 7.56 (d, 2H), 7.16 (d, 2H), 4.92 (s, 1H), 4.66 (m, 2H), 4.60 (s, 2H), 4.09 (m, 2H), 3.75 (m, 2H), 1.38 (m, 3H).

LC-MS (Methode 4): Rₜ = 1.45 min; MS (ESIpos): m/z = 533 [M+H]⁺.

### Beispiel 16

### 2-({[2-(4-Chlorphenyl)-1,3-thiazol-4-yl]methyl}sulfanyl)-6-ethoxy-4-[4-(2-hydroxyethoxy)phenyl]pyridin-3,5-dicarbonitril

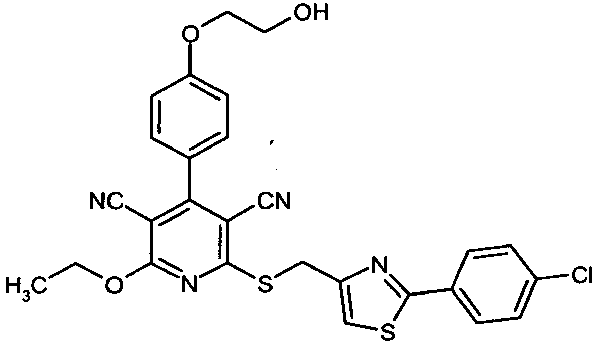

100 mg (0.293 mmol) 2-Ethoxy-4-[4-(2-hydroxyethoxy)phenyl]-6-sulfanylpyridin-3,5-dicarbonitril wurden gemeinsam mit 78 mg (0.322 mmol) 4-(Chlormethyl)-2-(4-chlorphenyl)-1,3-thiazol und 98 mg (1.172 mmol) Natriumhydrogencarbonat in 2 ml DMF über Nacht bei Raumtemperatur gerührt. Die Reaktionsmischung wurde mittels präparativer HPLC (Acetonitril/ Wasser: 10:90 → 95:5) gereinigt. Es wurden 77 mg (47 % d. Th.) der Zielverbindung erhalten.

¹H-NMR (400 MHz, DMSO-d₆): δ = 7.95 (d, 2H), 7.74 (s, 1H), 7.58 (d, 2H), 7.55 (d, 2H), 7.14 (d, 2H), 4.92 (t, 1H), 4.78 (s, 2H), 4.63 (q, 2H), 4.09 (t, 2H), 3.75 (q, 2H), 1.34 (t, 3H).

LC-MS (Methode 4): Rₜ = 1.51 min; MS (ESIpos): m/z = 549 [M+H]⁺.

### Beispiel 17

### 2-({[2-(4-Chlorphenyl)-1,3-oxazol-4-yl]methyl}sulfanyl)-4-(4-hydroxyphenyl)-6-methoxypyridin-3,5-dicarbonitril

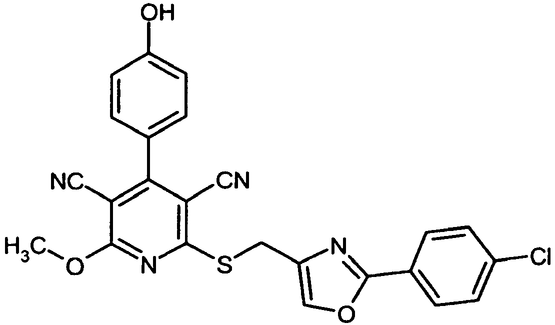

64 mg (0.224 mmol) 4-(4-Hydroxyphenyl)-2-methoxy-6-sulfanylpyridin-3,5-dicarbonitril wurden gemeinsam mit 88 mg (0.269 mmol) 4-(Chlormethyl)-2-(4-chlorphenyl)-1,3-oxazol und 57 mg (0.672 mmol) Natriumhydrogencarbonat in 2 ml DMF über Nacht bei Raumtemperatur gerührt. Die Reaktionsmischung wurde mittels präparativer HPLC (Acetonitril/ Wasser: 10:90 → 95:5, Zusatz von 0.1 % TFA) gereinigt. Es wurden 44 mg (41 % d. Th.) der Zielverbindung erhalten.

¹H-NMR (400 MHz, DMSO-d₆): δ = 10.2 (s, 1H), 8.23 (s, 1H), 7.97 (d, 2H), 7.61 (d, 2H), 7.44 (d, 2H), 6.95 (d, 2H), 4.62 (s, 2H), 4.20 (s, 3H).

LC-MS (Methode 4): Rₜ = 1.43 min; MS (ESIpos): m/z = 475 [M+H]⁺.

### Beispiel 18

### 4-({[3,5-Dicyan-6-(2-hydroxyethoxy)-4-phenylpyridin-2-yl]thio}methyl)-N-methylpyridin-2-carboxamid

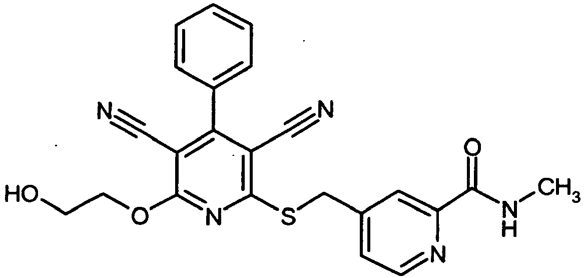

22 mg (0.357 mmol) 1,2-Ethandiol wurden in 0.5 ml DMF vorgelegt und mit 4.8 mg (0.179 mmol) Natriumhydrid versetzt. Nach 15 min bei RT wurden 50 mg (0.119 mmol) der Verbindung aus Beispiel 25A hinzugegeben und der Reaktionsansatz wurde über Nacht bei 115°C gerührt. Nach dem Abkühlen wurde die Reaktionslösung mittels präp. HPLC gereinigt.

Ausbeute: 19 mg (28 % d. Th.)

¹H-NMR (400 MHz, DMSO-d₆): δ = 8.79 (q, 1H), 8.58 (d, 1H), 8.10 (d, 1H), 7.70-7.58 (m, 6H), 4.69 (br s, 1H), 4.56 (t, 2H), 3.80 (t, 2H), 2.82 (d, 3H).

LC-MS (Methode 1): Rₜ = 1.95 min; MS (ESIpos): m/z = 446 [M+H]⁺.

Die in Tabelle 1 aufgeführten Beispiele wurden aus den entsprechenden Ausgangsverbindungen analog zu Beispiel 18 mit anschließender Aufreinigung hergestellt:

**Tabelle 1:**

| **Beis piel Nr.** | **Struktur (Ausbeute)** | **LC-MS: Rₜ [min] (Method e); MS (ESI): m/z [M+H]⁺** | **¹H-NMR (DMSO-d₆):** |
|---|---|---|---|
| **19** | 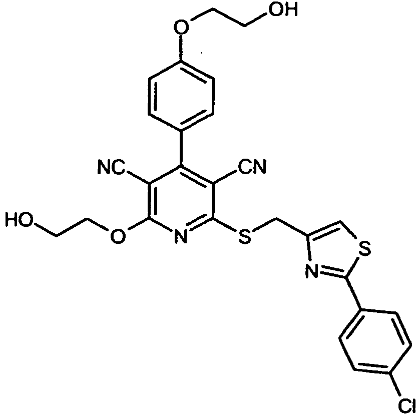 | 2.12 min (Method e 3); m/z = 565 | δ (400 MHz) = 7.97 (d, 2H), 7.74 (s, 1H), 7.59-7.52 (m, 4H), 7.15 (d, 2H), 4.98 (t, 1H), 4.91 (t, 1H), 4.79 (s, 2H), 4.63 (t, 2H), 4.09 (t, 2H), 3.78-3.70 (m, 4H). |
| | (12% d. Th.) | | |
| **20** | 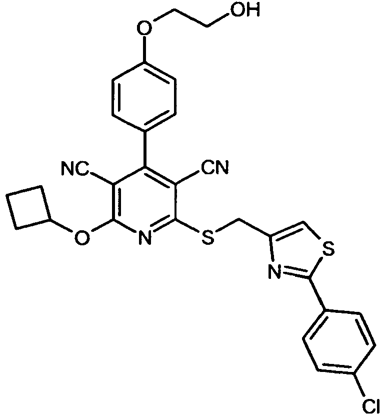 | 3.17 min (Method e 2); m/z = 575 | δ (400 MHz) = 7.98 (d, 2H), 7.74 (s, 1H), 7.61-7.53 (m, 4H), 7.14 (d, 2H), 5.37 (quintett, 1H), 4.79 (s, 2H), 4.09 (t, 2H), 3.73 (t, 2H), 2.44-2.32 (m, 2H), 2.23-2.10 (m, 2H), 1.85-1.75 (m, 1H), 1.68-1.53 (m, 1H). |
| | (30% d. Th.) | | |

### Beispiel 21

### Ameisensäure-4-[4-(2-Hydroxyethoxy)phenyl]-2-[(1H-imidazol-4-ylmethyl)sulfanyl]-6-methoxypyridin-3,5-dicarbonitril(1:1)

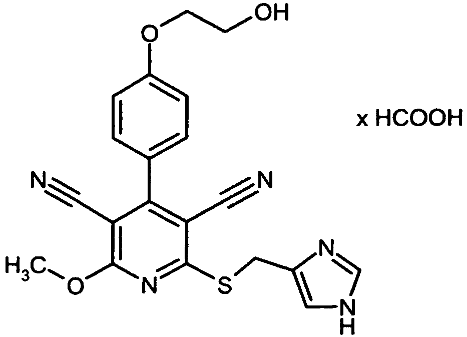

Die Verbindung wurde analog der Vorschrift für Beispiel 14 aus 2-Chlor-4-[4-(2-hydroxyethoxy)phenyl]-6-methoxypyridin-3,5-dicarbonitril und 4-Chlormethylimidazol hergestellt.

Ausb. 3.8 mg (7 % d. Th)

¹H-NMR (500 MHz, DMSO-d₆): δ = 8,12 (s, 1H) 7.61 (s, 1H), 7.54 (d, 2H), 7.18-7.00 (m, 3H), 4.92 (t, 1H), 4.58 (s, 2H), 4.18 (s, 3H), 4,20 (s, 3H) 4.10 (t, 2H), 3.73 (q, 2H).

LC-MS (Methode 11): Rₜ = 2.24 min; MS (ESIpos): m/z = 408 [M+H]⁺.

### Beispiel 22

### 2-({[2-(4-Chlorphenyl)-1,3-thiazol-4-yl]methyl}sulfanyl)-4-(4-{[(2S)-2,3-dihydroxypropyl]oxy}phenyl)-6-methoxypyridin-3,5-dicarbonitril

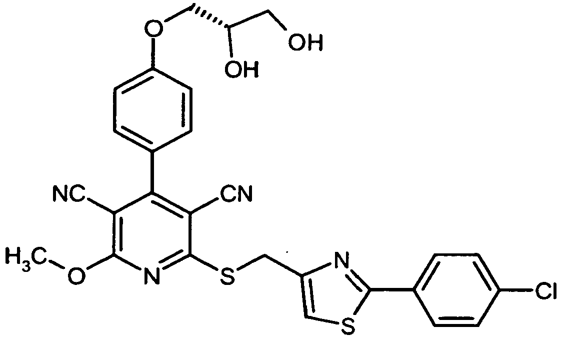

Die Lösung aus Beispiel 29A wurde gemeinsam mit 75 mg (0.306 mmol) 4-(Chlormethyl)-2-(4-chlorphenyl)-1,3-thiazol und 93 mg (1.112 mmol) Natriumhydrogencarbonat über Nacht bei Raumtemperatur gerührt. Die Reaktionsmischung wurde mittels präparativer HPLC (Acetonitril/Wasser: 10:90 → 95:5, Zusatz von 0.1 % TFA) gereinigt. Es wurden 33 mg (20% d. Th. über zwei Stufen) der Zielverbindung erhalten.

¹H-NMR (400 MHz, DMSO-d₆): δ = 7.95 (d, 2H), 7.76 (s, 1H), 7.57 (dd, 4H), 7.14 (d, 2H), 5.05 (d, 1H), 4.82 (s, 2H), 4.70 (t, 1H), 4.18 (s, 3H), 4.12-4.08 (m, 1H), 4.00-3.95 (m, 1H), 3.85-3.79 (m, 1H), 3.46 (t, 2H).

LC-MS (Methode 4): Rₜ = 1.41 min; MS (ESIpos): m/z = 565 [M+H]⁺.

### Beispiel 23

### 2-{[2-(4-Chlorphenyl)-1,3-oxazol-4-yl]methoxy}-4-[4-(2-hydroxyethoxy)phenyl]-6-methoxypyridin-3,5-dicarbonitril

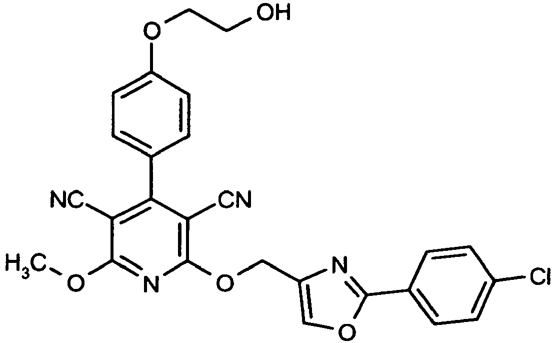

190 mg (0.910 mmol) [2-(4-Chlorphenyl)-1,3-oxazol-4-yl]methanol wurden in 2 ml DMF vorgelegt, mit 37 mg (0,334 mmol) Kalium-2-methylpropan-2-olat versetzt und 20 min bei Raumtemperatur gerührt. Anschließend wurden 100 mg (0.303 mmol) 2-Chlor-4-[4-(2-hydroxyethoxy)phenyl]-6-methoxypyridin-3,5-dicarbonitril, in 1 ml DMF gelöst, zugegeben. Nachdem über Nacht bei Raumtemperatur gerührt wurde, wurde das Produkt mittels präparativer HPLC (Acetonitril/Wasser: 10:90 → 95:5, Zusatz von 0.1 % TFA) isoliert. Man erhielt 31 mg (20 % d. Th.) der Zielverbindung.

¹H-NMR (400 MHz, DMSO-d₆): δ = 8.44 (s, 1H), 8.01 (d, 2H), 7.63 (d, 2H), 7.54 (d, 2H), 7.15 (d, 2H), 5.62 (s, 2H), 4.19 (s, 3H), 4.09 (t, 2H), 3.75 (t, 2H).

LC-MS (Methode 3): Rₜ = 2.23 min; MS (ESIpos): m/z = 503 [M+H]⁺.

### B. Bewertung der pharmakologischen und physiologischen Wirksamkeit

Die pharmakologische und physiologische Wirkung der erfindungsgemäßen Verbindungen kann in folgenden Assays gezeigt werden:

### B-1. Indirekte Bestimmung des Adenosin-Agonismus über Genexpression

Zellen der permanenten Linie CHO (Chinese Hamster Ovary) werden stabil mit der cDNA für die Adenosin-Rezeptor-Subtypen A1, A2a und A2b transfiziert. Die Adenosin-A1-Rezeptoren sind über Gᵢ-Proteine und die Adenosin-A2a- und A2b-Rezeptoren über Gₛ-Proteine an die Adenylatcyclase gekoppelt. Entsprechend wird die cAMP-Bildung in der Zelle inhibiert bzw. stimuliert. Über einen cAMP-abhängigen Promotor wird danach die Expression der Luziferase moduliert. Der Luziferase-Test wird mit dem Ziel hoher Sensitivität und Reproduzierbarkeit, geringer Varianz und guter Eignung für die Durchführung auf einem Robotersystem optimiert durch Variation mehrerer Testparameter, wie z.B. Zelldichte, Dauer der Anzuchtphase und der Testinkubation, Forskolin-Konzentration und Medium-Zusammensetzung. Zur pharmakologischen Charakterisierung der Zellen und zum Roboter-gestützten Substanz-Screening wird das folgende Testprotokoll verwendet:
Die Stammkulturen werden in DMEM/F12-Medium mit 10% FCS (fötales Kälberserum) bei 37°C unter 5% CO₂ gezüchtet und jeweils nach 2-3 Tagen 1:10 gesplittet. Testkulturen werden mit 2000 Zellen pro Napf in 384-well-Platten ausgesät und ca. 48 Stunden bei 37°C angezogen. Dann wird das Medium durch eine physiologische Kochsalzlösung (130 mM Natriumchlorid, 5 mM Kaliumchlorid, 2 mM Calciumchlorid, 20 mM HEPES, 1 mM Magnesiumchlorid-Hexahydrat, 5 mM Natriumhydrogencarbonat, pH 7.4) ersetzt. Die in DMSO gelösten zu testenden Substanzen werden in einer Verdünnungsreihe von 5 x 10⁻¹¹ M bis 3 x 10⁻⁶ M (Endkonzentration) zu den Testkulturen pipettiert (maximale DMSO-Endkonzentration im Testansatz: 0.5%). 10 Minuten später wird Forskolin zu den A1-Zellen zugegeben und anschließend werden alle Kulturen für vier Stunden bei 37°C inkubiert. Danach wird zu den Testkulturen 35 µl einer Lösung, bestehend zu 50% aus Lyse-Reagenz (30 mM Dinatriumhydrogenphosphat, 10% Glycerin, 3% TritonX100, 25 mM TrisHCl, 2 mM Dithiotreitol (DTT), pH 7.8) und zu 50% aus Luciferase-Substrat-Lösung (2.5 mM ATP, 0.5 mM Luciferin, 0.1 mM Coenzym A, 10 mM Tricin, 1.35 mM Magnesiumsulfat, 15 mM DTT, pH 7.8) zugegeben, ca. 1 Minute geschüttelt und die Luciferase-Aktivität mit einem Kamerasystem gemessen. Bestimmt werden die EC₅₀-Werte, d.h. die Konzentrationen, bei denen bei der A1-Zelle 50% der Luciferase-Antwort inhibiert bzw. bei den A2b- und A2a-Zellen 50% der maximalen Stimulierbarkeit mit der entsprechenden Substanz erreicht sind. Als Referenzverbindung dient in diesen Experimenten die Adenosin-analoge Verbindung NECA (5-N-Ethylcarboxamido-adenosin), die mit hoher Affinität an alle Adenosin-Rezeptor-Subtypen bindet und eine agonistische Wirkung besitzt [Klotz, K.N., Hessling, J., Hegler, J., Owman, C., Kull, B., Fredholm, B.B., Lohse, M.J., "Comparative pharmacology of human adenosine receptor subtypes - characterization of stably transfected receptors in CHO cells", Naunyn Schmiedebergs Arch. Pharmacol. 357, 1-9 (1998)].

In der folgenden Tabelle 1 sind die EC₅₀-Werte repräsentativer Ausführungsbeispiele für die Rezeptorstimulation an Adenosin A1-, A2a- und A2b-Rezeptor-Subtypen aufgeführt:

**Tabelle 6**

| **Beispiel Nr.** | **EC50 A1 [nM] (1 µM Forskolin)** | **EC50 A2a [nM]** | **EC50 A2b [nM]** |
|---|---|---|---|
| **1** | 1.3 | 3000 | 3000 |
| **2** | 0.7 | 3000 | 534 |
| **3** | 0.6 | 689 | 263 |
| **6** | 5.7 | 3000 | 3000 |
| **8** | 0.3 | 1220 | 105 |
| **11** | 0.8 | 1110 | 3000 |
| **12** | 1.2 | 547 | 3000 |
| **15** | 4.8 | 3000 | 3000 |
| **16** | 0.8 | 527 | 3000 |
| **18** | 0.3 | 1740 | 3000 |
| **19** | 0.5 | 685 | 177 |
| **21** | 73.2 | 3000 | 28.6 |
| **22** | 2.1 | 1260 | 142 |

### B-2. Untersuchung an isolierten Gefäßen

Aus narkotisierten Ratten wird die Arteria caudalis präpariert und in eine konventionelle Apparatur zur Messung isolierter Gefäße eingespannt. Die Gefäße werden in einem Wärmebad perfundiert und mit Phenylephrin kontrahiert. Das Maß der Kontraktion wird über einen Kontraktionsmesser ermittelt. Zu den vorkontrahierten Gefäßen werden Testsubstanzen gegeben und die Abnahme der Kontraktion der Gefäße gemessen. Eine Abnahme der Kontraktion entspricht einer Dilatation der Gefäße. Als EC₅₀-Wert einer Testsubstanz bzgl. ihrer relaxierenden Eigenschaften wird die Konzentration angegeben, bei der die Kontraktion der Gefäße um 50% verringert ist.

### B-3. Blutdruck- und Herzfrequenz-Messungen an wachen Ratten

Wachen SHR (spontaneously hypertensive rats)-Ratten, die einen internen Sender tragen, der dauerhaft sowohl Blutdruck als auch Herzfrequenz messen kann (telemetrische Erfassung von hämodynamischen Parametern), werden Testsubstanzen in verschiedenen Dosierungen oral verabreicht. Anschließend werden über 24 Stunden Blutdruck und Herzfrequenz und deren Veränderungen aufgezeichnet.

### B-4. Blutdruck- und Herzfrequenz-Messungen an wachen Krallenaffen

Wachen Krallenaffen, die einen internen Sender tragen, der dauerhaft sowohl Blutdruck als auch Herzfrequenz messen kann (telemetrische Erfassung von hämodynamischen Parametern), werden Testsubstanzen in verschiedenen Konzentrationen oral verabreicht. Anschließend werden über 6-24 Stunden Blutdruck und Herzfrequenz und deren Veränderungen aufgezeichnet.

### B-5. Indirekte Bestimmung des Adenosin-Antagonismus über Genexpression

Zellen der permanenten Linie CHO K1 (Chinese Hamster Ovary) werden stabil mit einem Reporterkonstrukt (CRE-Luciferase) und der cDNA für die Adenosin-Rezeptorsubtypen A2a oder A2b transfiziert. A2a- bzw. A2b-Rezeptoren sind über Gas-Proteine an die Adenylatcyclase gekoppelt. Durch Rezeptoraktivierung wird die Adenylatcyclase aktiviert und damit das cAMP-Level in der Zelle erhöht. Über das Reporterkonstrukt, einem cAMP-abhängigen Promotor, ist die Änderung des cAMP-Levels an die Luciferase-Expression gekoppelt.

Für die Bestimmung von Adenosin-Antagonismus am Adenosin-Rezeptorsubtyp A1 werden ebenfalls CHO K1-Zellen stabil transfiziert, diesmal allerdings mit einem Ca²⁺-sensitiven Reporterkonstrukt (NFAT-TA-Luc; Clontech) und einem A1-Gα16 Fusionskonstrukt. Diese Rezeptorchimäre ist im Gegensatz zum nativen A1-Rezeptor (Gai-Kopplung) an die Phospholipase C gekoppelt. Die Luciferase wird hier in Abhängigkeit von der cytosolischen Ca²⁺-Konzentration exprimiert.

Die permanenten Zelllinien werden in DMEM/F12 (Cat.-No BE04-687Q; BioWhittaker) mit 10% FCS (Fetales Kälberserum) und diversen Zusätzen (20 ml/Liter 1M HEPES (Cat.-No 15630; Gibco), 20 ml/Liter GlutaMAX (Cat.-No 35050-038, Gibco), 14 ml/Liter MEM Natriumpyruvat (Cat.-No 11360-039; Gibco), 10 ml/Liter PenStrep (Cat.-No 15070-063; Gibco)) bei 37°C unter 5% Kohlendioxid kultiviert und zweimal pro Woche gesplittet.

Für die Testung im 384-well Plattenformat werden die Zellen mit 2000 Zellen/Well in 25µl/Well Aussaatmedium ausgesät und bis zur Substanztestung bei 37°C unter 5% Kohlendioxid kultiviert. Die A2a- und A2b-Zellen werden 24 h vor der Substanztestung in Medium mit Zusätzen und 5% FCS ausgesät, wobei für die A2a-Zellen als Basismedium DMEM/F12 und für die A2b-Zellen OptiMEM (Cat.-No 31985-047; Gibco) verwendet wird. Die A1-Gα16-Zellen werden 48 h vor Substanztestung in OptiMEM mit 2.5% dialysiertem FCS und Zusätzen ausgesät. Am Testtag wird vor der Substanzzugabe das Medium durch 25 µl Cafty-Puffer (Cat.-No T21-154; PAA) mit 2 mM Calciumchlorid und 0.1% BSA (Rinderserumalbumin) ersetzt. Von den zu testenden, in DMSO gelösten Substanzen werden Verdünnungsreihen in Cafty-Puffer mit 2 mM Calciumchlorid und 0.1% BSA (Rinderserumalbumin) und einer geeigneten Konzentration des Agonisten angesetzt. Die Substanzen werden in einer Endkonzentration von 5 x 10⁻⁵ M bis 2.56 x 10⁻¹¹ M zu den Testkulturen pipettiert, wobei der DMSO-Gehalt auf den Zellen 0.5% nicht überschreiten darf. Als Agonist wird für die A2a- und A2b-Zellen NECA (5-N-Ethylcarboxamido-adenosin) in einer Endkonzentration von 30 nM, was etwa der EC₅₀-Konzentration entspricht, eingesetzt. Für die A1-Gα16-Zellen wird 25 nM CPA (N6-cyclopentyl-adenosine) als Agonist eingesetzt, was etwa der EC₇₅-Konzentration entspricht. Nach der Substanzzugabe werden die Zellplatten 3-4 h bei 37°C unter 5% Kohlendioxid inkubiert. Anschließend werden die Zellen direkt vor der Messung mit 25 µl einer Lösung, bestehend zu 50% aus Lyse-Reagenz (30 mM Dinatriumhydrogenphosphat, 10% Glycerin, 3% Triton X-100, 25 mM TrisHCl, 2 mM Dithiotreitol (DTT), pH 7.8) und zu 50% aus Luciferase-Substrat-Lösung (2.5 mM ATP, 0.5 mM Luciferin, 0.1 mM Coenzym A, 10 mM Tricin, 1.35 mM Magnesiumsulfat, 15 mM DTT, pH 7.8) versetzt. Die Luciferase-Aktivität wird mit einem Lumineszenz-Reader detektiert. Bestimmt werden die IC₅₀-Werte, d.h. die Konzentration, bei denen die Luciferase-Antwort, hervorgerufen durch den jeweiligen Agonisten zu 50% inhibiert wird. Als Referenz-Antagonist wird für die A2a- und A2b-Zellen ZM241385 und für die A1-Gα16-Zellen DPCPX (1,3-dipropyl-8-cyclopentylxanthine) verwendet.

### B-6. Bestimmung pharmakokinetischer Kenngrößen nach intravenöser und oraler Gabe

Die zu untersuchende Substanz wird Tieren (z.B. Maus, Ratte, Hund) intravenös als Lösung appliziert, die orale Applikation erfolgt als Lösung oder Suspension über eine Schlundsonde. Nach Substanzgabe wird den Tieren zu festgelegten Zeitpunkten Blut entnommen. Dieses wird heparinisiert, anschließend wird daraus durch Zentrifugation Plasma gewonnen. Die Substanz wird im Plasma über LC/MS-MS analytisch quantifiziert. Aus den so ermittelten Plasmakonzentration-Zeit-Verläufen werden die pharmakokinetischen Kenngrößen wie AUC (Fläche unter der Konzentration-Zeit-Kurve), Cₘₐₓ (maximale Plasmakonzentration), T_{1/2} (Halbwertszeit) und CL (Clearance) mittels eines validierten pharmakokinetischen Rechenprogramms berechnet.

### B-7. Bestimmung der Löslichkeit

### Benötigte Reagentien:

● PBS-Puffer pH 6.5: 90.00 g NaCl p.a. (z.B. Fa. Merck, Art.-Nr. 1.06404.1000), 13.61 g KH₂PO₄ p.a. (z.B. Fa. Merck, Art.-Nr. 1.04873.1000) und 83.35 g 1 N Natronlauge (z.B. Fa. Bernd Kraft GmbH, Art.-Nr. 01030.4000) werden in einen 1 Liter-Messkolben eingewogen, mit destilliertem Wasser auf 1 Liter aufgefüllt und für 1 Stunde gerührt. Danach wird mit 1 N Salzsäure (z.B. Fa. Merck, Art.-Nr. 1.09057.1000) der pH-Wert auf 6.5 eingestellt.
● PEG/Wasser-Lösung (70:30 v/v): 70 ml Polyethylenglykol 400 (z.B. Fa. Merck, Art.-Nr. 8.17003.1000) und 30 ml destilliertes Wasser werden in einem 100 ml-Messkolben homogenisiert.
● PEG/PBS-Puffer pH 6.5 (20:80 v/v): 20 ml Polyethylenglykol 400 (z.B. Fa. Merck, Art.-Nr. 8.17003.1000) und 80 ml PBS-Puffer pH 6.5 werden in einem 100 ml-Messkolben homogenisiert.
● Dimethylsulfoxid (z.B. Fa. Baker, Art.-Nr. 7157.2500)
● destilliertes Wasser.

### Herstellung der Ausgangslösung (Urlösung):

Mindestens 4 mg der Testsubstanz werden in ein Weithals-10 mm Screw V-Vial (Fa. Glastechnik Gräfenroda GmbH, Art.-Nr. 8004-WM-H/V15µ) mit passender Schraubkappe und Septum genau eingewogen, in einem Pipettierroboter mit DMSO bis zu einer Konzentration von 50 mg/ml versetzt und 10 Minuten geschüttelt.

### Herstellung der Kalibrierlösungen:

*Herstellung der Ausgangslösung für Kalibrierlösungen (Stammlösung):* In eine Mikrotiterplatte werden 10 µl der Urlösung mit Hilfe eines Pipettierroboters überführt und mit DMSO bis zu einer Konzentration von 600 µg/ml aufgefüllt. Die Probe wird bis zu ihrer vollständigen Lösung geschüttelt.

*Kalibrierlösung 1 (20 µg*/*ml):* 34.4 µl der Stammlösung werden mit 1000 µl DMSO versetzt und homogenisiert.

*Kalibrierlösung 2 (2.5 µg*/*ml):* 100 µl der Kalibrierlösung 1 werden mit 700 µl DMSO versetzt und homogenisiert.

### Herstellung der Probenlösungen:

*Probenlösung für Löslichkeit bis 5 g*/*Liter in PBS-Puffer pH 6.5:* In eine Mikrotiterplatte werden 10 µl Urlösung transferiert und mit 1000 µl PBS-Puffer pH 6.5 versetzt.

*Probenlösung für Löslichkeit bis 5 g*/*Liter in PEG*/*Wasser (70:30):* In eine Mikrotiterplatte werden 10 µl Urlösung transferiert und mit 1000 µl PEG/Wasser (70:30) versetzt.

*Probenlösung für Löslichkeit bis 5 g*/*Liter in PEG*/*PBS-Puffer pH 6.5 (20:80):* In eine Mikrotiterplatte werden 10 µl Urlösung transferiert und mit 1000 µl PEG/PBS-Puffer pH 6.5 (20:80) versetzt.

### Durchführung:

Die so hergestellten Probenlösungen werden 24 Stunden bei 1400 rpm mittels eines temperierbaren Schüttlers (z.B. Fa. Eppendorf Thermomixer comfort Art.-Nr. 5355 000.011 mit Wechselblock Art.-Nr. 5362.000.019) bei 20°C geschüttelt. Von diesen Lösungen werden jeweils 180 µl abgenommen und in Beckman Polyallomer Centrifuge Tubes (Art.-Nr. 343621) überführt. Diese Lösungen werden 1 Stunde mit ca. 223.000 x g zentrifugiert (z.B. Fa. Beckman Optima L-90K Ultracentrifuge mit Type 42.2 Ti Rotor bei 42.000 rpm). Von jeder Probenlösung werden 100 µl des Überstandes abgenommen und mit DMSO zu 1:5 und 1:100 verdünnt. Es wird von jeder Verdünnung eine Abfüllung in ein geeignetes Gefäß für die HPLC-Analytik vorgenommen.

### Analytik:

Die Proben werden mittels RP-HPLC analysiert. Quantifiziert wird über eine Zwei-Punkt-Kalibrationskurve der Testverbindung in DMSO. Die Löslichkeit wird in mg/Liter ausgedrückt. Analysensequenz: 1) Kalibrierlösung 2.5 mg/ml; 2) Kalibrierlösung 20 µg/ml; 3) Probenlösung 1:5; 4) Probenlösung 1:100.

### HPLC-Methode für Säuren:

Agilent 1100 mit DAD (G1315A), quat. Pumpe (G1311A), Autosampler CTC HTS PAL, Degaser (G1322A) und Säulenthermostat (G1316A); Säule: Phenomenex Gemini C 18, 50 mm x 2 mm, 5 µ; Temperatur: 40°C; Eluent A: Wasser/Phosphorsäure pH 2; Eluent B: Acetonitril; Flussrate: 0.7 ml/min; Gradient: 0-0.5 min 85% A, 15% B; Rampe: 0.5-3 min 10% A, 90% B; 3-3.5 min 10% A, 90% B; Rampe: 3.5-4 min 85% A, 15% B; 4-5 min 85% A, 15% B.

### HPLC-Methode für Basen:

Agilent 1100 mit DAD (G1315A), quat. Pumpe (G1311A), Autosampler CTC HTS PAL, Degaser (G1322A) und Säulenthermostat (G1316A); Säule: VDSoptilab Kromasil 100 C18, 60 mm x 2.1 mm, 3.5 µ; Temperatur: 30°C; Eluent A: Wasser + 5 ml Perchlorsäure/Liter; Eluent B: Acetonitril; Flussrate: 0.75 ml/min; Gradient: 0-0.5 min 98% A, 2% B; Rampe: 0.5-4.5 min 10% A, 90% B; 4.5-6 min 10% A, 90% B; Rampe: 6.5-6.7 min 98% A, 2% B; 6.7-7.5 min 98% A, 2% B.

### B-8. Bestimmung der metabolischen Stabilität

Zur Bestimmung der metabolischen Stabilität von Testverbindungen werden diese *in vitro* mit Lebermikrosomen oder bevorzugt mit primären frischen Hepatozyten verschiedener Tierspezies (z.B. von Ratte und Hund) als auch humanen Ursprungs inkubiert, um Metabolitenprofile eines möglichst kompletten hepatischen Phase I- und Phase II-Metabolismus zu erhalten und zu vergleichen.

Die Testverbindungen werden mit einer Konzentration von 10-20 µM inkubiert. Dazu werden Stammlösungen der Substanzen mit einer Konzentration von 1-2 mM in Acetonitril hergestellt und dann mit einer 1:100-Verdünnung in den Inkubationsansatz pipettiert. Die Lebermikrosomen werden in 50 mM Kaliumphosphat-Puffer (pH 7.4) mit und ohne NADPH-generierendem System, bestehend aus 1 mM NADP⁺, 10 mM Glucose-6-phosphat und 1 Einheit Glucose-6-phosphat-Dehydrogenase, bei 37°C inkubiert. Primäre Hepatozyten werden in Suspension in Williams E-Medium ebenfalls bei 37°C inkubiert. Nach einer Inkubationszeit von 0-4 Stunden werden die Inkubationsansätze mit Acetonitril abgestoppt (Endkonzentration ca. 30%) und das Protein bei ca. 15000 x g abzentrifugiert. Die so abgestoppten Proben werden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung chromatographiert. Die UV-Chromatogramme in Verbindung mit massenspektrometrischen MS/MS-Daten dienen zur Identifizierung und Strukturaufklärung der Metabolite.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 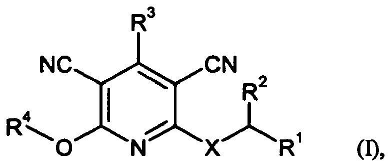 in welcher
X für O oder S steht,
R¹ für (C₆-C₁₀)-Aryl oder 5- bis 10-gliedriges Heteroaryl steht,
wobei (C₆-C₁₀)-Aryl und 5- bis 10-gliedriges Heteroaryl mit 1 oder 2 Substituenten unabhängig voneinander ausgewählt aus der Gruppe Halogen, Nitro, Cyano, (C₁-C₆)-Alkyl, Trifluormethyl, Hydroxy, (C₁-C₆)-Alkoxy, Amino, Mono-(C₁-C₆)-alkylamino, Di-(C₁-C₆)-alkylamino, Hydroxycarbonyl, (C₁-C₆)-Alkoxycarbonyl, Aminocarbonyl, Mono-(C₁-C₆)-alkylaminocarbonyl, Di-(C₁-C₆)alkylaminocarbonyl, (C₃-C₇)-Cycloalkylaminocarbonyl, Aminosulfonyl, Mono-(C₁-C₆)-alkylaminosulfonyl, Di-(C₁-C₆)-alkylaminosulfonyl, (C₁-C₆)-Alkylsulfonylamino, Pyrrolidino, Piperidino, Morpholino, Piperazino, *N*'-(C₁-C₄)-Alkylpiperazino, Pyrrolidinocarbonyl, Piperidinocarbonyl, Morpholinocarbonyl, Piperazinocarbonyl, *N*'-(C₁-C₄)-Alkylpiperazinocarbonyl und -L-R⁵ substituiert sein können,
worin
L für eine Bindung, NH oder O steht,
R⁵ für Phenyl oder 5- oder 6-gliedriges Heteroaryl steht,
worin Phenyl und 5- oder 6-gliedriges Heteroaryl ihrerseits mit 1 bis 3 Substituenten unabhängig voneinander ausgewählt aus der Gruppe Halogen, Nitro, Cyano, (C₁-C₆)-Alkyl, Trifluormethyl, Hydroxy, (C₁-C₆)-Alkoxy, Difluormethoxy, Trifluormethoxy, Amino, Mono-(C₁-C₆)-alkylamino, Di-(C₁-C₆)-alkylamino, Hydroxycarbonyl und (C₁-C₆)-Alkoxycarbonyl sein können,
R² für Wasserstoff oder (C₁-C₄)-Alkyl steht,
R³ für Phenyl oder 5- oder 6-gliedriges Heteroaryl steht,
wobei Phenyl und 5- oder 6-gliedriges Heteroaryl mit 1 bis 3 Substituenten unabhängig voneinander ausgewählt aus der Gruppe Halogen, Cyano, Hydroxy, (C₁-C₆)-Alkyl, (C₁-C₆)-Alkoxy, (C₃-C₇)-Cycloalkoxy, Tetrahydrofuranyloxy, Pyrrolidinyloxy und -NR^{A}R^{B} substituiert sein können,
worin (C₁-C₆)-Alkyl und (C₁-C₆)-Alkoxy mit 1 bis 3 Substituenten unabhängig voneinander ausgewählt aus der Gruppe Fluor, Trifluormethyl, (C₃-C₇)-Cycloalkyl, Hydroxy, (C₁-C₄)-Alkoxy, Hydroxycarbonyl, (C₁-C₄)-Alkoxycarbonyl, Amino, Aminocarbonyl, Mono-(C₁-C₄)-alkylamino und Di-(C₁-C₄)-alkylamino substituiert sein können,
und
worin (C₃-C₇)-Cycloalkoxy, Tetrahydrofuranyloxy und Pyrrolidinyloxy mit 1 oder 2 Substituenten unabhängig voneinander ausgewählt aus der Gruppe (C₁-C₄)-Alkyl, Hydroxy, Oxo und (C₁-C₄)-Alkoxy substituiert sein können,
und
worin
R^{A} für Wasserstoff oder (C₁-C₆)-Alkyl steht,
worin (C₁-C₆)-Alkyl seinerseits mit 1 bis 3 Substituenten Fluor substituiert sein kann,
und
worin (C₁-C₆)-Alkyl seinerseits mit einem Substituenten ausgewählt aus der Gruppe Hydroxy und (C₁-C₄)-Alkoxy substituiert sein kann,
R^{B} für Wasserstoff, (C₁-C₆)-Alkyl, (C₃-C₇)-Cycloalkyl, (C₁-C₆)-Alkylcarbonyl, (C₁-C₄)-Alkylsulfonyl oder (C₃-C₇)-Cycloalkylsulfonyl steht,
worin (C₁-C₆)-Alkyl seinerseits mit 1 oder 2 Substituenten unabhängig voneinander ausgewählt aus der Gruppe Fluor, Trifluormethyl, (C₃-C₇)-Cycloalkyl, Hydroxy, (C₁-C₄)-Alkoxy, Hydroxycarbonyl, (C₁-C₄)Alkoxycarbonyl, Amino, Mono-(C₁-C₄)-alkylamino und Di-(C₁-C₄)-alkylamino substituiert sein kann,
und
worin (C₃-C₇)-Cycloalkyl seinerseits mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
oder
R^{A} und R^{B} zusammen mit dem Stickstoffatom, an das sie gebunden sind, einen 4- bis 7-gliedrigen Heterocyclus bilden, der ein weiteres Ring-Heteroatom aus der Reihe N, O oder S enthalten und mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
oder
worin zwei benachbarte Substituenten am Phenyl zusammen mit den Kohlenstoffatomen, an die sie gebunden sind, ein 1,3-Dioxolan, 1,3-Dioxan oder 2,2-Difluor-1,3-dioxolan bilden können,
R⁴ für (C₁-C₆)-Alkyl, (C₃-C₇)-Cycloalkyl oder 4- bis 6-gliedriges Heterocyclyl steht,
wobei (C₁-C₆)-Alkyl mit 1 oder 2 Substituenten unabhängig voneinander ausgewählt aus der Gruppe Fluor, Trifluormethyl, (C₃-C₇)-Cycloalkyl, Hydroxy, (C₁-C₄)-Alkoxy, Hydroxycarbonyl, (C₁-C₄)-Alkoxycarbonyl, Amino, Mono-(C₁-C₄)-alkylamino, Di-(C₁-C₄)-alkylamino, Aminocarbonyl, Mono-(C₁-C₄)-alkylaminocarbonyl, Di-(C₁-C₄)-alkylaminocarbonyl und 5- oder 6-gliedriges Heterocyclyl substituiert sein kann,
worin (C₁-C₄)-Alkoxy mit 1 oder 2 Substituenten unabhängig voneinander ausgewählt aus der Gruppe Hydroxy und (C₁-C₄)-Alkoxy substituiert sein kann
und
worin 5- oder 6-gliedriges Heterocyclyl mit einem Substituenten ausgewählt aus der Gruppe Oxo und (C₁-C₄)-Alkyl substituiert sein kann
und
wobei (C₃-C₇)-Cycloalkyl und 4- bis 6-gliedriges Heterocyclyl mit 1 oder 2 Substituenten unabhängig voneinander ausgewählt aus der Gruppe (C₁-C₄)-Alkyl, Hydroxy, (C₁-C₄)-Alkoxy, Hydroxycarbonyl, (C₁-C₄)-Alkoxycarbonyl, Amino, Mono-(C₁-C₄)-alkylamino und Di-(C₁-C₄)-alkylamino substituiert sein können,
sowie ihre N-Oxide, Salze, Solvate, Salze der N-Oxide und Solvate der N-Oxide und Salze.

2. Verbindung der Formel (I) nach Anspruch 1, in welcher
X für S steht,
R¹ für Phenyl oder 5- oder 6-gliedriges Heteroaryl steht,
wobei Phenyl und 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Trifluormethyl, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, Aminocarbonyl, Mono-(C₁-C₄)-alkylaminocarbonyl, Di-(C₁-C₄)-alkylaminocarbonyl, (C₃-C₆)-Cycloalkylaminocarbonyl, (C₁-C₄)-Alkylsulfonylamino, Morpholino, Piperazino, *N*'-(C₁-C₄)-Alkylpiperazino und -L-R⁵ substituiert sein können,
worin
L für eine Bindung oder NH steht,
R⁵ für Phenyl oder 5- oder 6-gliedriges Heteroaryl steht,
worin Phenyl und 5- oder 6-gliedriges Heteroaryl ihrerseits mit 1 oder 2 Substituenten unabhängig voneinander ausgewählt aus der Gruppe Fluor, Chlor, Cyano, (C₁-C₄)-Alkyl, Trifluormethyl, (C₁-C₄)-Alkoxy, Trifluormethoxy, Amino, Hydroxycarbonyl und (C₁-C₄)-Alkoxycarbonyl sein können,
R² für Wasserstoff oder Methyl steht,
R³ für Phenyl, Pyrrolyl, Oxazolyl, Thiazolyl, Isoxazolyl, Pyrazolyl, Imidazolyl und Pyridyl steht,
wobei Phenyl, Pyrrolyl, Oxazolyl, Thiazolyl, Isoxazolyl, Pyrazolyl, Imidazolyl und Pyridyl mit 1 oder 2 Substituenten unabhängig voneinander ausgewählt aus der Gruppe Fluor, (C₁-C₆)-Alkyl, Hydroxy, (C₁-C₄)-Alkoxy und -NR^{A}R^{B} substituiert sein können,
worin (C₁-C₆)-Alkyl und (C₁-C₄)-Alkoxy mit 1 bis 3 Substituenten unabhängig voneinander ausgewählt aus der Gruppe Fluor, Trifluormethyl, Hydroxy, Methoxy, Ethoxy, Hydroxycarbonyl, Amino, Methylamino, Ethylamino, *N,N*-Dimethylamino und *N,N*-Diethylamino substituiert sein können,
und
worin
R^{A} für Wasserstoff oder (C₁-C₄)-Alkyl steht,
worin (C₁-C₄)-Alkyl seinerseits mit einem Substituenten ausgewählt aus der Gruppe Hydroxy und (C₁-C₄)-Alkoxy substituiert sein kann,
R^{B} für Wasserstoff oder (C₁-C₄)-Alkyl steht,
worin (C₁-C₄)-Alkyl seinerseits mit 1 oder 2 Substituenten unabhängig voneinander ausgewählt aus der Gruppe Hydroxy, (C₁-C₄)-Alkoxy und Hydroxycarbonyl substituiert sein kann,
R⁴ für (C₁-C₆)-Alkyl, (C₄-C₆)-Cycloalkyl oder 5- oder 6-gliedriges Heterocyclyl steht,
wobei (C₁-C₆)-Alkyl mit 1 oder 2 Substituenten unabhängig voneinander ausgewählt aus der Gruppe Fluor, Trifluormethyl, (C₃-C₇)-Cycloalkyl, Hydroxy, Methoxy, Ethoxy, Hydroxycarbonyl, Methoxycarbonyl, Ethoxycarbonyl, Amino, Methylamino, Ethylamino, *N,N*-Dimethylamino, *N,N*-Diethylamino und 5- oder 6-gliedriges Heterocyclyl substituiert sein kann,
worin 5- oder 6-gliedriges Heterocyclyl seinerseits mit einem Substituenten ausgewählt aus der Gruppe Oxo und Methyl substituiert sein kann,
und
wobei (C₃-C₇)-Cycloalkyl und 5- oder 6-gliedriges Heterocyclyl mit 1 oder 2 Substituenten unabhängig voneinander ausgewählt aus der Gruppe Methyl, Hydroxy, Methoxy, Hydroxycarbonyl, Methoxycarbonyl, Ethoxycarbonyl, Amino, Methylamino und *N*,*N*-Dimethylamino substituiert sein können,
sowie ihre Salze, Solvate und Solvate der Salze.

3. Verbindung der Formel (I) nach Anspruch 1, in welcher
X für O oder S steht,
R¹ für Phenyl, Thiazolyl, Oxazolyl oder Pyridyl steht,
wobei Phenyl und Pyridyl mit 1 oder 2 Substituenten unabhängig voneinander ausgewählt aus der Gruppe Fluor, Chlor, Cyano, Methyl, Trifluormethyl, Methoxy, Hydroxycarbonyl, Methoxycarbonyl, Ethoxycarbonyl, Aminocarbonyl, Methylaminocarbonyl, Ethylaminocarbonyl, *N*,*N*-Dimethylaminocarbonyl und *N*,*N*-Diethylaminocarbonyl substituiert sind,
und
wobei Thiazolyl und Oxazolyl mit einem Substituenten -L-R⁵ substituiert sind,
worin
L für eine Bindung oder NH steht,
R⁵ für Phenyl steht,
worin Phenyl seinerseits mit 1 oder 2 Substituenten unabhängig voneinander ausgewählt aus der Gruppe Fluor, Chlor, Methyl, Methoxy, Ethoxy, Hydroxycarbonyl, Methoxycarbonyl und Ethoxycarbonyl sein kann,
und
wobei Thiazolyl und Oxazolyl mit einem Substituenten ausgewählt aus der Gruppe Fluor, Methyl, Ethyl, Methoxy, Hydroxycarbonyl und Methoxycarbonyl substituiert sein können,
R² für Wasserstoff oder Methyl steht,
R³ für Phenyl, Pyrrolyl, Oxazolyl, Thiazolyl, Isoxazolyl, Pyrazolyl, Imidazolyl und Pyridyl steht,
wobei Phenyl, Pyrrolyl, Oxazolyl, Thiazolyl, Isoxazolyl, Pyrazolyl, Imidazolyl und Pyridyl mit 1 oder 2 Substituenten unabhängig voneinander ausgewählt aus der Gruppe Fluor, (C₁-C₆)-Alkyl, Hydroxy, (C₁-C₄)-Alkoxy und -NR^{A}R^{B} substituiert sein können,
worin (C₁-C₆)-Alkyl und (C₁-C₄)-Alkoxy mit 1 bis 3 Substituenten unabhängig voneinander ausgewählt aus der Gruppe Fluor, Trifluormethyl, Hydroxy, Methoxy, Ethoxy, Hydroxycarbonyl, Amino, Methylamino, Ethylamino, *N*,*N*-Dimethylamino und *N*,*N*-Diethylamino substituiert sein können,
und
worin
R^{A} für Wasserstoff oder (C₁-C₄)-Alkyl steht,
worin (C₁-C₄)-Alkyl seinerseits mit einem Substituenten ausgewählt aus der Gruppe Hydroxy und (C₁-C₄)-Alkoxy substituiert sein kann,
R^{B} für Wasserstoff oder (C₁-C₄)-Alkyl steht,
worin (C₁-C₄)-Alkyl seinerseits mit 1 oder 2 Substituenten unabhängig voneinander ausgewählt aus der Gruppe Hydroxy, (C₁-C₄)-Alkoxy und Hydroxycarbonyl substituiert sein kann,
R⁴ für (C₁-C₆)-Alkyl oder (C₄-C₆)-Cycloalkyl steht,
wobei (C₁-C₆)-Alkyl mit 1 oder 2 Substituenten unabhängig voneinander ausgewählt aus der Gruppe Fluor, Trifluormethyl, (C₃-C₇)-Cycloalkyl, Hydroxy, Methoxy und Ethoxy substituiert sein kann,
und
wobei (C₄-C₆)-Cycloalkyl mit 1 oder 2 Substituenten unabhängig voneinander ausgewählt aus der Gruppe Methyl, Hydroxy und Methoxy substituiert sein kann,
sowie ihre Salze, Solvate und Solvate der Salze.

4. Verbindung der Formel (I) nach Anspruch 1, 2 oder 3, in welcher
X für S steht,
R¹ für Phenyl, Thiazolyl, Oxazolyl oder Pyridyl steht,
wobei Phenyl und Pyridyl mit 1 oder 2 Substituenten unabhängig voneinander ausgewählt aus der Gruppe Fluor, Chlor, Cyano, Methyl, Trifluormethyl, Methoxy, Hydroxycarbonyl, Methoxycarbonyl, Ethoxycarbonyl, Aminocarbonyl, Methylaminocarbonyl, Ethylaminocarbonyl, N,N-Dimethylaminocarbonyl und *N*,*N*-Diethylaminocarbonyl substituiert sind,
und
wobei Thiazolyl und Oxazolyl mit einem Substituenten -L-R⁵ substituiert sind,
worin
L für eine Bindung oder NH steht,
R⁵ für Phenyl steht,
worin Phenyl seinerseits mit 1 oder 2 Substituenten unabhängig voneinander ausgewählt aus der Gruppe Fluor, Chlor, Methyl, Methoxy, Ethoxy, Hydroxycarbonyl, Methoxycarbonyl und Ethoxycarbonyl sein kann,
und
wobei Thiazolyl und Oxazolyl mit einem Substituenten ausgewählt aus der Gruppe Fluor, Methyl, Ethyl, Methoxy, Hydroxycarbonyl und Methoxycarbonyl substituiert sein können,
R² für Wasserstoff steht,
R³ für Phenyl oder Thiazolyl steht,
wobei Phenyl mit 1 oder 2 Substituenten unabhängig voneinander ausgewählt aus der Gruppe (C₁-C₆)-Alkyl, Hydroxy und (C₁-C₄)-Alkoxy substituiert sein kann,
worin (C₁-C₆)-Alkyl und (C₂-C₄)-Alkoxy mit 1 oder 2 Substituenten unabhängig voneinander ausgewählt aus der Gruppe Hydroxy und Methoxy substituiert sein können,
und
wobei Thiazolyl mit einem Substituenten (C₁-C₆)-Alkyl substituiert sein kann,
worin (C₁-C₆)-Alkyl mit 1 oder 2 Substituenten unabhängig voneinander ausgewählt aus der Gruppe Hydroxy und Methoxy substituiert sein kann,
R⁴ für (C₁-C₄)-Alkyl steht,
wobei Alkyl mit 1 oder 2 Substituenten Hydroxy substituiert sein kann,
sowie ihre Salze, Solvate und Solvate der Salze.

5. Verfahren zur Herstellung von Verbindungen der Formel (I), wie in den Ansprüchen 1 bis 4 definiert, **dadurch gekennzeichnet, dass** man
[A] eine Verbindung der Formel (II-A) 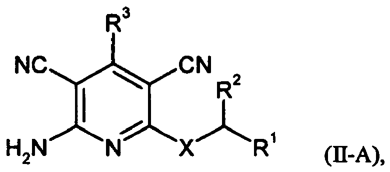 in welcher X, R¹, R² und R³ jeweils die in den Ansprüchen 1 bis 4 angegebenen Bedeutungen haben,
zunächst mit Kupfer(II)chlorid und Isoamylnitrit in einem geeigneten Lösungsmittel in eine Verbindung der Formel (III-A) 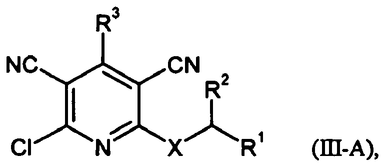 in welcher X, R¹, R² und R³ jeweils die in den Ansprüchen 1 bis 4 angegebenen Bedeutungen haben,
überführt, und diese anschliessend in einem inerten Lösungsmittel in Gegenwart einer geeigneten Base mit einer Verbindung der Formel (IV)
R⁴-OH (IV),
in welcher R⁴ die die in den Ansprüchen 1 bis 4 angegebene Bedeutung hat, umsetzt,
oder
[B] im Fall, dass X für S steht, eine Verbindung der Formel (II-B) 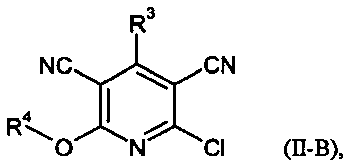 in welcher R³ und R⁴ jeweils die die in den Ansprüchen 1 bis 4 angegebenen Bedeutungen haben,
in einem inerten Lösungsmittel mit einem Alkalisulfid zu einer Verbindung der Formel (III-B) 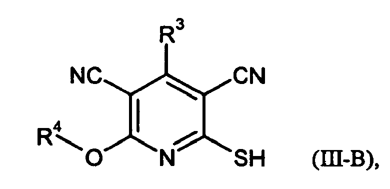 in welcher R³ und R⁴ jeweils die die in den Ansprüchen 1 bis 4 angegebenen Bedeutungen haben,
umsetzt, und diese anschliessend in einem inerten Lösungsmittel in Gegenwart einer Base mit einer Verbindung der Formel (V) 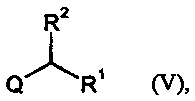 in welcher R¹ und R² die die in den Ansprüchen 1 bis 4 angegebenen Bedeutungen haben und
Q für eine geeignete Abgangsgruppe, vorzugsweise für Halogen, insbesondere Chlor, Brom oder Iod, oder für Mesylat, Tosylat oder Triflat steht,
umsetzt,
anschließend gegebenenfalls vorhandene Schutzgruppen abspaltet und die resultierenden Verbindungen der Formel (I) gegebenenfalls mit den entsprechenden (*i*) Lösungsmitteln und/oder (*ii*) Basen oder Säuren in ihre Solvate, Salze und/oder Solvate der Salze überführt.

6. Verbindung der Formel (I), wie in einem der Ansprüche 1 bis 4 definiert, zur Behandlung und/oder Prävention von Krankheiten.

7. Verbindung der Formel (I), wie in einem der Ansprüche 1 bis 4 definiert, zur Verwendung in einem Verfahren zur Behandlung und/oder Prophylaxe von Hypertonie, koronarer Herzerkrankung, akutem Koronarsyndrom, Angina pectoris, Herzinsuffizienz, Myokardinfarkt und Vorhofflimmern.

8. Verbindung der Formel (I), wie in einem der Ansprüche 1 bis 4 definiert, zur Verwendung in einem Verfahren zur Behandlung und/oder Prophylaxe von Diabetes, Metabolischem Syndrom und Dyslipidämien.

9. Verwendung einer Verbindung der Formel (I), wie in einem der Ansprüche 1 bis 4 definiert, zur Herstellung eines Arzneimittels zur Behandlung und/oder Prävention von Hypertonie, koronarer Herzerkrankung, akutem Koronarsyndrom, Angina pectoris, Herzinsuffizienz, Myokardinfarkt und Vorhofflimmern.

10. Arzneimittel enthaltend eine Verbindung der Formel (I), wie in einem der Ansprüche 1 bis 4 definiert, in Kombination mit einem inerten, nicht-toxischen, pharmazeutisch geeigneten Hilfsstoff.

11. Arzneimittel enthaltend eine Verbindung der Formel (I), wie in einem der Ansprüche 1 bis 4 definiert, in Kombination mit einem oder mehreren weiteren Wirkstoffen ausgewählt aus der Gruppe bestehend aus den Fettstoffwechsel verändernden Wirkstoffen, Antidiabetika, blutdrucksenkenden Wirkstoffen und antithrombotisch wirkenden Mitteln.

12. Arzneimittel nach Anspruch 10 oder 111 zur Behandlung und/oder Prävention von Hypertonie, koronarer Herzerkrankung, akutem Koronarsyndrom, Angina pectoris, Herzinsuffizienz, Myokardinfarkt und Vorhofflimmern.

13. Arzneimittel nach Anspruch 10 oder 11 zur Behandlung und/oder Prävention von Diabetes, Metabolischem Syndrom und Dyslipidämien.

## Claims

1. Compound of the formula (I) 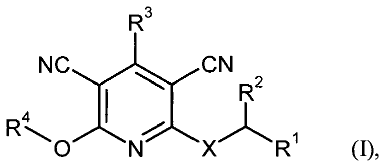 in which
X represents O or S,
R¹ represents (C₆-C₁₀)-aryl or 5- to 10-membered heteroaryl,
where (C₆-C₁₀)-aryl and 5- to 10-membered heteroaryl may be substituted by 1 or 2 substituents independently of one another selected from the group consisting of halogen, nitro, cyano, (C₁-C₆)-alkyl, trifluoromethyl, hydroxyl, (C₁-C₆)-alkoxy, amino, mono-(C₁-C₆)-alkylamino, di-(C₁-C₆)-alkylamino, hydroxycarbonyl, (C₁-C₆)-alkoxycarbonyl, aminocarbonyl, mono-(C₁-C₆)-alkylaminocarbonyl, di-(C₁-C₆)-alkylaminocarbonyl, (C₃-C₇)-cycloalkylaminocarbonyl, aminosulphonyl, mono-(C₁-C₆)-alkylaminosulphonyl, di-(C₁-C₆)-alkylaminosulphonyl, (C₁-C₆)-alkylsulphonylamino, pyrrolidino, piperidino, morpholino, piperazino, *N'*-(C₁-C₄)-alkylpiperazino, pyrrolidinocarbonyl, piperidinocarbonyl, morpholinocarbonyl, piperazinocarbonyl, *N'*-(C₁-C₄)-alkylpiperazinocarbonyl and -L-R⁵,
in which
L represents a bond, NH or O,
R⁵ represents phenyl or 5- or 6-membered heteroaryl,
where phenyl and 5- or 6-membered heteroaryl for their part may be substituted by 1 to 3 substituents independently of one another selected from the group consisting of halogen, nitro, cyano, (C₁-C₆)-alkyl, trifluoromethyl, hydroxyl, (C₁-C₆)-alkoxy, difluoromethoxy, trifluoromethoxy, amino, mono-(C₁-C₆)-alkylamino, di-(C₁-C₆)-alkylamino, hydroxycarbonyl and (C₁-C₆)-alkoxycarbonyl,
R² represents hydrogen or (C₁-C₄)-alkyl,
R³ represents phenyl or 5- or 6-membered heteroaryl,
where phenyl and 5- or 6-membered heteroaryl may be substituted by 1 to 3 substituents independently of one another selected from the group consisting of halogen, cyano, hydroxyl, (C₁-C₆)-alkyl, (C₁-C₆)-alkoxy, (C₃-C₇)-cycloalkoxy, tetrahydrofuranyloxy, pyrrolidinyloxy and -NR^{A}R^{B},
where (C₁-C₆)-alkyl and (C₁-C₆)-alkoxy may be substituted by 1 to 3 substituents independently of one another selected from the group consisting of fluorine, trifluoromethyl, (C₃-C₇)-cycloalkyl, hydroxyl, (C₁-C₄)-alkoxy, hydroxycarbonyl, (C₁-C₄)-alkoxycarbonyl, amino, aminocarbonyl, mono-(C₁-C₄)-alkylamino and di-(C₁-C₄)-alkylamino,
and
where (C₃-C₇)-cycloalkoxy, tetrahydrofuranyloxy and pyrrolidinyloxy may be substituted by 1 or 2 substituents independently of one another selected from the group consisting of (C₁-C₄)-alkyl, hydroxyl, oxo and (C₁-C₄)-alkoxy,
and
in which
R^{A} represents hydrogen or (C₁-C₆)-alkyl,
where (C₁-C₆)-alkyl for its part may be substituted by 1 to 3 fluorine substituents,
and
where (C₁-C₆)-alkyl for its part may be substituted by a substituent selected from the group consisting of hydroxyl and (C₁-C₄)-alkoxy,
R^{B} represents hydrogen, (C₁-C₆)-alkyl, (C₃-C₇)-cycloalkyl, (C₁-C₆)-alkylcarbonyl, (C₁-C₄)-alkylsulphonyl or (C₃-C₇)-cycloalkylsulphonyl,
where (C₁-C₆)-alkyl for its part may be substituted by 1 or 2 substituents independently of one another selected from the group consisting of fluorine, trifluoromethyl, (C₃-C₇)-cycloalkyl, hydroxyl, (C₁-C₄)-alkoxy, hydroxycarbonyl, (C₁-C₄)-alkoxycarbonyl, amino, mono-(C₁-C₄)-alkylamino and di-(C₁-C₄)-alkylamino,
and
where (C₃-C₇)-cycloalkyl for its part may be substituted by 1 or 2 substituents independently of one another selected from the group consisting of (C₁-C₄)-alkyl, hydroxyl, oxo and (C₁-C₄)-alkoxy,
or
R^{A} and R^{B} together with the nitrogen atom to which they are attached form a 4- to 7-membered heterocycle which may contain a further ring heteroatom from the group consisting of N, O and S and may be substituted by 1 or 2 substituents independently of one another selected from the group consisting of (C₁-C₄)-alkyl, hydroxyl, oxo and (C₁-C₄)-alkoxy,
or
where two adjacent substituents at phenyl together with the carbon atoms to which they are attached may form a 1,3-dioxolane, 1,3-dioxane or 2,2-difluoro-1,3-dioxolane,
R⁴ represents (C₁-C₆)-alkyl, (C₃-C₇)-cycloalkyl or 4- to 6-membered heterocyclyl,
where (C₁-C₆)-alkyl may be substituted by 1 or 2 substituents independently of one another selected from the group consisting of fluorine, trifluoromethyl, (C₃-C₇)-cycloalkyl, hydroxyl, (C₁-C₄)-alkoxy, hydroxycarbonyl, (C₁-C₄)-alkoxycarbonyl,
amino, mono-(C₁-C₄)-alkylamino, di-(C₁-C₄)-alkylamino, aminocarbonyl, mono-(C₁-C₄)-alkylaminocarbonyl, di-(C₁-C₄)-alkylaminocarbonyl and 5- or 6-membered heterocyclyl,
where (C₁-C₄)-alkoxy may be substituted by 1 or 2 substituents independently of one another selected from the group consisting of hydroxyl and (C₁-C₄)-alkoxy
and
where 5- or 6-membered heterocyclyl may be substituted by a substituent selected from the group consisting of oxo and (C₁-C₄)-alkyl
and
where (C₃-C₇)-cycloalkyl and 4- to 6-membered heterocyclyl may be substituted by 1 or 2 substituents independently of one another selected from the group consisting of (C₁-C₄)-alkyl, hydroxyl, (C₁-C₄)-alkoxy, hydroxycarbonyl, (C₁-C₄)-alkoxycarbonyl, amino, mono-(C₁-C₄)-alkylamino and di-(C₁-C₄)-alkylamino,
and N-oxides, salts, solvates, salts of the N-oxides and solvates of the N-oxides and salts thereof.

2. Compound of the formula (I) according to Claim 1 in which
X represents S,
R¹ represents phenyl or 5- or 6-membered heteroaryl,
where phenyl and 5- or 6-membered heteroaryl may be substituted by 1 or 2 substituents independently of one another selected from the group consisting of fluorine, chlorine, cyano, (C₁-C₄)-alkyl, trifluoromethyl, (C₁-C₄)-alkoxy, amino, mono-(C₁-C₄)-alkylamino, di-(C₁-C₄)-alkylamino, hydroxycarbonyl, (C₁-C₄)-alkoxycarbonyl, aminocarbonyl, mono-(C₁-C₄)-alkylaminocarbonyl, di-(C₁-C₄)-alkylaminocarbonyl, (C₃-C₆)-cycloalkylaminocarbonyl, (C₁-C₄)-alkylsulphonylamino, morpholino, piperazino, *N'*-(C₁-C₄)-alkylpiperazino and -L-R⁵,
in which
L represents a bond or NH,
R⁵ represents phenyl or 5- or 6-membered heteroaryl,
where phenyl and 5- or 6-membered heteroaryl for their part may be substituted by 1 or 2 substituents independently of one another selected from the group consisting of fluorine, chlorine, cyano, (C₁-C₄)-alkyl, trifluoromethyl, (C₁-C₄)-alkoxy, trifluoromethoxy, amino, hydroxycarbonyl and (C₁-C₄)-alkoxycarbonyl,
R² represents hydrogen or methyl,
R³ represents phenyl, pyrrolyl, oxazolyl, thiazolyl, isoxazolyl, pyrazolyl, imidazolyl and pyridyl,
where phenyl, pyrrolyl, oxazolyl, thiazolyl, isoxazolyl, pyrazolyl, imidazolyl and pyridyl may be substituted by 1 or 2 substituents independently of one another selected from the group consisting of fluorine, (C₁-C₆)-alkyl, hydroxyl, (C₁-C₄)-alkoxy and -NR^{A}R^{B},
where (C₁-C₆)-alkyl and (C₁-C₄)-alkoxy may be substituted by 1 to 3 substituents independently of one another selected from the group consisting of fluorine, trifluoromethyl, hydroxyl, methoxy, ethoxy, hydroxycarbonyl, amino, methylamino, ethylamino, *N*,*N*-dimethylamino and *N*,*N*-diethylamino,
and
in which
R^{A} represents hydrogen or (C₁-C₄)-alkyl,
where (C₁-C₄)-alkyl for its part may be substituted by a substituent selected from the group consisting of hydroxyl and (C₁-C₄)-alkoxy,
R^{B} represents hydrogen or (C₁-C₄)-alkyl,
where (C₁-C₄)-alkyl for its part may be substituted by 1 or 2 substituents independently of one another selected from the group consisting of hydroxyl, (C₁-C₄)-alkoxy and hydroxycarbonyl,
R⁴ represents (C₁-C₆)-alkyl, (C₄-C₆)-cycloalkyl or 5- or 6-membered heterocyclyl,
where (C₁-C₆)-alkyl may be substituted by 1 or 2 substituents independently of one another selected from the group consisting of fluorine, trifluoromethyl, (C₃-C₇)-cycloalkyl, hydroxyl, methoxy, ethoxy, hydroxycarbonyl, methoxycarbonyl, ethoxycarbonyl, amino, methylamino, ethylamino, *N*,*N*-dimethylamino, *N,N-*diethylamino and 5- or 6-membered heterocyclyl,
where 5- or 6-membered heterocyclyl for its part may be substituted by a substituent selected from the group consisting of oxo and methyl,
and
where (C₃-C₇)-cycloalkyl and 5- or 6-membered heterocyclyl may be substituted by 1 or 2 substituents independently of one another selected from the group consisting of methyl, hydroxyl, methoxy, hydroxycarbonyl, methoxycarbonyl, ethoxycarbonyl, amino, methylamino and *N*,*N*-dimethylamino,
and salts, solvates and solvates of the salts thereof.

3. Compound of the formula (I) according to Claim 1 in which
X represents O or S,
R¹ represents phenyl, thiazolyl, oxazolyl or pyridyl,
where phenyl and pyridyl are substituted by 1 or 2 substituents independently of one another selected from the group consisting of fluorine, chlorine, cyano, methyl, trifluoromethyl, methoxy, hydroxycarbonyl, methoxycarbonyl, ethoxycarbonyl, aminocarbonyl, methylaminocarbonyl, ethylaminocarbonyl, *N*,*N-*dimethylaminocarbonyl and *N*,*N*-diethylaminocarbonyl,
and
where thiazolyl and oxazolyl are substituted by a -L-R⁵ substituent,
in which
L represents a bond or NH,
R⁵ represents phenyl,
where phenyl for its part may be substituted by 1 or 2 substituents independently of one another selected from the group consisting of fluorine, chlorine, methyl, methoxy, ethoxy, hydroxycarbonyl, methoxycarbonyl and ethoxycarbonyl,
and
where thiazolyl and oxazolyl may be substituted by a substituent selected from the group consisting of fluorine, methyl, ethyl, methoxy, hydroxycarbonyl and methoxycarbonyl,
R² represents hydrogen or methyl,
R³ represents phenyl, pyrrolyl, oxazolyl, thiazolyl, isoxazolyl, pyrazolyl, imidazolyl and pyridyl ,
where phenyl, pyrrolyl, oxazolyl, thiazolyl, isoxazolyl, pyrazolyl, imidazolyl and pyridyl may be substituted by 1 or 2 substituents independently of one another selected from the group consisting of fluorine, (C₁-C₆)-alkyl, hydroxyl, (C₁-C₄)-alkoxy and -NR^{A}R^{B},
where (C₁-C₆)-alkyl and (C₁-C₄)-alkoxy may be substituted by 1 to 3 substituents independently of one another selected from the group consisting of fluorine, trifluoromethyl, hydroxyl, methoxy, ethoxy, hydroxycarbonyl, amino, methylamino, ethylamino, *N*,*N*-dimethylamino and *N*,*N*-diethylamino,
and
in which
R^{A} represents hydrogen or (C₁-C₄)-alkyl,
where (C₁-C₄)-alkyl for its part may be substituted by a substituent selected from the group consisting of hydroxyl and (C₁-C₄)-alkoxy,
R^{B} represents hydrogen or (C₁-C₄)-alkyl,
where (C₁-C₄)-alkyl for its part may be substituted by 1 or 2 substituents independently of one another selected from the group consisting of hydroxyl, (C₁-C₄)-alkoxy and hydroxycarbonyl,
R⁴ represents (C₁-C₆)-alkyl or (C₄-C₆)-cycloalkyl,
where (C₁-C₆)-alkyl may be substituted by 1 or 2 substituents independently of one another selected from the group consisting of fluorine, trifluoromethyl, (C₃-C₇)-cycloalkyl, hydroxyl, methoxy and ethoxy,
and
where (C₄-C₆)-cycloalkyl may be substituted by 1 or 2 substituents independently of one another selected from the group consisting of methyl, hydroxyl and methoxy,
and salts, solvates and solvates of the salts thereof.

4. Compound of the formula (I) according to any of Claims 1, 2 or 3 in which
X represents S,
R¹ represents phenyl, thiazolyl, oxazolyl or pyridyl,
where phenyl and pyridyl are substituted by 1 or 2 substituents independently of one another selected from the group consisting of fluorine, chlorine, cyano, methyl, trifluoromethyl, methoxy, hydroxycarbonyl, methoxycarbonyl, ethoxycarbonyl, aminocarbonyl, methylaminocarbonyl, ethylaminocarbonyl, N,N-dimethylaminocarbonyl and *N,N-*diethylaminocarbonyl,
and
where thiazolyl and oxazolyl are substituted by a -L-R⁵ substituent,
in which
L represents a bond or NH,
R⁵ represents phenyl,
where phenyl for its part may be substituted by 1 or 2 substituents independently of one another selected from the group consisting of fluorine, chlorine, methyl, methoxy, ethoxy, hydroxycarbonyl, methoxycarbonyl and ethoxycarbonyl,
and
where thiazolyl and oxazolyl may be substituted by a substituent selected from the group consisting of fluorine, methyl, ethyl, methoxy, hydroxycarbonyl and methoxycarbonyl,
R² represents hydrogen,
R³ represents phenyl or thiazolyl,
where phenyl may be substituted by 1 or 2 substituents independently of one another selected from the group consisting of (C₁-C₆)-alkyl, hydroxyl and (C₁-C₄)-alkoxy,
where (C₁-C₆)-alkyl and (C₂-C₄)-alkoxy may be substituted by 1 or 2 substituents independently of one another selected from the group consisting of hydroxyl and methoxy,
and
where thiazolyl may be substituted by a (C₁-C₆)-alkyl substituent,
where (C₁-C₆)-alkyl may be substituted by 1 or 2 substituents independently of one another selected from the group consisting of hydroxyl and methoxy,
R⁴ represents (C₁-C₄)-alkyl,
where alkyl may be substituted by 1 or 2 hydroxyl substituents,
and salts, solvates and solvates of the salts thereof.

5. Process for preparing the compounds of the formula (I) as defined in any of Claims 1 to 4, **characterized in that**
[A] a compound of the formula (II-A) 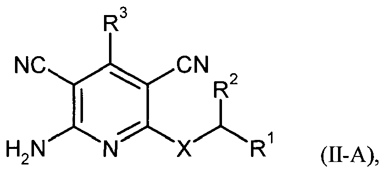 in which X, R¹, R² and R³ each have the meanings given in any of Claims 1 to 4,
is initially, using copper(II) chloride and isoamyl nitrite in a suitable solvent, converted into a compound of the formula (III-A) 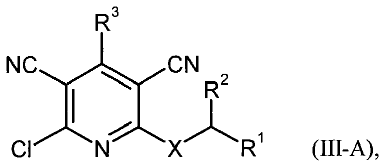 in which X, R¹, R² and R³ each have the meanings given in any of Claims 1 to 4, and this is then reacted in an inert solvent in the presence of a suitable base with a compound of the formula (IV)
R⁴-OH (IV),
in which R⁴ has the meaning given in any of Claims 1 to 4,
or
[B] in the case that X represents S, a compound of the formula (II-B) 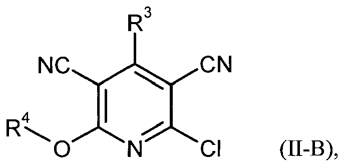 in which R³ and R⁴ each have the meanings given in any of Claims 1 to 4,
is reacted in an inert solvent with an alkali metal sulphide to give a compound of the formula (III-B) 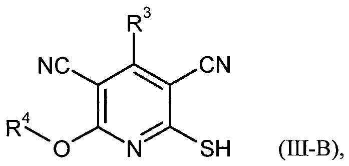 in which R³ and R⁴ each have the meanings given in any of Claims 1 to 4,
and this is then reacted in an inert solvent in the presence of a base with a compound of the formula (V) 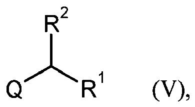 in which R¹ and R² each have the meanings given in any of Claims 1 to 4 and
Q represents a suitable leaving group, preferably halogen, in particular chlorine, bromine or iodine, or represents mesylate, tosylate or triflate,
any protective groups present are then removed and the resulting compounds of the formula (I) are, if appropriate, converted with the appropriate (i) solvents and/or (ii) bases or acids into their solvates, salts and/or solvates of the salts.

6. Compound of the formula (I) as defined in any of Claims 1 to 4 for the treatment and/or prophylaxis of diseases.

7. Compound of the formula (I) as defined in any of Claims 1 to 4 for use in a method for the treatment and/or prophylaxis of hypertension, coronary heart disease, acute coronary syndrome, angina pectoris, heart failure, myocardial infarction and atrial fibrillation.

8. Compound of the formula (I) as defined in any of Claims 1 to 4 for use in a method for the treatment and/or prophylaxis of diabetes, metabolic syndrome and dyslipidemias.

9. Use of a compound of the formula (I) as defined in any of Claims 1 to 4 for preparing a medicament for the treatment and/or prophylaxis of hypertension, coronary heart disease, acute coronary syndrome, angina pectoris, heart failure, myocardial infarction and atrial fibrillation.

10. Medicament, comprising a compound of the formula (I) as defined in any of Claims 1 to 4 in combination with an inert nontoxic pharmaceutically suitable auxiliary.

11. Medicament, comprising a compound of the formula (I) as defined in any of Claims 1 to 4 in combination with one or more further active ingredients selected from the group consisting of lipid metabolism-modifying active ingredients, antidiabetics, antihypertensive drugs and antithrombotic drugs.

12. Medicament according to Claim 10 or 11 for the treatment and/or prophylaxis of hypertension, coronary heart disease, acute coronary syndrome, angina pectoris, heart failure, myocardial infarction and atrial fibrillation.

13. Medicament according to Claim 10 or 11 for the treatment and/or prophylaxis of diabetes, metabolic syndrome and dyslipidemias.

## Revendications

1. Composé de formule (I) 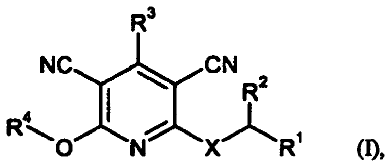 dans laquelle
X représente O ou S,
R¹ représente aryle en (C₆-C₁₀) ou hétéroaryle de 5 à 10 éléments,
aryle en (C₆-C₁₀) et hétéroaryle de 5 à 10 éléments pouvant être substitués avec 1 ou 2 substituants choisis indépendamment l'un de l'autre dans le groupe constitué par halogène, nitro, cyano, alkyle en (C₁-C₆), trifluorométhyle, hydroxy, alcoxy en (C₁-C₆), amino, mono-alkylamino en (C₁-C₆), di-alkylamino en (C₁-C₆) , hydroxycarbonyle, alcoxycarbonyle en (C₁-C₆), aminocarbonyle, mono-alkylaminocarbonyle en (C₁-C₆), di-alkylaminocarbonyle en (C₁-C₆), cycloalkylaminocarbonyle en (C₃-C₇), aminosulfonyle, mono-alkylaminosulfonyle en (C₁-C₆), di-alkylaminosulfonyle en (C₁-C₆), alkylsulfonylamino en (C₁-C₆), pyrrolidino, pipéridino, morpholino, pipérazino, N'-alkylpipérazino en (C₁-C₄), pyrrolidinocarbonyle, pipéridinocarbonyle, morpholinocarbonyle, pipérazinocarbonyle, N'-alkylpipérazinocarbonyle en (C₁-C₄) et -L-R⁵,
L représentant une liaison, NH ou O,
R⁵ représentant phényle ou hétéroaryle de 5 ou 6 éléments,
phényle et hétéroaryle de 5 ou 6 éléments pouvant de leur côté être substitués avec 1 à 3 substituants choisis indépendamment les uns des autres dans le groupe constitué par halogène, nitro, cyano, alkyle en (C₁-C₆), trifluorométhyle, hydroxy, alcoxy en (C₁-C₆) , difluorométhoxy, trifluorométhoxy, amino, mono-alkylamino en (C₁-C₆), di-alkylamino en (C₁-C₆) , hydroxycarbonyle et alcoxycarbonyle en (C₁-C₆),
R² représente hydrogène ou alkyle en (C₁-C₄),
R³ représente phényle ou hétéroaryle de 5 ou 6 éléments,
phényle et hétéroaryle de 5 ou 6 éléments pouvant être substitués avec 1 à 3 substituants choisis indépendamment les uns des autres dans le groupe constitué par halogène, cyano, hydroxy, alkyle en (C₁-C₆) , alcoxy en (C₁-C₆) , cycloalcoxy en (C₃-C₇), tétrahydrofuranyloxy, pyrrolidinyloxy et -NR^{A}R^{B},
alkyle en (C₁-C₆) et alcoxy en (C₁-C₆) pouvant être substitués avec 1 à 3 substituants choisis indépendamment les uns des autres dans le groupe constitué par fluor, trifluorométhyle, cycloalkyle en (C₃-C₇), hydroxy, alcoxy en (C₁-C₄), hydroxycarbonyle, alcoxycarbonyle en (C₁-C₄), amino, aminocarbonyle, mono-alkylamino en (C₁-C₄) et di-alkylamino en (C₁-C₄), et
cycloalcoxy en (C₃-C₇), tétrahydrofuranyloxy et pyrrolidinyloxy pouvant être substitués avec 1 ou 2 substituants choisis indépendamment l'un de l'autre dans le groupe constitué par alkyle en (C₁-C₄) , hydroxy, oxo et alcoxy en (C₁-C₄),
et
R^{A} représentant hydrogène ou alkyle en (C₁-C₆),
alkyle en (C₁-C₆) pouvant de son côté être substitué avec 1 à 3 substituants fluor,
et
alkyle en (C₁-C₆) pouvant de son côté être substitué avec un substituant choisi dans le groupe constitué par hydroxy et alcoxy en (C₁-C₄) ,
R^{B} représentant hydrogène, alkyle en (C₁-C₆), cycloalkyle en (C₃-C₇), alkylcarbonyle en (C₁-C₆), alkylsulfonyle en (C₁-C₄) ou cycloalkylsulfonyle en (C₃-C₇),
alkyle en (C₁-C₆) pouvant de son côté être substitué avec 1 ou 2 substituants choisis indépendamment l'un de l'autre dans le groupe constitué par fluor, trifluorométhyle, cycloalkyle en (C₃-C₇), hydroxy, alcoxy en (C₁-C₄), hydroxycarbonyle, alcoxycarbonyle en (C₁-C₄), amino, mono-alkylamino en (C₁-C₄) et di-alkylamino en (C₁-C₄),
et
cycloalkyle en (C₃-C₇) pouvant de son côté être substitué avec 1 ou 2 substituants choisis indépendamment l'un de l'autre dans le groupe constitué par alkyle en (C₁-C₄), hydroxy, oxo et alcoxy en (C₁-C₄),
ou
R^{A} et R^{B} formant ensemble avec l'atome d'azote auquel ils sont reliés un hétérocycle de 4 à 7 éléments qui peut contenir un hétéroatome de cycle supplémentaire de la série N, O ou S et être substitué avec 1 ou 2 substituants choisis indépendamment l'un de l'autre dans le groupe constitué par alkyle en (C₁-C₄), hydroxy, oxo et alcoxy en (C₁-C₄),
ou
deux substituants voisins sur le phényle pouvant former ensemble avec les atomes de carbone auxquels ils sont reliés un 1,3-dioxolane, un 1,3-dioxane ou un 2,2-difluoro-1,3-dioxolane,
R⁴ représente alkyle en (C₁-C₆) , cycloalkyle en (C₃-C₇) ou hétérocyclyle de 4 à 6 éléments,
alkyle en (C₁-C₆) pouvant être substitué avec 1 ou 2 substituants choisis indépendamment l'un de l'autre dans le groupe constitué par fluor, trifluorométhyle, cycloalkyle en (C₃-C₇), hydroxy, alcoxy en (C₁-C₄), hydroxycarbonyle, alcoxycarbonyle en (C₁-C₄), amino, mono-alkylamino en (C₁-C₄), di-alkylamino en (C₁-C₄), aminocarbonyle, mono-alkylaminocarbonyle en (C₁-C₄), di-alkylaminocarbonyle en (C₁-C₄) et hétérocyclyle de 5 ou 6 éléments,
alcoxy en (C₁-C₄) pouvant être substitué avec 1 ou 2 substituants choisis indépendamment l'un de l'autre dans le groupe constitué par hydroxy et alcoxy en (C₁-C₄),
et
hétérocyclyle de 5 ou 6 éléments pouvant être substitué avec un substituant choisi dans le groupe constitué par oxo et alkyle en (C₁-C₄),
et
cycloalkyle en (C₃-C₇) et hétérocyclyle de 4 à 6 éléments pouvant être substitués avec 1 ou
2 substituants choisis indépendamment l'un de l'autre dans le groupe constitué par (C₁-C₄), hydroxy, alcoxy en (C₁-C₄), hydroxycarbonyle, alcoxycarbonyle en (C₁-C₄), amino, mono-alkylamino en (C₁-C₄) et di-alkylamino en (C₁-C₄),
ainsi que leurs N-oxydes, sels, solvates, sels des N-oxydes et solvates des N-oxydes et des sels.

2. Composé de formule (I) selon la revendication 1, dans lequel
X représente S,
R¹ représente phényle ou hétéroaryle de 5 ou 6 éléments, phényle et hétéroaryle de 5 ou 6 éléments pouvant être substitués avec 1 ou 2 substituants choisis indépendamment l'un de l'autre dans le groupe constitué par fluor, chlore, cyano, alkyle en (C₁-C₄), trifluorométhyle, alcoxy en (C₁-C₄), amino, mono-alkylamino en (C₁-C₄), di-alkylamino en (C₁-C₄), hydroxycarbonyle, alcoxycarbonyle en (C₁-C₄), aminocarbonyle, mono-alkylaminocarbonyle en (C₁-C₄), di-alkylaminocarbonyle en (C₁-C₄), cycloalkylaminocarbonyle en (C₃-C₆), alkylsulfonylamino en (C₁-C₄), morpholino, pipérazino, *N'*-alkylpipérazino en (C₁-C₄) et -L-R⁵,
L représentant une liaison ou NH,
R⁵ représentant phényle ou hétéroaryle de 5 ou 6 éléments,
phényle et hétéroaryle de 5 ou 6 éléments pouvant de leur côté être substitués avec 1 ou 2 substituants choisis indépendamment l'un de l'autre dans le groupe constitué par fluor, chlore, cyano, alkyle en (C₁-C₄), trifluorométhyle, alcoxy en (C₁-C₄), trifluorométhoxy, amino, hydroxycarbonyle et alcoxycarbonyle en (C₁-C₄) ,
R² représente hydrogène ou méthyle,
R³ représente phényle, pyrrolyle, oxazolyle, thiazolyle, isoxazolyle, pyrazolyle, imidazolyle et pyridyle,
phényle, pyrrolyle, oxazolyle, thiazolyle, isoxazolyle, pyrazolyle, imidazolyle et pyridyle pouvant être substitués avec 1 ou 2 substituants choisis indépendamment l'un de l'autre dans le groupe constitué par fluor, alkyle en (C₁-C₆), hydroxy, alcoxy en (C₁-C₄) et -NR^{A}R^{B},
alkyle en (C₁-C₆) et alcoxy en (C₁-C₄) pouvant être substitués avec 1 à 3 substituants choisis indépendamment les uns des autres dans le groupe constitué par fluor, trifluorométhyle, hydroxy, méthoxy, éthoxy, hydroxycarbonyle, amino, méthylamino, éthylamino, *N,N*-diméthylamino et *N,N-*diéthylamino,
et
R^{A} représentant hydrogène ou alkyle en (C₁-C₄),
alkyle en (C₁-C₄) pouvant de son côté être substitué avec un substituant choisi dans le groupe constitué par hydroxy et alcoxy en (C₁-C₄),
R^{B} représentant hydrogène ou alkyle en (C₁-C₄),
alkyle en (C₁-C₄) pouvant de son côté être substitué avec 1 ou 2 substituants choisis indépendamment l'un de l'autre dans le groupe constitué par hydroxy, alcoxy en (C₁-C₄) et hydroxycarbonyle,
R⁴ représente alkyle en (C₁-C₆), cycloalkyle en (C₄-C₆) ou hétérocyclyle de 5 ou 6 éléments,
alkyle en (C₁-C₆) pouvant être substitué avec 1 ou 2 substituants choisis indépendamment l'un de l'autre dans le groupe constitué par fluor, trifluorométhyle, cycloalkyle en (C₃-C₇), hydroxy, méthoxy, éthoxy, hydroxycarbonyle, méthoxycarbonyle, éthoxycarbonyle, amino, méthylamino, éthylamino, *N,N*-diméthylamino, *N,N*-diéthylamino et hétérocyclyle de 5 ou 6 éléments,
hétérocyclyle de 5 ou 6 éléments pouvant être substitué avec un substituant choisi dans le groupe constitué par oxo et méthyle,
et
cycloalkyle en (C₃-C₇) et hétérocyclyle de 5 ou 6 éléments pouvant être substitués avec 1 ou 2 substituants choisis indépendamment l'un de l'autre dans le groupe constitué par méthyle, hydroxy, méthoxy, hydroxycarbonyle, méthoxycarbonyle, éthoxycarbonyle, amino, méthylamino et *N,N*-diméthylamino,
ainsi que leurs sels, solvates et solvates des sels.

3. Composé de formule (I) selon la revendication 1, dans lequel
X représente O ou S,
R¹ représente phényle, thiazolyle, oxazolyle ou pyridyle,
phényle et pyridyle étant substitués avec 1 ou 2 substituants choisis indépendamment l'un de l'autre dans le groupe constitué par fluor, chlore, cyano, méthyle, trifluorométhyle, méthoxy, hydroxycarbonyle, méthoxycarbonyle, éthoxycarbonyle, aminocarbonyle, méthylaminocarbonyle, éthylaminocarbonyle, *N,N-*diméthylaminocarbonyle et *N,N-*diéthylaminocarbonyle,
et
thiazolyle et oxazolyle étant substitués avec un substituant -L-R⁵,
L représentant une liaison ou NH,
R⁵ représentant phényle,
phényle pouvant de son côté être substitués avec 1 ou 2 substituants choisis indépendamment l'un de l'autre dans le groupe constitué par fluor, chlore, méthyle, méthoxy, éthoxy, hydroxycarbonyle, méthoxycarbonyle et éthoxycarbonyle,
et
thiazolyle et oxazolyle pouvant être substitués avec un substituant choisi dans le groupe constitué par fluor, méthyle, éthyle, méthoxy, hydroxycarbonyle et méthoxycarbonyle,
R² représente hydrogène ou méthyle,
R³ représente phényle, pyrrolyle, oxazolyle, thiazolyle, isoxazolyle, pyrazolyle, imidazolyle et pyridyle,
phényle, pyrrolyle, oxazolyle, thiazolyle, isoxazolyle, pyrazolyle, imidazolyle et pyridyle pouvant être substitués avec 1 ou 2 substituants choisis indépendamment l'un de l'autre dans le groupe constitué par fluor, alkyle en (C₁-C₆), hydroxy, alcoxy en (C₁-C₄) et -NR^{A}R^{B},
alkyle en (C₁-C₆) et alcoxy en (C₁-C₄) pouvant être substitués avec 1 à 3 substituants choisis indépendamment les uns des autres dans le groupe constitué par fluor, trifluorométhyle, hydroxy, méthoxy, éthoxy, hydroxycarbonyle, amino, méthylamino, éthylamino, *N,N*-diméthylamino et *N,N-*diéthylamino,
et
R^{A} représentant hydrogène ou alkyle en (C₁-C₄),
alkyle en (C₁-C₄) pouvant de son côté être substitué avec un substituant choisi dans le groupe constitué par hydroxy et alcoxy en (C₁-C₄),
R^{B} représentant hydrogène ou alkyle en (C₁-C₄),
alkyle en (C₁-C₄) pouvant de son côté être substitué avec 1 ou 2 substituants choisis indépendamment l'un de l'autre dans le groupe constitué par hydroxy, alcoxy en (C₁-C₄) et hydroxycarbonyle,
R⁴ représente alkyle en (C₁-C₆) ou cycloalkyle en (C₄-C₆),
alkyle en (C₁-C₆) pouvant être substitué avec 1 ou 2 substituants choisis indépendamment l'un de l'autre dans le groupe constitué par fluor, trifluorométhyle, cycloalkyle en (C₃-C₇), hydroxy, méthoxy et éthoxy,
et
cycloalkyle en (C₄-C₆) pouvant être substitué avec 1 ou 2 substituants choisis indépendamment l'un de l'autre dans le groupe constitué par méthyle, hydroxy et méthoxy,
ainsi que leurs sels, solvates et solvates des sels.

4. Composé de formule (I) selon la revendication 1, 2 ou 3, dans lequel
X représente S,
R¹ représente phényle, thiazolyle, oxazolyle ou pyridyle,
phényle et pyridyle étant substitués avec 1 ou 2 substituants choisis indépendamment l'un de l'autre dans le groupe constitué par fluor, chlore, cyano, méthyle, trifluorométhyle, méthoxy, hydroxycarbonyle, méthoxycarbonyle, éthoxycarbonyle, aminocarbonyle, méthylaminocarbonyle, éthylaminocarbonyle, *N,N-*diméthylaminocarbonyle et *N,N-*diéthylaminocarbonyle,
et
thiazolyle et oxazolyle étant substitués avec un substituant -L-R⁵,
L représentant une liaison ou NH,
R⁵ représentant phényle, phényle pouvant de son côté être
substitué avec 1 ou 2 substituants choisis indépendamment l'un de l'autre dans le groupe constitué par fluor, chlore, méthyle, méthoxy, éthoxy, hydroxycarbonyle, méthoxycarbonyle et éthoxycarbonyle,
et
thiazolyle et oxazolyle pouvant être substitués avec un substituant choisi dans le groupe constitué par fluor, méthyle, éthyle, méthoxy, hydroxycarbonyle et méthoxycarbonyle
R² représente hydrogène,
R³ représente phényle ou thiazolyle, phényle pouvant être substitué avec 1 ou 2 substituants choisis indépendamment l'un de l'autre dans le groupe constitué par alkyle en (C₁-C₆), hydroxy et alcoxy en (C₁-C₄),
alkyle en (C₁-C₆) et alcoxy en (C₂-C₄) pouvant être substitués avec 1 ou 2 substituants choisis indépendamment l'un de l'autre dans le groupe constitué par hydroxy et méthoxy, et
thiazolyle pouvant être substitué avec un substituant alkyle en (C₁-C₆),
alkyle en (C₁-C₆) pouvant être substitué avec 1 ou 2 substituants choisis indépendamment l'un de l'autre dans le groupe constitué par hydroxy et méthoxy,
R⁴ représente alkyle en (C₁-C₄),
alkyle pouvant être substitué avec 1 ou 2 substituants hydroxy,
ainsi que leurs sels, solvates et solvates des sels.

5. Procédé de fabrication de composés de formule (I) tels que définis dans les revendications 1 à 4, **caractérisé en ce que**
[A] un composé de formule (II-A) 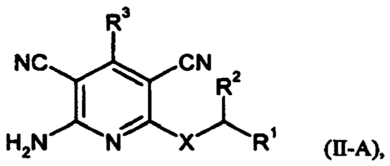 dans laquelle X, R¹, R² et R³ ont chacun les significations indiquées dans les revendications 1 à 4,
est tout d'abord transformé avec du chlorure de cuivre (II) et de l'isoamylnitrite dans un solvant approprié en un composé de formule (III-A) 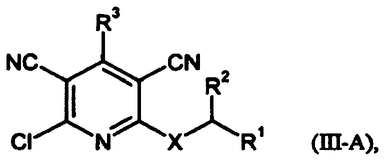 dans laquelle X, R¹, R² et R³ ont chacun les significations indiquées dans les revendications 1 à 4,
puis celui-ci est mis en réaction dans un solvant inerte en présence d'une base appropriée avec un composé de formule (IV)
R⁴-OH (IV)
dans laquelle R⁴ a la signification indiquée dans les revendications 1 à 4,
ou
[B] lorsque X représente S, un composé de formule (II-B) 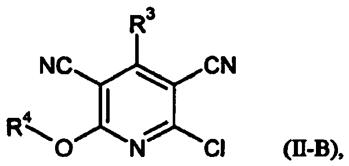 dans laquelle R³ et R⁴ ont chacun les significations indiquées dans les revendications 1 à 4,
est mis en réaction dans un solvant inerte avec un sulfure alcalin pour former un composé de formule (III-B) 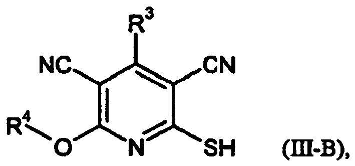 dans laquelle R³ et R⁴ ont chacun les significations indiquées dans les revendications 1 à 4,
puis celui-ci est mis en réaction dans un solvant inerte en présence d'une base avec un composé de formule (V) 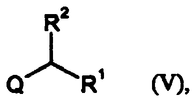 dans laquelle R¹ et R² ont les significations indiquées dans les revendications 1 à 4, et
Q représente un groupe partant approprié, de préférence halogène, notamment chlore, brome ou iode, ou mésylate, tosylate ou triflate,
puis les groupes protecteurs éventuellement présents sont clivés et les composés de formule (I) résultants sont éventuellement transformés avec les (i) solvants et/ou (ii) bases ou acides correspondants en leurs solvates, sels et/ou solvates des sels.

6. Composé de formule (I) tel que défini dans l'une quelconque des revendications 1 à 4, pour le traitement et/ou la prévention de maladies.

7. Composé de formule (I) tel que défini dans l'une quelconque des revendications 1 à 4, destiné à être utilisé dans un procédé de traitement et/ou de prophylaxie de l'hypertension, d'une maladie coronarienne, du syndrome coronarien aigu, de l'angine de poitrine, de l'insuffisance cardiaque, de l'infarctus du myocarde et de la fibrillation auriculaire.

8. Composé de formule (I) tel que défini dans l'une quelconque des revendications 1 à 4, destiné à être utilisé dans un procédé de traitement et/ou de prophylaxie du diabète, du syndrome métabolique et des dyslipidémies.

9. Utilisation d'un composé de formule (I) tel que défini dans l'une quelconque des revendications 1 à 4, pour la fabrication d'un médicament pour le traitement et/ou la prévention de l'hypertension, d'une maladie coronarienne, du syndrome coronarien aigu, de l'angine de poitrine, de l'insuffisance cardiaque, de l'infarctus du myocarde et de la fibrillation auriculaire.

10. Médicament contenant un composé de formule (I) tel que défini dans l'une quelconque des revendications 1 à 4 en combinaison avec un adjuvant inerte, non toxique, pharmaceutiquement approprié.

11. Médicament contenant un composé de formule (I) tel que défini dans l'une quelconque des revendications 1 à 4 en combinaison avec un ou plusieurs agents actifs supplémentaires choisis dans le groupe constitué par les agents actifs modifiant le métabolisme des lipides, les antidiabétiques, les agents actifs abaissant la tension artérielle et les agents à action antithrombotique.

12. Médicament selon la revendication 10 ou 11 pour le traitement et/ou la prévention de l'hypertension, d'une maladie coronarienne, du syndrome coronarien aigu, de l'angine de poitrine, de l'insuffisance cardiaque, de l'infarctus du myocarde et de la fibrillation auriculaire.

13. Médicament selon la revendication 10 ou 11 pour le traitement et/ou la prévention du diabète, du syndrome métabolique et des dyslipidémies.
